# EUROPEAN PATENT APPLICATION

(11) **EP 4 807 000 A1**
(43) Date of publication of application: **16.09.2026**
(21) Application number: 24866764.4
(22) Date of filing: 06.02.2024
(51) Int. Cl.: C12N 15/11, C12N 15/113, C12N 15/64, C12N 15/85, C12N 5/10, A61K 31/7088

(54) **UNIVERSAL METHOD FOR PREPARING CIRCULAR RNA AND USE OF CIRCULAR RNA**

(30) Priority: 19.09.2023 CN 202311210689; 09.10.2023 CN 202311299940
(71) Applicant: Byterna Therapeutics Ltd., Shanghai 201203 (CN)
(72) Inventor: ZHAO, Hui, Shanghai 201203 (CN)
(74) Representative: dompatent
(86) International application number: PCT/CN2024/076385
(87) International publication number: WO 2025/060337

(57) **Abstract**

Provided are a universal method for preparing a circular RNA and a use of the circular RNA. Specifically, optimized ribozyme recognition sites are determined on the basis of a specific local secondary structure, and correspondingly, ribozyme sequences are designed, so that circular RNA precursor molecules are constructed. Also provided are a method for preparing the circular RNA on the basis of the precursor molecules and the use of the circular RNA prepared. The circular RNA has high circularization efficiency, no sequences other than a target circular RNA sequence are introduced into a circularization product, the circularization efficiency is not affected by different sequences of the circular RNA, and the method is the universal method for preparing the circular RNA. The circular RNA prepared by the method can be used in various biomedicine fields such as diagnosis, prevention, and treatment of human or animal diseases.

## Description

### Technical Field

The present invention relates to the field of molecular biology and genetic engineering, and in particular to a universal method for preparing circular RNA and use thereof.

### Background

Circular RNA (circRNA) is a class of molecules with a covalently closed continuous loop structure without 5' or 3' ends. Due to this special "circular" structure, circular RNA molecules are resistant to degradation by RNases and exhibit higher stability both *in vivo* and *in vitro* compared to linear RNA molecules. Furthermore, recent studies have discovered that synthetic circular mRNAs can efficiently express proteins in mammalian cells. These excellent characteristics make circular RNA widely applicable in fields such as biomedicine and bioengineering.

Research indicates that circular RNA molecules possess various functions and potential uses, for example, as functional non-coding RNAs, as mRNA translation templates for protein expression within cells (e.g., circular mRNAs for vaccines, therapeutic proteins, etc.), as circular aptamers for diagnosis, treatment, and imaging, as circular guide RNAs (circ-gRNAs) for DNA and RNA editing, as circular RNAs recruiting ADAR editing systems, and for *in vivo* and *in vitro* cell therapies (e.g., CAR-T, CAR-NK, CAR-M cell therapies, stem cell therapies, etc.). Circular RNA has broad potential applications in biomedicine and bioengineering.

The preparation of circular RNA is a fundamental condition for expanding its potential uses in various fields. Circularization methods for preparing circular RNA need to address several critical concerns: First, the circularization efficiency should be as high as possible. Second, the circularization process must ensure sequence accuracy, avoiding the introduction of extra sequences as much as possible to ensure the sequence of the circularized RNA molecule remains unchanged, thereby avoiding potential risks caused by sequence alterations. Finally, the circularization platform should be universal to prepare diverse circular RNAs for various applications.

Currently, the most commonly used strategy for preparing circular RNA is the ribozyme self-catalysis strategy based on permuted intron-exon (PIE), i.e., the PIE strategy. The PIE strategy has significant advantages in the circularization of long-chain RNAs, which is crucial for the biomedical applications of circular RNA. The PIE method has a simple reaction system, and its circularization efficiency mainly depends on the efficient design of the self-splicing ribozyme circular RNA precursor molecule.

Circularization preparation methods for short-chain RNAs usually include chemical ligation, enzymatic ligation, and other methods. Compared to PIE, these methods have more complex reaction systems, such as requiring the introduction of additional enzymes or splint sequences. Furthermore, the sequence of the circularization product may be altered, and intermolecular polymerization ligation is prone to occur. Currently known PIE strategies can avoid the above drawbacks, but are less commonly used for preparing short-chain circular RNAs, mainly because they require introducing an additional circularization auxiliary sequence, or rely on sequence matching to select suitable splicing sites, or rely on matching larger splicing site structural modules to select suitable splicing sites. Therefore, existing PIE methods are difficult to achieve optimal design of self-splicing ribozyme circular RNA precursor molecules for short RNA sequences.

In summary, currently known PIE methods still have the drawback of not being able to universally circularize various types of RNA sequences and RNA sequences with various total lengths.

Therefore, there is an urgent need in the art to develop a universal circularization method with high circularization efficiency, precise sequence accuracy, and the ability to circularize various RNAs, to promote and expand the application of circular RNA in fields such as biomedicine and engineering.

### Summary of the Invention

The purpose of the present invention is to provide a universal circularization method with high circularization efficiency that can circularize various RNAs.

In a first aspect of the present invention, a polynucleotide for preparing a circular RNA is provided, which comprises, from the 5' end to the 3' end, operatively linked module Z1, module L to be circularized, and module Z2, and the polynucleotide is in a linear structure;
the modules Z1 and Z2 reconstitute into a ribozyme with self-splicing function, and can perform self-splicing (cleavage and ligation) under a suitable condition, thereby causing the module L to be circularized to form the circular RNA, wherein the splicing site is located in the distal loop region of the stem-loop structure in the circular RNA formed by the module L to be circularized.

In another preferred embodiment, the suitable condition refer to the condition suitable for self-splicing.

In another preferred embodiment, the modules Z1 and Z2 reconstitute into a ribozyme with self-splicing function under the suitable condition (or annealing condition).

In another preferred embodiment, the stem-loop structure is a single stem-loop structure or a poly stem-loop structure.

In another preferred embodiment, the stem-loop structure is a single stem-loop structure.

In another preferred embodiment, the single stem-loop structure has a stem region (also referred to as an "arm region") and a distal loop region located at the distal end of the stem region.

In another preferred embodiment, in the single stem-loop structure, the stem region has or does not have one or more internal loop regions.

In another preferred embodiment, in the single stem-loop structure, the stem region has or does not have one or more bulge regions.

In another preferred embodiment, the stem-loop structure is a poly stem-loop structure.

In another preferred embodiment, the poly stem-loop structure has 2-5, preferably 2-4, more preferably 3-4 distal loop regions.

In another preferred embodiment, the poly stem-loop structure has stem regions (also referred to as "arm regions") and distal loop regions located at the distal ends of the stem regions.

In another preferred embodiment, in the poly stem-loop structure, the numbers of stem regions and distal loop regions are the same or substantially the same.

In another preferred embodiment, in the poly stem-loop structure, the stem regions each independently have or do not have one or more internal loop regions.

In another preferred embodiment, in the poly stem-loop structure, the stem regions each independently have or do not have one or more bulge regions.

In another preferred embodiment, the splicing site is a splicing site for circularization.

In another preferred embodiment, the distal loop region comprises 2-15 nucleotides, preferably 2-10, more preferably 3-8 nucleotides.

In another preferred embodiment, the length Y of the stem region is not less than 2 nt, preferably 4 ≤ Y ≤ 100 nt, more preferably 10 ≤ Y ≤ 100 nt.

In another preferred embodiment, the module Z1 and/or module Z2 are respectively directly connected to the splicing site.

In another preferred embodiment, the module Z1 comprises the 3' end fragment h1 of the ribozyme, and the module Z2 comprises the 5' end fragment h2 of the ribozyme; or the module Z1 comprises the 5' end fragment h2 of the ribozyme, and the module Z2 comprises the 3' end fragment h1 of the ribozyme.

In another preferred embodiment, the module L to be circularized is a linear counterpart corresponding to the target sequence.

In another preferred embodiment, the target sequence is the circularized circular RNA.

In another preferred embodiment, the polynucleotide comprises RNA, DNA, or an RNA/DNA hybrid form.

In another preferred embodiment, the polynucleotide is RNA.

In another preferred embodiment, the polynucleotide is single-stranded.

In another preferred embodiment, the polynucleotide is L-RNA composed of L-ribonucleotides.

In another preferred embodiment, the polynucleotide has the structure shown in Formula I:

Z1-L-Z2 (Formula I)

Z1 comprises the 3' end fragment h1 of the ribozyme;
L is the linear counterpart of the target sequence;
Z2 comprises the 5' end fragment h2 of the ribozyme.

In another preferred embodiment, the Z1 further comprises the 3' end fragment h1 of the ribozyme and a random sequence r1, and the Z2 further comprises the 5' end fragment h2 of the ribozyme and a random sequence r2, and the structural formula of the polynucleotide is as shown in Formula II:

r1-h1-L-h2-r2 (Formula II)

wherein r1 and r2 are reverse complementary sequences.

In another preferred embodiment, the number of reverse complementary base pairs is ≥4, preferably ≥5.

In another preferred embodiment, the number of reverse complementary base pairs is ≤100 nt, preferably ≤50 nt, more preferably ≤30 nt.

In another preferred embodiment, r1 and r2 are completely complementary or substantially complementary (e.g., homology ≥80%, preferably ≥90% or ≥95%, or 100%).

In another preferred embodiment, the 3' end of the module L comprises ribozyme recognition site i, and the ribozyme recognition site i is the first n nucleotides at the 3' end of L, n≥1, preferably 1≤n≤7;

The 5' end of L comprises ribozyme recognition site ii, and the ribozyme recognition site ii is the first m nucleotides at the 5' end of L, m≥1, preferably 1≤m≤7.

In another preferred embodiment, the polynucleotide can undergo autocatalytic splicing reaction to generate a circular RNA molecule.

In another preferred embodiment, the ribozyme is an autocatalytic splicing ribozyme, and the ribozyme can recognize a specific site in module L.

In another preferred embodiment, the ribozyme can recognize a specific secondary structure in L (i.e., the secondary structure to be spliced constituted by the specific splicing site and surrounding sequences thereof).

In another preferred embodiment, the ribozyme is a Group I intron (or type I intron).

In another preferred embodiment, the ribozyme is selected from the group consisting of: Anabaena pre-tRNA-Leu gene intron, T4 phage Td gene intron, Tetrahymena intron, etc.

In another preferred embodiment, the ribozyme is the Anabaena pre-tRNA-Leu gene intron.

In another preferred embodiment, the ribozyme is the T4 phage Td gene intron.

In another preferred embodiment, the ribozyme is the Tetrahymena intron.

In another preferred embodiment, the ribozyme is a ribozyme modified by mutation in key regions. In another preferred embodiment, the ribozyme is a ribozyme modified by mutation in the internal guide sequence (IGS).

In another preferred embodiment, the ribozyme is a ribozyme obtained by modifying the IGS of the Anabaena pre-tRNA-Leu gene intron.

In another preferred embodiment, the ribozyme modification involves mutating the IGS of the Anabaena pre-tRNA-Leu gene intron such that the mutated IGS can form n base complementary pairs with the ribozyme recognition site on the target sequence, n≥1, preferably 1≤n≤7.

In another preferred embodiment, the ribozyme is a modified ribozyme obtained by partially or completely mutating positions 10 to 16 at the 5' end of the Anabaena pre-tRNA-Leu gene intron. Wherein, the mutation rule is: the base at position 10 at the 5' end of the intron is mutated to the complementary base of the base at position 2 of the 3' end of the sequence to be circularized, the base at position 11 is mutated to the complementary base of the base at position 3 of the 3' end of the sequence to be circularized, the base at position 12 is mutated to the complementary base of the base at position 4 of the 3' end of the sequence to be circularized, the base at position 13 is mutated to the complementary base of the base at position 5 of the 3' end of the sequence to be circularized, the base at position 14 is mutated to the complementary base of the base at position 6 of the 3' end of the sequence to be circularized, the base at position 15 is mutated to the complementary base of the base at position 7 of the 3' end of the sequence to be circularized, and the base at position 16 is mutated to the complementary base of the base at position 8 of the 3' end of the sequence to be circularized.

In another preferred embodiment, the ribozyme modification involves mutating positions 10 and 11 at the 5' end of the Anabaena pre-tRNA-Leu gene intron.

In another preferred embodiment, the ribozyme modification involves mutating positions 10, 11, and 12 at the 5' end of the Anabaena pre-tRNA-Leu gene intron.

In another preferred embodiment, the ribozyme modification involves mutating positions 10, 11, 12, and 13 at the 5' end of the Anabaena pre-tRNA-Leu gene intron.

In another preferred embodiment, the ribozyme modification involves mutating positions 10, 11, 12, 13, and 14 at the 5' end of the Anabaena pre-tRNA-Leu gene intron.

In another preferred embodiment, the module L comprises:
(L1) fragment i of a non-coding region;
(L2) mRNA coding region; and
(L3) fragment ii of a non-coding region;
wherein L1 and L3 are formed by splitting one non-coding region at a specific site.

In another preferred embodiment, the module L comprises:
(L1a) fragment i of a single stem-loop structure element;
(L2a) sequence excluding the single stem-loop structure; and
(L3a) fragment ii of a single stem-loop structure element;
wherein L1a and L3a are formed by splitting one single stem-loop structure element at a specific site.

In another preferred embodiment, the module L is an mRNA. In another preferred embodiment, the single stem-loop structure is located in the coding region or non-coding region of the mRNA.

In another preferred embodiment, the single stem-loop structure is located in the non-coding region of the mRNA.

In another preferred embodiment, the single stem-loop structure is located in a translation initiation element of the mRNA.

In another preferred embodiment, the translation initiation element includes but is not limited to: Internal Ribosome Entry Site (IRES), Kozak sequence, methylation modification site, cap-independent translation enhancers (CITE), 5' untranslated region (5'-UTR), 3' untranslated region (3'-UTR), other elements capable of regulating mRNA translation activity.

In another preferred embodiment, the translation initiation element is an IRES.

In another preferred embodiment, the single stem-loop structure is located in a regulatory element of the mRNA.

In another preferred embodiment, the mRNA regulatory element is an element capable of regulating the behavior of the circular mRNA molecule within a cell, the mRNA regulatory element including but not limited to: aptamer sequence elements for binding to aptamer substrates, binding elements for binding to microRNAs (miRNA), elements for interacting with other RNA molecules to alter the secondary structure or spatial conformation of the circular mRNA.

In another preferred embodiment, the mRNA coding sequence comprised in the L2 module is used to encode 1 target protein.

In another preferred embodiment, the mRNA coding sequence comprised in the L2 module is used to encode n (n≥2) target proteins.

In another preferred embodiment, the n target proteins are the same protein or different proteins.

In another preferred embodiment, a spacer sequence is inserted between the mRNA coding sequences of the n target proteins, wherein the product encoded by the spacer sequence can be degraded under specific conditions, thereby separating the multiple proteins.

In another preferred embodiment, the spacer sequence is an RNA sequence encoding a 2A peptide.

In another preferred embodiment, the single stem-loop structure is an SL structure (Stem Loop structure).

In another preferred embodiment, the non-coding region or coding region can form an SL structure.

In another preferred embodiment, the poly stem-loop structure is a TLSL structure (TLS-like structure).

In another preferred embodiment, the non-coding region or coding region can form a specific secondary structure.

In another preferred embodiment, the secondary structure comprises a TLSL structure (TLS-like structure) module.

In another preferred embodiment, the module L is determined by the following method:
(1) directly connecting the 5' and 3' ends of the linear target sequence to be circularized to form a target circular sequence.
(2) determining a splicing site (SS) based on the local secondary structure of the target sequence, wherein the polynucleotide sequences on both sides of the splicing site are ribozyme recognition sites.
(3) splitting the target circular sequence at the selected splicing site (SS) to form the module L.

In another preferred embodiment, the splicing site is located in an SL structure.

In another preferred embodiment, the SL is composed of an arm connection and a distal loop connected.

In another preferred embodiment, the arm may comprise a bulge and/or an internal loop.

In another preferred embodiment, the splicing site is located on the distal loop of the SL structure.

In another preferred embodiment, the splicing site is located between nucleotide U (or T) on the distal loop and an adjacent nucleotide, and the nucleotide U (or T) is located at the 3' end of module L formed after splitting at the splicing site.

In another preferred embodiment, for the distal loop region in the stem-loop structure, the size of the distal loop is ≥4 nt, preferably 4-25 nt, more preferably 4-20 nt, most preferably 5-15 nt.

In another preferred embodiment, the splicing site is located in an SL structure of a translation initiation element.

In another preferred embodiment, the translation initiation element is a cap-independent translation initiation element.

In another preferred embodiment, the translation initiation element is an internal ribosome entry site (IRES).

In another preferred embodiment, the splicing site is located in an SL structure of an mRNA coding region.

In another preferred embodiment, the splicing site is located in an SL structure of an mRNA 5'-UTR region.

In another preferred embodiment, the splicing site is located in an SL structure of an mRNA 3'-UTR region.

In another preferred embodiment, the coding region encodes a protein product (polypeptide, protein or fragment thereof) or a non-protein product (such as miRNA precursor, ncRNA, etc.).

In another preferred embodiment, the module L is a non-coding RNA (ncRNA).

In another preferred embodiment, the splicing site is located in an SL structure of a non-coding RNA (ncRNA).

In another preferred embodiment, the splicing site is located in a TLSL structure.

In another preferred embodiment, the TLSL is composed of a multi-arm junction and multiple distal loops connected, wherein one arm of the multi-arm junction is a terminal arm, the terminal arm is not connected to a distal loop, and each of the other arms is respectively connected to a distal loop to form a closed connection, forming a continuous multi-distal-loop structure with exposed 5' and 3' ends only on the terminal arm.

In another preferred embodiment, the arms of the multi-arm junction may comprise bulges and internal loops, and the junction of the multi-arm junction may comprise a multiloop.

In another preferred embodiment, the TLSL structure is a TLSL structure containing 2-5 distal loops.

In another preferred embodiment, the splicing site is located on any one distal loop of the TLSL structure.

In another preferred embodiment, the splicing site is located between nucleotide U (or T) on any one distal loop of the TLSL structure and an adjacent nucleotide, and the nucleotide U (or T) is located at the 3' end of module L formed after splitting at the splicing site.

In another preferred embodiment, the size of the distal loop is ≥4 nt.

In another preferred embodiment, the splicing site is located in a TLSL structure of a translation initiation element.

In another preferred embodiment, the translation initiation element is a cap-independent translation initiation element.

In another preferred embodiment, the translation initiation element is an internal ribosome entry site (IRES).

In another preferred embodiment, the splicing site is located in a TLSL structure of an mRNA coding region.

In another preferred embodiment, the splicing site is located in a TLSL structure of an mRNA 5'-UTR region.

In another preferred embodiment, the splicing site is located in a TLSL structure of an mRNA 3'-UTR region.

In another preferred embodiment, the splicing site is located in a TLSL structure of a non-coding RNA (ncRNA).

In another preferred embodiment, the method for determining the splicing site is as follows:
1) obtaining the secondary structure of the target circular RNA;
2) determining the SL structure or TLSL structure based on the secondary structure information;
3) selecting the splicing site SS on the SL structure or TLSL structure;
the SL structure or TLSL structure has ribozyme recognition activity.

In another preferred embodiment, the method for obtaining the secondary structure of the target circular RNA includes prediction using computer algorithms, determination through experiments.

In another preferred embodiment, the computer algorithm prediction methods include RNAfold, Linearfold, etc.

In a second aspect of the present invention, a vector is provided, which comprises the polynucleotide according to the first aspect of the present invention.

In another preferred embodiment, the vector comprises a nucleic acid sequence (e.g., DNA sequence) for producing the polynucleotide according to the first aspect of the present invention.

In another preferred embodiment, the vector is a plasmid.

In another preferred embodiment, the vector is a virus-like particle.

In another preferred embodiment, the vector is a non-virus-like particle.

In another preferred embodiment, the vector is a viral vector.

In another preferred embodiment, the vector is a non-viral vector.

In a third aspect of the present invention, a method for preparing a circular RNA is provided, comprising the steps of:
(1) determining a splicing site based on the local secondary structure of the target sequence, obtaining module L in the polynucleotide according to the first aspect of the present invention, i.e., the linear counterpart of the target sequence;
(2) connecting the 3' end fragment of the ribozyme, the linear counterpart of the target sequence, and the 5' end fragment of the ribozyme by an appropriate method to obtain a circular RNA precursor molecule;
(3) the circular RNA precursor molecule obtained in step (2) undergoes a self-splicing reaction under a suitable condition to produce the target circular RNA.

In another preferred embodiment, the reaction condition is a reaction system containing GTP and magnesium ions.

In another preferred embodiment, the reaction system contains 0.01 mM to 10 mM GTP.

In another preferred embodiment, the reaction system contains 1 mM to 100 mM magnesium ions.

In another preferred embodiment, the pH of the reaction system is 5-8.

In another preferred embodiment, the temperature of the reaction system is 25-65°C.

In another preferred embodiment, the reaction is carried out *in vitro* for a time of 5 min to 60 min.

In another preferred embodiment, the reaction condition is reacted at 2±1 mM GTP and a buffer containing magnesium ions (e.g., 50 mM Tris HCl, 10 mM MgCl₂, 1 mM DTT, pH is 7.5), 55±3°C for 15±5 minutes.

In a fourth aspect of the present invention, a circular RNA is provided, which is formed by self-splicing of the polynucleotide according to the first aspect of the present invention.

In another preferred embodiment, the circular RNA is prepared by the method according to the third aspect of the present invention.

In another preferred embodiment, the circular RNA is selected from the group consisting of: a circular guide RNA, circular RNA molecular sponge, circular interfering RNA, circular RNA aptamer, circular RNA probe, circular RNA molecular sensor, circular RNA molecular diagnostics, functional non-coding circular RNA, or a combination thereof.

In another preferred embodiment, the polynucleotide for preparing circular RNA or the circular RNA of the present invention comprises one or more modified nucleotides.

In another preferred embodiment, the manner in which nucleotides in the RNA molecule are modified comprises but is not limited to: N6-methyladenosine (m6A), pseudouridine (ψ), N1-methylpseudouridine (m1ψ), and 5-methoxyuridine (5moU).

In another preferred embodiment, the nucleotides modification in the RNA molecule is selected from one or more of N6-methyladenosine (m6A), pseudouridine (ψ), N1-methylpseudouridine (mly), and 5-methoxyuridine (5moU).

In another preferred embodiment, the circular RNA molecule is capable of expressing a polypeptide or protein within a cell.

In another preferred embodiment, wherein the cell is *in vivo.*

In another preferred embodiment, wherein the cell is a mammalian cell.

In another preferred embodiment, wherein the mammalian cell is derived from a human.

In another preferred embodiment, wherein the production of the polypeptide or protein is tissue-specific.

In another preferred embodiment, wherein the tissue specificity is localized to a tissue selected from muscle, heart, spleen, liver, kidney, brain, lung, skin, pancreas, blood, and lymph nodes.

In another preferred embodiment, wherein the cell is *in vitro.*

In another preferred embodiment, the polypeptide or protein is a therapeutic polypeptide or protein. In another preferred embodiment, the polypeptide or protein is a disease-preventive polypeptide or protein.

In a fifth aspect of the present invention, an engineered host cell is provided, comprising the circular RNA according to the fourth aspect of the present invention, or comprising the vector according to the second aspect of the present invention, or having integrated into its genome a coding sequence for producing the polynucleotide according to the first aspect of the present invention.

In a sixth aspect of the present invention, a pharmaceutical composition is provided, comprising (a) the polynucleotide for preparing a circular RNA according to the first aspect of the present invention, the circular RNA according to the fourth aspect of the present invention, or a combination thereof; and (b) a pharmaceutically acceptable carrier thereof.

In another preferred embodiment, the pharmaceutical composition comprises a vaccine composition.

In another preferred embodiment, the pharmaceutical composition further comprises an additional other drug.

In another preferred embodiment, the other drug comprises but is not limited to one or more selected from the group consisting of: an additional circular RNA, mRNA vaccine, mRNA drug, vaccine adjuvant, siRNA, small molecule drug, polypeptide drug, protein drug, antibody drug, cell therapy drug, gene editing drug, gene therapy drug, microbial therapy.

In another preferred embodiment, the pharmaceutical composition comprises: a lipid nanoparticle, liposome, exosome, cationic liposome, targeted lipid nanoparticle, micelle, polymer material, nano-drug delivery system, mesoporous material, adeno-associated virus, Sendai virus, protamine carrier system, live cell carrier.

In another preferred embodiment, the pharmaceutically acceptable carrier is a modified lipid nanoparticle.

In another preferred embodiment, the modified lipid nanoparticle has targeted delivery function.

In another preferred embodiment, the pharmaceutical composition is a liquid formulation, solid formulation, or gel formulation.

In another preferred embodiment, the pharmaceutical composition is administered by a mode selected from the group consisting of: gene gun injection, intravenous injection, intratumoral injection, intramuscular injection, intrathecal injection, intradermal injection, subcutaneous injection, intranodal injection, nasal inhalation, mucosal infiltration, microneedle.

In a seventh aspect of the present invention, a method for producing a polypeptide or protein in a cell is provided, the method comprising: contacting the cell with (i) the circular RNA according to the fourth aspect of the present invention, or (ii) the vector according to the second aspect of the present invention, under a condition that the polypeptide or protein encoding nucleic acid sequence of the circular RNA can be translated and the polypeptide or protein can be produced in the cell;
alternatively, the method comprising: culturing the engineered host cell according to the fifth aspect of the present invention under a condition suitable for expression, thereby producing the polypeptide or protein.

In another preferred embodiment, wherein the cell is *in vivo.*

In another preferred embodiment, wherein the cell is a mammalian cell.

In another preferred embodiment, wherein the mammalian cell is derived from a human.

In another preferred embodiment, wherein the production of the polypeptide or protein has tissue specificity.

In another preferred embodiment, wherein the tissue specificity is localized to a tissue selected from muscle, heart, spleen, liver, kidney, brain, lung, skin, pancreas, blood, and lymph node.

In another preferred embodiment, wherein the cell is *in vitro.*

In another preferred embodiment, the polypeptide or protein is a therapeutic polypeptide or protein.

In another preferred embodiment, the polypeptide or protein is a disease-preventive polypeptide or protein.

In an eighth aspect of the present invention, a use of the circular RNA according to the fourth aspect of the present invention, or the vector according to the second aspect of the present invention, or the engineered host cell according to the fifth aspect of the present invention, or the pharmaceutical composition according to the sixth aspect of the present invention, or the method for producing a polypeptide or protein in a cell according to the seventh aspect of the present invention, for the preparation of a pharmaceutical composition or a cosmetic composition is provided.

In another preferred embodiment, the pharmaceutical composition is used for a purpose selected from the group consisting of: anti-aging, medical aesthetics, healthcare, prevention, diagnosis, and treatment of a disease in human or animal.

In another preferred embodiment, the circular RNA is a circular mRNA, and the circular mRNA encodes various types of proteins, the proteins comprising but not limited to: a functional protein, preventive vaccine, therapeutic vaccine, monoclonal antibody, bispecific antibody, multispecific antibody, intrabody, chimeric antigen, fusion protein, cytokine.

In another preferred embodiment, the circular mRNA is used for preparing an mRNA cell therapy drug *in vitro,* the mRNA cell therapy drug comprising but not limited to: chimeric antigen receptor T-cell immunotherapy (CAR-T), chimeric antigen receptor NK-cell immunotherapy (CAR-NK), tumor-infiltrating lymphocyte therapy (TIL), chimeric antigen receptor macrophage immunotherapy (CAR-M), induced pluripotent stem cell therapy (iPS), embryonic stem cell therapy (ESC), adult stem cell therapy (ASC), mesenchymal stem cell therapy (MSC), hematopoietic stem cell therapy (HSC).

In another preferred embodiment, the circular mRNA is used to generate an mRNA cell therapy drug *in vivo,* wherein the circular mRNA is transported to a specific cell type in the body via an appropriate delivery vehicle, and expresses a protein required for cell therapy within the cell, thereby converting the cell type into a therapeutic cell drug, the cell drug including but not limited to chimeric antigen receptor T-cell immunotherapy (CAR-T), chimeric antigen receptor NK-cell immunotherapy (CAR-NK), tumor-infiltrating lymphocyte therapy (TIL), chimeric antigen receptor macrophage immunotherapy (CAR-M), induced pluripotent stem cell therapy (iPS), embryonic stem cell therapy (ESC), adult stem cell therapy (ASC), mesenchymal stem cell therapy (MSC), hematopoietic stem cell therapy (HSC).

It should be understood that within the scope of the present invention, the above-mentioned technical features of the present invention and the technical features specifically described in the following text (such as in the embodiments) can be combined with each other to constitute new or preferred technical solutions. Due to space limitations, they will not be repeated here one by one.

### Brief Description of the Drawings

Figure 1 shows the design principle of a classic PIE circularization construct.
Figure 2 shows the brief design principle of Hi-scarless-PIE.
Figure 3 shows the basic secondary structure elements constituting a TLS-like structure (TLSL).
Figure 4 shows a schematic diagram of the secondary structure of a typical TLS-like structure (TLSL).
Figure 5 shows the process and results of identifying TLSL structural elements, modifying the Ana-PIE intron ribozyme, and forming a "ribozyme-TLSL structure module" constituting a Hi-Scarless-PIE circular RNA construct. Figure 5A shows the TLSL structural element, with the arrow indicating the position of the splicing site; Figure 5B shows the capillary electrophoresis detection results of the self-splicing reaction of the native Ana-PIE intron and the modified Ana-PIE intron.
Figure 6 shows the influence of different self-splicing ribozyme recognition sites on circularization efficiency. Figure 6A shows the position of the first splicing site; Figure 6B shows the position of the second splicing site; Figure 6C shows the capillary electrophoresis detection results.
Figure 7 shows the identification of whether the product of the present invention is the target circular RNA molecule. Figure 7A shows agarose gel electrophoresis analysis of the product; Figure 7B shows agarose gel electrophoresis analysis of the product after RNase H treatment; Figure 7C shows the sequencing results of the splicing site.
Figure 8 shows the influence of different ribozyme mutations on Hi-Scarless-PIE circularization efficiency. Figure 8A shows the secondary structure of the native Anabaena intron and the default numbering of nucleotides, as well as the region for ribozyme modification; Figure 8B shows agarose gel electrophoresis analysis of circular RNA synthesized by different modified ribozymes.
Figure 9 shows experimental results of construct design based on the TLSL structure from PV3 IRES and circularization verification.
Figure 10 shows experimental results of construct design based on TLSL structures from HRV-B3 IRES and EV-B107 IRES and circularization verification.
Figure 11 shows experimental results of construct design based on the TLSL structure from SimianV4 IRES and circularization verification.
Figure 12 shows experimental results of construct design based on the TLSL structure from BEV1 IRES and circularization verification.
Figure 13 shows circularization results of different "ribozyme-TLSL circularization modules." Figure 13A shows the capillary electrophoresis detection results; Figure 13B shows the peak diagram of crude RNA products.
Figure 14 shows the circularization effect of EMCV after inserting TLSL. Figure 14A shows the secondary structures of TLSL, EMCV IRES, and EMCV+TLSL; Figure 14B shows agarose gel electrophoresis detection results of the products; Figure 14C shows protein expression results.
Figure 15 shows the circularization effect of CrPV after inserting TLSL. Figure 15A shows the secondary structures of TLSL, CrPV IRES, and CrPV+TLSL; Figure 15B shows agarose gel electrophoresis detection results of the products; Figure 15C shows protein expression results.
Figure 16 shows the analysis results of the distribution of TLSL structure elements in various types of RNA molecules. Figure 16A shows a typical cloverleaf TLSL structure element; Figure 16B shows the universal Hi-scarless-PIE circularization platform; Figures 16C-G respectively show some representative cloverleaf TLSL structure elements derived from IRES, human 5'-UTR, human 3'-UTR, human CDS, and non-coding RNA sequence sets.
Figure 17 shows the expression of immunogenic cytokines induced in A549 cells by circ-GLuc-mRNA prepared using classic PIE and Hi-Scarless-PIE methods.
Figure 18 shows the detection results of protein expression levels of circular GLuc mRNA with different IRES sequences in HEK-293T and A549 cells.
Figure 19 shows the protein expression of circular hEPO and circular EGFP mRNA prepared using the Hi-Scarless-PIE method.
Figure 20 shows the trend of protein expression levels over time in HEK-293T and A549 cells for traditional capped and tailed linear mRNA, circular mRNA prepared by the classic PIE system, and circular mRNA prepared by the Hi-Scarless-PIE disclosed herein.
Figure 21 shows the preparation and characterization of circular FLuc-mRNA encapsulated in LNP lipid nanoparticles prepared using the classic PIE method and the Hi-Scarless-PIE method, respectively.
Figure 22 shows the *in vivo* protein expression of circular FLuc-mRNA-LNP lipid nanoparticles prepared using the classic PIE method and the Hi-Scarless-PIE method in mice.
Figure 23 shows the brief design principle of Uni-cRNA of the present invention.
Figure 24 shows a schematic diagram of splicing site selection in the IRES region in the Uni-cRNA construct design process. In Figure 24B, the blue part is the distal loop, the green part is the complementary paired bases in the stem region, the yellow part is the bulge or internal loop in the stem region, and the arrow indicates the splicing site.
Figure 25 shows that the splicing site selected in the Uni-cRNA construct design process is located on the distal loop of the CDS region. The arrow indicates the splicing site.
Figure 26 shows the splicing site selected after sequence optimization of the CDS region. The arrow indicates the splicing site.
Figure 27 shows the splicing site selected on the internal loop of the IRES region during the Uni-cRNA construct design process. The arrow indicates the splicing site.
Figure 28 shows the results of capillary electrophoresis detection of circularization efficiency for circular RNA prepared by Uni-cRNA.
Figure 29 shows the identification of the circular RNA product prepared by Uni-cRNA.
Figure 30 shows the detection results of protein expression from circular mRNA prepared by Uni-cRNA.
Figure 31 shows the distribution of single stem-loop structures in the "ribozyme-SL circularization module" among various types of RNA sequences.

### Detailed Description

Through extensive and in-depth research and extensive screening, the present inventors have developed for the first time a circularization technology based on the secondary structure of circular RNA, comprising a precursor polynucleotide for constructing circular RNA, and provided a method for preparing circular RNA based on the precursor polynucleotide and the use of the prepared circular RNA. Specifically, the inventors determined optimized ribozyme recognition sites based on specific local secondary structures (i.e., stem-loop structures), and combined them with wild-type or modified RNA ribozymes to construct a circular RNA precursor polynucleotide comprising operably linked module Z1, module L to be circularized, and module Z2. Under annealing conditions, the modules Z1 and Z2 of the circular RNA precursor polynucleotide of the present invention reconstitute into a ribozyme with self-splicing function, thereby splicing (cleaving and ligating) the module L to be circularized, causing the module L to be circularized to form circular RNA. The inventors unexpectedly found that the circularization technology based on single stem-loop structures and poly stem-loop structures of the present invention has excellent circularization efficiency, and the circularization efficiency is not affected by different sequences of the RNA to be circularized, making it a universal method for circularizing RNA. Furthermore, the circular RNA product of the present invention can be free of any extra sequences, so the method is a truly scarless circularization. Based on this, the present invention was completed.

### RNA Circularization Strategy of the Present Invention

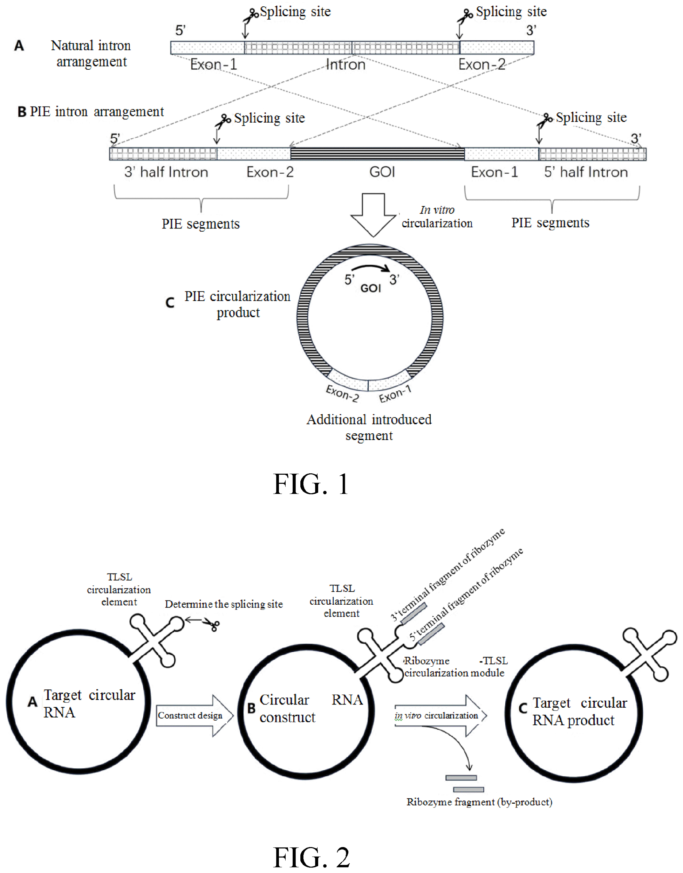

Circularization of RNA can be achieved utilizing the self-splicing properties of Group I intron ribozymes or Group II intron ribozymes. In bacteria and non-metazoan eukaryotes, certain introns located in rRNA, tRNA, and mRNA possess autocatalytic activity and can excise the intron in a "5'-exon-intron ribozyme-exon-3'" arrangement through back-splicing, while simultaneously ligating the two exons (Past, present, and future of circRNAs. Patop IL, Wüst S, Kadener S. EMBO J. 2019 Aug 15;38(16):e100836.). Based on the above principle, the permuted intron exon (PIE) system was proposed as early as 1992. The PIE system splits the intron ribozyme into 5' and 3' half fragments, then places the 3' intron fragment upstream of the 5' end of the exon and the 5' intron fragment downstream of the 3' end of the exon, forming a "5'-intron ribozyme 3' fragment-exon-intron ribozyme 5' fragment-3'" arrangement (Group I permuted intron-exon (PIE) sequences self-splice to produce circular exons. M Puttaraju, M D Been. Nucleic Acids Res. 1992 Oct 25;20(20):5357-64.). At this point, the two back-splicing sites of the ribozyme are located at the two ends of the exon (3'SS and 5'SS), so the autocatalytic back-splicing reaction will produce a circularized exon. By replacing the sequence between the two splicing sites with a target gene, circularization of the target gene can be achieved. The design principle of a classic PIE circularization construct is shown in Figure 1.

In the present invention, an improved high-efficiency and high-precision PIE circularization system based on poly stem-loop secondary structure (High-efficient and Scarless PIE, hereinafter referred to as Hi-scarless-PIE) is provided. Based on the secondary structure characteristics of the target sequence, an appropriate splicing site is selected, and the Group I intron ribozyme is correspondingly modified to construct a circular RNA precursor molecule. This molecule undergoes autocatalytic splicing under suitable conditions, and the formed circular RNA does not contain any sequence other than the target sequence (e.g., exon sequences or spacer sequences from the ribozyme), ensuring sequence accuracy during RNA circularization. Experiments prove that this system has high efficiency in circularizing RNA, and the circularization effect is not affected by the target sequence, making it applicable for circularizing any RNA. Figure 2 shows the brief design principle of Hi-scarless-PIE.

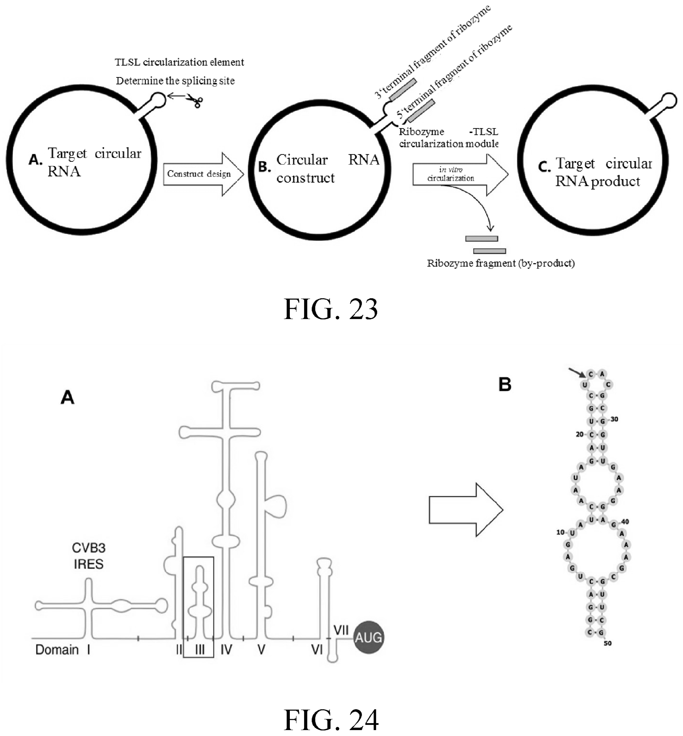

In the present invention, a universal, high-efficiency, high-fidelity RNA circularization platform (Universal & High-efficient & High-fidelity RNA Circularization Platform, i.e., Uni-cRNA) is also provided. Uni-cRNA selects an appropriate splicing site based on the single stem-loop secondary structure characteristics of the target sequence, and correspondingly modifies the Group I intron ribozyme to construct a circular RNA precursor molecule. This molecule undergoes autocatalytic splicing under suitable conditions, and the formed circular RNA does not contain any sequence other than the target sequence (e.g., exon sequences or spacer sequences from the ribozyme), ensuring high sequence fidelity during RNA circularization (i.e., the sequence of the circular RNA product is completely identical to the target sequence). Experiments have demonstrated from multiple perspectives that this system can prepare target circular RNA with high circularization efficiency, and the circularization efficiency is not affected by the target sequence, enabling circularization of any RNA. Figure 23 shows the brief design principle of Uni-cRNA.

### Circular RNA Precursor Molecule of the Present Invention

Based on the above Hi-scarless-PIE or Uni-cRNA strategies, the circular RNA precursor molecule of the present invention is designed, comprising, from the 5' end to the 3' end, operably linked module Z1, module L, and module Z2, as shown in Formula I:

Z1-L-Z2 (Formula I)

Z1 comprises the 3' end fragment h1 of the ribozyme;
L is the linear counterpart of the target sequence;
Z2 comprises the 5' end fragment h2 of the ribozyme.

The circular RNA precursor molecule can undergo autocatalytic splicing reaction to generate the circular RNA molecule of the present invention.

In some embodiments, the Z1 further comprises the 3' end fragment h1 of the ribozyme and a random sequence r1, and the Z2 further comprises the 5' end fragment h2 of the ribozyme and a random sequence r2, and the structure formula of the polynucleotide is as shown in Formula II:

r1-h1-L-h2-r2 (Formula II)

wherein r1 and r2 are reverse complementary sequences.

In the present invention, module L is determined by the following method:
(1) first, directly connecting the 5' and 3' ends of the linear target sequence to be circularized to form a target circular RNA sequence.
(2) second, determining the splicing site (SS) of the target circular sequence. The splicing site is located on the distal loop of the SL structure element or the TLSL structure element. The polynucleotide sequences on both sides of the splicing site are ribozyme recognition sites.
(3) splitting the target circular sequence at the selected splicing site SS to form module L.

### TLSL Structure Element

In the Hi-scarless-PIE circularization strategy of the present invention, the splicing site is located in the TLSL structure element of the target sequence.

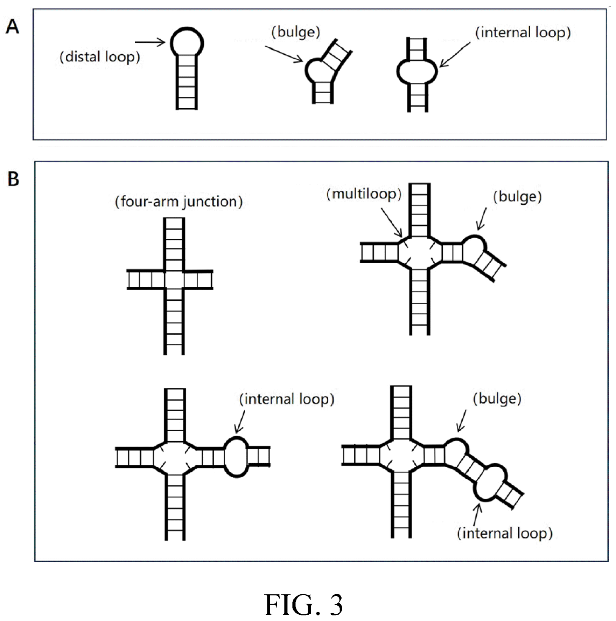

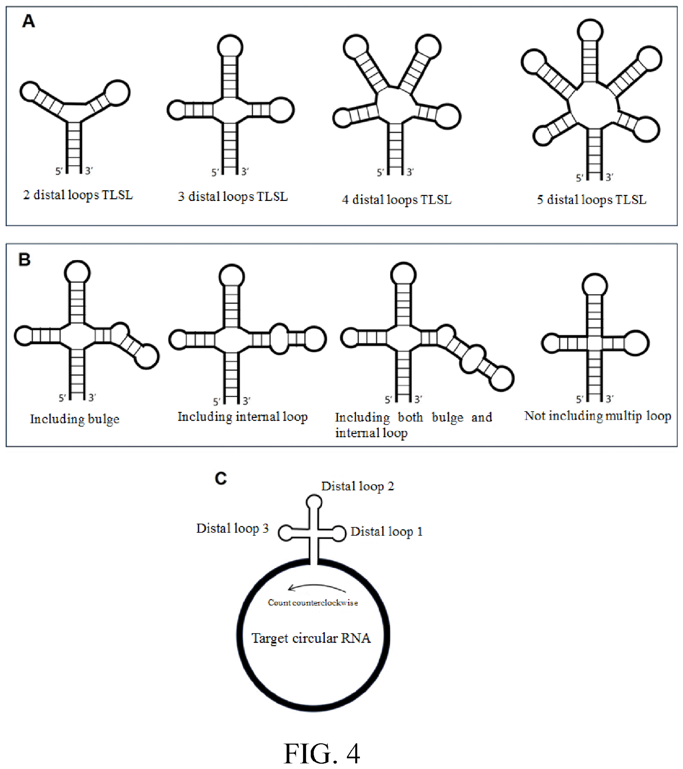

The TLSL structure of the present invention is shown in Figure 3 and Figure 4, consisting of a multi-arm junction connected to multiple distal loops, wherein one arm of the multi-arm junction is a terminal arm, the terminal arm is not connected to a distal loop, and each of the other arms is respectively connected to a distal loop to form a closed connection, forming a continuous multi-distal-loop structure with exposed 5' and 3' ends only on the terminal arm. The arms of the multi-arm junction may comprise bulges and internal loops, and the junction of the multi-arm junction may comprise a multiloop.

In one embodiment, the TLSL structure is a TLSL structure containing 2-5 distal loops.

In one embodiment, the splicing site is located on any one distal loop of the TLSL structure.

In one embodiment, the size of the distal loop is ≥4 nt.

In some embodiments, the TLSL structure module is located at a translation initiation element. The TLSL may be naturally present in the translation initiation element or may be artificially inserted into the translation initiation element. The translation initiation element is a cap-independent translation initiation element, including but not limited to: Internal Ribosome Entry Site (IRES), Kozak sequence, translation initiation element containing a methylation modification site, cap-independent translation enhancers (CITE), 5' untranslated region (5'-UTR), 3' untranslated region (3'-UTR), other elements with cap-independent translation initiation activity, and other newly discovered elements with cap-independent translation initiation activity in the future. Among them, mammalian mRNA genes containing CITE include but are not limited to: human Fibroblast growth factor 9 (FGF-9).

In some embodiments, the cap-independent translation initiation element is an internal ribosome entry site (IRES). IRES sequences usually contain a fragment that can fold into a structure resembling initiator tRNA; this structure element is a typical TLSL structure that can mediate ribosome RNA binding, thereby enhancing the initiation of protein translation. A large number of IRES sequences possess conserved TLSL structure modules that can be recognized by the ribozyme of the present invention.

In some embodiments, the IRES sequence is a non-natural sequence. In some embodiments, the IRES sequence is an IRES sequence into which a TLSL module has been artificially inserted.

### SL Structure Element

In the Uni-cRNA circularization strategy of the present invention, the splicing site is located in the SL structure element of the target sequence.

As used herein, the terms "SL structure," "Stem loop structure," and "single stem-loop structure" are interchangeable and all refer to a single stem-loop secondary structure contained in the target circular RNA molecule. The SL structure is composed of a stem region (also called an "arm") and a distal loop connected. The stem region may comprise bulges and/or internal loops.

In some embodiments, the size of the distal loop is ≥4 nt, preferably 4-25 nt, more preferably 4-20 nt, most preferably 5-15 nt.

In some embodiments, for an SL structure, the stem region is composed of two nucleotide fragments that are fully or partially complementary. The distal end of each nucleotide fragment is connected to the distal loop, while the proximal end of each nucleotide fragment is respectively connected to the main body part of the polynucleotide for preparing circular RNA of the present invention (i.e., the remaining part of the target circular RNA after removing the SL structure). When the lengths of the two nucleotide fragments are Y1 and Y2 respectively, the length Y of the stem region = (Y1+Y2)/2. Typically, this length Y is not particularly limited. Typically, Y is 4-100 nt, more preferably 6-80 nt, most preferably 6-40 nt. Generally, when the two nucleotide fragments are fully complementary, Y can be shorter, such as 3-10 nt; when the two nucleotide fragments are not fully complementary, Y can be longer, such as 4-20 nt or 20-60 nt.

In some embodiments, the number of complementary base pairs P in the stem region is usually ≥3, preferably P is 5-100, more preferably 10-100.

In some embodiments, the ratio of the number of complementary base pairs (P) to the stem region length (Y) (i.e., P/Y) is 40% to 100%, preferably 50% to 100%, more preferably 60% to 95%.

An exemplary SL structure is shown in Figure 3B, where the lengths Y1 and Y2 of the two nucleotide fragments in the stem region are 23 and 23 respectively, the stem region length Y = (23+23)/2 = 23, the number of complementary base pairs P = 13, and the P/Y ratio is 57%.

In some embodiments, in an SL structure of the present invention, the number of the bulges and/or internal loops is 0-4, preferably 0-3, more preferably 0-2.

The SL structure elements of the present invention are widely present in different types of RNA molecules. Stem-loop structures exist in almost any natural RNA molecule and are one of the most fundamental elements constituting complex RNA secondary structures and further forming three-dimensional conformations. The present invention creatively designs a self-splicing ribozyme system capable of efficiently splicing RNA sequences characterized by secondary structures having stem-loop structures.

In the present invention, based on the secondary structure of the target circular sequence, a splicing site is selected on the SL structure element.

In some embodiments, the splicing site is located on the distal loop of the SL structure.

In some embodiments, the splicing site is located between nucleotide U (or T) on the distal loop and an adjacent nucleotide, and the nucleotide U (or T) is located at the 3' end of the linear RNA sequence formed after splitting at the splicing site.

In some embodiments, the target circular RNA molecule is a target circular mRNA molecule, and the SL structure is located in the coding region or non-coding region of the target circular mRNA molecule.

In some embodiments, the SL structure is located at a translation initiation element of the target circular mRNA molecule. The SL structure may be naturally present in the translation initiation element or may be artificially inserted into the translation initiation element. The translation initiation element is a cap-independent translation initiation element, including but not limited to: Internal Ribosome Entry Site (IRES), Kozak sequence, translation initiation element containing a methylation modification site, cap-independent translation enhancers (CITE), 5' untranslated region (5'-UTR), 3' untranslated region (3'-UTR), other elements with cap-independent translation initiation activity, and other newly discovered elements with cap-independent translation initiation activity in the future. Among them, mammalian mRNA genes containing CITE include but are not limited to: human Fibroblast growth factor 9 (FGF-9).

In some embodiments, the translation initiation element is an internal ribosome entry site (IRES). IRES are highly structured regions, forming abundant secondary structures internally. Almost any IRES contains an SL structure that can be recognized by the ribozyme of the present invention.

In some embodiments, the IRES sequences of the present invention include but are not limited to IRES sequences derived from the following species or genes: Coxsackievirus, Echovirus, Poliovirus, Rhinovirus, Human rhinovirus, Cricket paralysis virus, Bovine enterovirus, Enterovirus, Human poliovirus, Encephalomyocarditis virus, Hepatitis C virus, Hepatitis A virus, Simian virus, Aphid lethal paralysis virus, Reticuloendotheliosis virus, Theiler's murine encephalomyelitis virus, Human parechovirus, Bovine viral diarrhea virus, Foot-and-mouth disease virus, Kashmir bee virus, Acute bee paralysis virus, Rhopalosiphum padi virus, Taura syndrome virus, GB virus C, Bovine rhinitis B virus, Kobuvirus, Picornavirus, Swine vesicular disease virus. Human FGF2, Human SFTPA1, Human AML1/RUNX1, Human AQP4, Human AT1R, Human BAG-1, Human BCL2, Human BiP, Human c-IAPl, Human c-myc, Human eIF4G, Human LEF1, Human n-myc, Human p27kipl, Human PDGF2/c-sis, Human p53, Human Pim-1, Human UNR, Human VEGF-A, Human XIAP, Human c-src, Human FGF-1, Drosophila antennapedia, Mouse NDST4L, Mouse HIF1α, Mouse Gtx, Mouse Rbm3, Mouse UtrA, Canine Scamper, Drosophila reaper, Drosophila Ubx, Drosophila hairless, Saccharomyces cerevisiae TFIID, Saccharomyces cerevisiae YAP1, or aptamers of eIF4G.

In some embodiments, the IRES sequence is a non-natural sequence.

In some embodiments, the IRES sequence is an artificially synthesized IRES sequence containing an SL structure.

In some embodiments, the SL structure is located in the coding region of the target circular mRNA sequence.

In some embodiments, the SL structure is located in the 5'-UTR region of the target circular mRNA sequence.

In some embodiments, the SL structure is located in the 3'-UTR region of the target circular mRNA sequence.

In some embodiments, the target circular RNA sequence is a non-coding RNA (ncRNA).

In some embodiments, the target circular RNA sequence is an RNA aptamer, Riboswitch, Toehold switch, gene editing or RNA editing guide-RNA, snRNA, snoRNA, siRNA, miRNA, piRNA, IncRNA, tRNA, rRNA.

### Ribozyme of the Present Invention

As used herein, the term "ribozyme" refers to an RNA with catalytic activity. The ribozyme in the present invention is a ribozyme capable of autocatalytic splicing. The chemical essence of circularization by self-splicing ribozymes is the breakage and reformation of chemical bonds between the ribozyme's splicing active region and the splicing sites, and the activity of the ribozyme and the kinetics of bond breakage and formation depend on the relative spatial positions of the participating functional modules (Joining the two domains of a Group I ribozyme to form the catalytic core. Tanner MA, Cech TR. Science. 1997 Feb 7;275(5301):847-9.). The ribozyme design of the present invention is based on Group I introns.

A large number of natural Group I introns have been discovered. Although different Group I introns vary greatly in sequence, all Group I introns contain the same core secondary structure skeleton, which constitutes the self-splicing ribozyme (Crystal structure of a Group I intron splicing intermediate. Adams PL, Stahley MR, Gill ML, et al. RNA. 2004 Dec;10(12):1867-87.). The self-splicing activity of Group I intron ribozymes depends on their conserved secondary structure; therefore, all ribozyme systems derived from Group I introns with self-splicing function rely on the same self-splicing reaction mechanism (Joining the two domains of a Group I ribozyme to form the catalytic core. Tanner MA, Cech TR. Science. 1997 Feb 7;275(5301):847-9. Crystal structure of a self-splicing Group I intron with both exons. Peter L Adams, Mary R Stahley, Anne B Kosek, et al. Nature. 2004 Jul 1;430(6995):45-50.).

Specifically, the main mechanism of the self-splicing reaction of Group I introns is as follows: The intron located between the upstream exon Exon1 and the downstream exon Exon2 can undergo an autocatalytic splicing reaction, excising the intron fragment and ligating Exon1 and Exon2. The splicing reaction mechanism of Group I introns mainly involves two esterification steps. A sequence at the 3' end of Exon1 forms base complementary pairing with a sequence at the 5' end of the intron; this pairing is called helix P1. In helix P1, the sequence on the ribozyme P1 is called the internal guide sequence (IGS). The G-U mismatch formed by the IGS and the first nucleotide of Exon1 is crucial for the first esterification reaction; the ribozyme relies on this G-U mismatch to precisely recognize the splicing site of the upstream exon (5' splicing site, 5'SS). In the first esterification step, the 3'-OH of a free guanosine or guanylate attacks the 5'SS, causing the exon and intron to be cleaved at the 5'SS, and the exon 5'SS exposes a free 3'-OH for use in the second esterification step. In the second step, the free 3'-OH of the 5'SS acts as a nucleophile to attack the phosphodiester bond at the splicing site (3' splicing site, 3'SS) at the 5' end of Exon2, causing the intron to be excised from Exon2, while a new phosphodiester bond is formed between the 3' end of Exon1 and the 5' end of Exon2, ligating Exon1 and Exon2. Precise recognition of the 5'SS must depend on the formation of helix P1 between the intron and the exon. In contrast, whether complementary pairing is formed between the intron and Exon2 is not a necessary condition for precise recognition of the 3'SS. Therefore, the design of the ribozyme in the present invention mainly focuses on modifying the P1 of the Group I intron. On the exon, the sequence at the 5'SS end that forms complementary pairing (i.e., helix P1) with the ribozyme P1 is also called the ribozyme recognition site. The ribozyme recognition site comprises from the first nucleotide at the 5' end of the exon to the last complementary paired nucleotide in helix P1. The length of the ribozyme recognition site required for an efficient autocatalytic splicing reaction varies among different Group I introns.

In some embodiments, the ribozyme of the present invention is a ribozyme obtained by mutating key regions based on a natural ribozyme.

In some embodiments, the IGS in the natural ribozyme is mutated to form a modified ribozyme.

In some embodiments, the IGS in the natural ribozyme is mutated such that the mutated IGS forms multiple base complementary pairs with the ribozyme recognition site. In these embodiments, the present inventors discovered for the first time that when the Anabaena intron IGS forms 2-7 nt complementary pairing with the ribozyme recognition site (i.e., the length of the ribozyme recognition site is within the range of 2-7 nt), an efficient autocatalytic splicing reaction can be achieved. However, when the length of the ribozyme recognition site is 8 nt, the circularization efficiency is significantly reduced, possibly because the structure of the P2 module is affected, thereby impacting ribozyme activity.

In some embodiments, the ribozyme of the present invention is a modified ribozyme obtained by partially or completely mutating positions 10 to 16 at the 5' end of the Anabaena pre-tRNA-Leu gene intron (abbreviated as Ana-PIE). Wherein, the mutation rule is: the base at position 10 at the 5' end of the PIE intron is mutated to the complementary base of the base at position 2 of the 3' end of the sequence to be circularized, the base at position 11 is mutated to the complementary base of the base at position 3 of the 3' end of the sequence to be circularized, the base at position 12 is mutated to the complementary base of the base at position 4 of the 3' end of the sequence to be circularized, the base at position 13 is mutated to the complementary base of the base at position 5 of the 3' end of the sequence to be circularized, the base at position 14 is mutated to the complementary base of the base at position 6 of the 3' end of the sequence to be circularized, the base at position 15 is mutated to the complementary base of the base at position 7 of the 3' end of the sequence to be circularized, and the base at position 16 is mutated to the complementary base of the base at position 8 of the 3' end of the sequence to be circularized.

In some embodiments, positions 10 and 11 at the 5' end of the Anabaena-derived intron Ana-PIE are mutated.

In some embodiments, positions 10, 11, and 12 at the 5' end of the Anabaena-derived intron Ana-PIE are mutated.

In some embodiments, positions 10, 11, 12, and 13 at the 5' end of the Anabaena-derived intron Ana-PIE are mutated.

In some embodiments, positions 10, 11, 12, 13, and 14 at the 5' end of the Anabaena-derived intron Ana-PIE are mutated.

### Circular RNA of the Present Invention

As used herein, the terms "circular RNA" and "circRNA" are interchangeable and refer to a class of molecules with a covalently closed continuous loop structure without 5' or 3' ends. Due to this special "circular" structure, circular RNA molecules are resistant to degradation by RNases and exhibit higher stability both *in vivo* and *in vitro* compared to linear RNA molecules.

The circular RNA of the present invention is formed by a self-splicing reaction of the circular RNA precursor of the present invention. In some embodiments, the circular RNA is a circular mRNA, and the circular mRNA is capable of efficiently expressing protein in mammals.

In some embodiments, the circular RNA is a circular non-coding RNA (ncRNA).

The circular RNA of the present invention is prepared by the following method:
(1) obtaining the circular RNA precursor molecule of the present invention, which can be obtained by chemical synthesis or by transcription from a vector.

In some embodiments, the circular RNA precursor molecule of the present invention is directly obtained by chemical synthesis.

In some embodiments, the circular RNA precursor molecule of the present invention is obtained by transcription from a vector. The steps are:
a) constructing a plasmid vector containing the sequence of the circular RNA precursor molecule;
b) transcribing the plasmid vector to produce the circular RNA precursor molecule.

(2) Exposing the circular RNA precursor molecule obtained in step (1) to certain reaction conditions to undergo a self-splicing reaction to obtain the target circular RNA.

In some embodiments, the reaction conditions are a reaction system containing GTP and magnesium ions.

In some embodiments, the reaction conditions are a reaction system containing 0.01 mM to 10 mM GTP, 1 mM to 100 mM magnesium ions, the pH of the reaction system is between 5 and 8, the temperature at which the circularization reaction occurs is between 25 and 65°C, and the time for the circularization reaction to proceed *in vitro* is about 5 min to 60 min.

In some embodiments, the reaction conditions are 2±1 mM GTP and a buffer containing magnesium ions (50 mM Tris HCl, 10 mM MgCl₂, 1 mM DTT, pH 7.5), reacted at 55±3°C for 15±5 minutes.

### The main advantages of the present invention include:

(1) The circularization platform has excellent universality; the circularization effect is not affected by different target sequences, enabling high-fidelity and high-efficiency circularization for diverse RNA sequences. The core circularization module of the circular RNA construct of the present invention is a single stem-loop structure or a poly stem-loop structure (collectively referred to as stem-loop structures). The stem-loop structures of the present invention can be widely present in any type of RNA molecule (including various long-chain and short-chain RNA molecules), especially the single stem-loop structure, which is more widely distributed, endowing the Uni-cRNA circularization platform of the present invention with strong universality.
(2) The circularization reaction does not introduce any extra sequences; in the final product of the circular RNA, the linear RNA sequence to be circularized is completely identical to the generated circular RNA sequence. In biomedical applications, ensuring the sequence of the RNA molecule remains unchanged after circularization can avoid potential risks caused by sequence alterations.
(3) High circularization efficiency: The Uni-cRNA circularization efficiency for single stem-loop structures of the present invention can be as high as about 80% or higher, while the Hi-scarless-PIE circularization efficiency for poly stem-loop structures of the present invention is about 90-95% or higher. The circularization efficiencies for both types of stem-loop structures are unexpectedly high, facilitating downstream processes after circularization and demonstrating excellent practicality.
(4) The prepared circular mRNA has extended stability, a long *in vivo* half-life, and significantly increased protein expression efficiency. Circular RNA molecules persist longer in the body and translate proteins for a longer duration, thus exhibiting better druggability properties than linear mRNA, laying the foundation for preparing circular RNA products for pharmaceutical applications.
(5) The circular RNA molecules prepared by the present invention have lower immunogenicity. Because they do not carry extra sequences, the circular RNA of the present invention has high purity, which is beneficial for reducing its immunogenicity.

The present invention is further elaborated below in conjunction with specific examples. It should be understood that these examples are only for illustrating the present invention and are not intended to limit the scope of the present invention. For experimental methods in the following examples where specific conditions are not indicated, they are usually carried out under conventional conditions, such as those described in Sambrook et al., Molecular Cloning: A Laboratory Manual (New York: Cold Spring Harbor Laboratory Press, 1989), or under conditions suggested by the manufacturer. Unless otherwise indicated, percentages and parts are by weight percentages and parts by weight.

### Example 1. Design and Verification of Hi-Scarless-PIE Circular RNA Constructs

This example relates to the process and method of identifying a TLSL circularization element, modifying the Ana-PIE intron ribozyme, forming a "ribozyme-TLSL circularization module," and further composing a Hi-Scarless-PIE circular RNA construct, and experimentally verifies the effect of the construct in synthesizing circular RNA.

The design principle of the Hi-Scarless-PIE circular RNA construct in the present invention is shown in Figure 2.

The TLSL circularization element consists of the ribozyme recognition site and its nearby secondary structure.

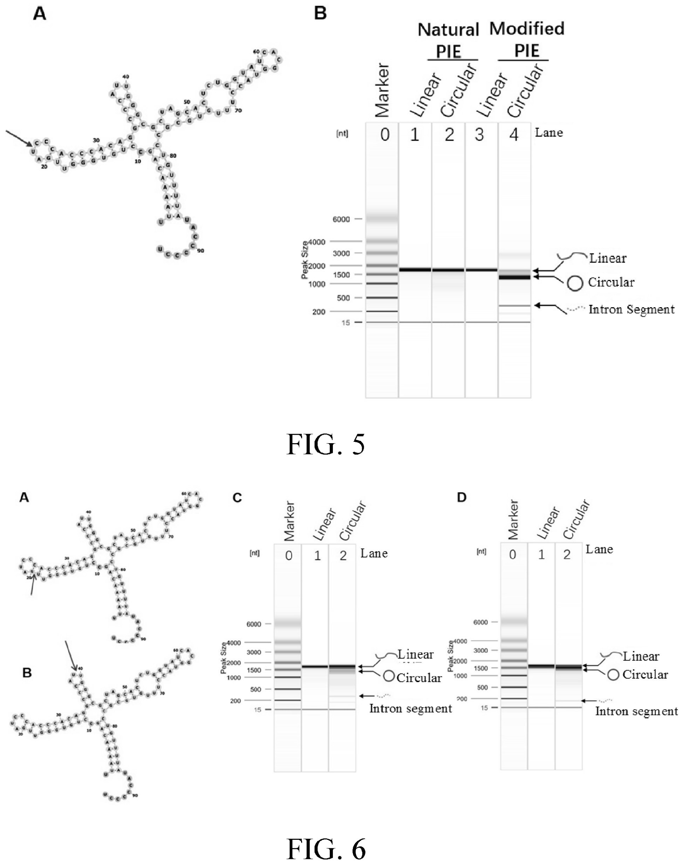

First, the TLSL circularization module was obtained through the following steps: In CVB3 IRES (SEQ ID NO: 1), based on the secondary structure, it was determined that the TLSL circularization element is located in the nucleotide sequence from positions 1 to 94 of CVB3 IRES, named TLSL-CVB3-IRES (SEQ ID NO: 2). Its secondary structure is shown in Figure 5A.

Second, the splicing site was selected: The splicing site is located between U at position 22 and C at position 23 of the TLSL-CVB3-IRES sequence. This splicing site is located on the loop of the first stem-loop near the 5' end in the secondary structure of TLSL-CVB3-IRES. The arrow in Figure 5A indicates the position of the splicing site.

In this example, the target circular RNA sequence (SEQ ID NO: 4) was composed of CVB3-IRES (SEQ ID NO: 1) and the mRNA coding region of Gaussia Luciferase (GLuc) (SEQ ID NO: 3). According to the selected splicing site, the Seq 4 sequence was split at this splicing site to form the linear counterpart of the target circular RNA sequence (SEQ ID NO: 5).

Design of a GLuc circular RNA precursor molecule based on the modified Ana-PIE ribozyme: The 3' end fragment of the Ana-PIE ribozyme (SEQ ID NO: 6), the linear counterpart of the target circular RNA sequence (SEQ ID NO: 5), and the modified 5' end fragment of the Ana-PIE ribozyme (SEQ ID NO: 7) were connected to each other to form a GLuc circular RNA precursor molecule based on the modified Ana-PIE ribozyme, named circ-CVB3-IRES-GLuc-precursor-1 (SEQ ID NO: 8).

Design of a GLuc circular RNA precursor molecule based on the unmodified Ana-PIE ribozyme: The 3' end fragment of the Ana-PIE ribozyme (SEQ ID NO: 6), the linear counterpart of the target circular RNA sequence (SEQ ID NO: 5), and the 5' end fragment of the Ana-PIE ribozyme (SEQ ID NO: 9) were connected to each other to form a GLuc circular RNA precursor molecule based on the unmodified Ana-PIE ribozyme, named circ-CVB3-IRES-GLuc-precursor-2 (SEQ ID NO: 10).

Synthesis of the above-designed circular RNA precursor molecules and constructs: First, DNA sequences corresponding to SEQ ID NO: 1, SEQ ID NO: 3, SEQ ID NO: 6, and SEQ ID NO: 9 were synthesized (Beijing Tsingke Biotech Co., Ltd.). These DNA sequences were cloned via molecular cloning methods into a linear plasmid vector pUC57 (GenBuilder Plus Cloning Kit, L00744, GenScript Biotech Corporation) containing a T7 promoter generated by PCR, to obtain the corresponding constructs (i.e., the construct using the native Ana-PIE intron). The following plasmid was obtained: pUC57-CVB3-GLuc-1. Based on this construct, the sequence of the Ana-PIE ribozyme 5' end fragment (SEQ ID NO: 9) was modified, wherein A at position 10 of the 5' end of SEQ ID NO: 9 was mutated to T, and G at position 11 was mutated to C, forming the modified Ana-PIE intron construct pUC57-CVB3-GLuc-2.

Preparation of linear plasmid template: The plasmid was transformed into Top10 competent cells, plated, and the next day clones were picked and expanded in LB medium containing Amp resistance (37°C/200 rpm/overnight). Then plasmid extraction was performed (Endo-Free Plasmid Mini Kit II, DP118, Tiangen Biotech (Beijing) Co., Ltd.). Subsequently, the linearized plasmid template was prepared by digestion with EcorI (1040, Takara) (37°C, 1 hour). The digestion product was recovered using Monarch^{®} PCR & DNA Cleanup Kit (T1030, New England Biolabs), concentration was determined by Nano-Drop (Thermo), and the digestion product was identified by 1.5% agarose gel electrophoresis. The purified linear plasmid served as the template for *in vitro* transcription.

Preparation of circRNA precursor by *in vitro* transcription: *In vitro* transcription was performed using the HiScribe^{®} T7 High Yield RNA Synthesis Kit (E2040S, New England Biolabs) on the linearized plasmid DNA template (37°C, 2 hours) to synthesize the circRNA precursor. After *in vitro* transcription, the linear DNA template was digested with DNase I (M0303S, New England Biolabs) (37°C, 15 min). Then, the above product was column-purified using Monarch^{®} RNA Cleanup Kit (T2050, New England Biolabs).

circRNA synthesis: The circular RNA precursor molecule was added to a buffer (50 mM Tris-HCl, 10 mM MgCl2, 1 mM DTT, pH 7.5) containing GTP (final concentration 2 mM). The solution was heated at 55°C for 15 min, followed by column purification (Monarch^{®} RNA Cleanup Kit, T2050, New England Biolabs).

Identification of circular RNA using capillary electrophoresis: After determining the concentration of the circularized RNA precursor and crude circularized RNA product using Nano-Drop (Thermo Fisher Scientific), they were processed using the QIAxcel RNA High Sensitivity Cartridge Kit (929112) according to the instructions, and then detected and analyzed using the QIAxcel Connect capillary electrophoresis instrument according to the operating instructions. In this experiment, the high-sensitivity RNA marker (QIAxcel RNA High-Sensitivity Marker Set, 929112, QIAGEN) was used to indicate different RNA molecular bands.

Experiment Results: Figure 5A shows the local secondary structure of the ribozyme recognition site. Figure 5B shows the self-splicing reaction of the native Ana-PIE intron and the modified Ana-PIE intron. As shown in Figure 5B, there was no significant difference in the bands between capillary lanes 1 and 2; lane 2 did not produce a significant band at the position of circular RNA, indicating that the native Ana-PIE intron (Natural PIE) could not perform the self-splicing circularization reaction efficiently. In contrast, comparing lanes 3 and 4, most of the bands in lane 4 were located at the position of circular RNA, with only a small amount of linear RNA bands, and there was a clear band of the intron fragment, indicating that the modified Ana-PIE intron group (Modified PIE) underwent a highly efficient self-splicing circularization reaction.

### Example 2. Effect of Self-Splicing Ribozyme Splicing site Selection on Circularization Efficiency

This example detected the impact of different ribozyme splicing sites on circularization efficiency.

To verify the effect of splicing site positions on circularization efficiency, in addition to the splicing site determined in Example 1 (located between U at position 22 and C at position 23 of the TLSL-CVB3-IRES sequence, on the distal loop of the first stem-loop near the 5' end in the secondary structure of TLSL-CVB3-IRES, as indicated by the arrow in Figure 5A), two other splicing sites on TLSL-CVB3-IRES were selected in this example for experimental verification.

Among them, the first splicing site was selected on the stem (specific position indicated by the arrow in Figure 6A). Correspondingly, positions 10 and 11 at the 5' end of the Ana-PIE ribozyme were mutated accordingly, resulting in the circular RNA precursor molecule sequence SEQ ID NO: 11. The second splicing site was selected on a small distal loop (this distal loop contained 3 nucleotides). The specific position of the splicing site is shown by the arrow in Figure 6B. Correspondingly, positions 10 and 11 at the 5' end of the Ana-PIE ribozyme were mutated accordingly, resulting in the circular RNA precursor molecule sequence SEQ ID NO: 12.

The experimental results are shown in Figure 6C. Lanes 0, 1, and 2 are the RNA molecule marker, the linear precursor molecule of circular RNA, and the product of the linear precursor molecule after the circularization reaction, respectively. In capillary lane 2, there was a band at the position of circular RNA, but its brightness was weak, while the band at the linear RNA position was very obvious, proving that only a small amount of circular RNA molecules were formed. Similarly, in Figure 6D, only a small amount of band at the circular RNA position was observed in lane 2, indicating that the circularization efficiency at this splicing site was also low. These results collectively indicate that when the splicing site of the self-splicing ribozyme is on the stem of the TLSL circularization element or on a distal loop but the selected distal loop is small, circularization can be achieved, but the circularization efficiency is significantly reduced. Therefore, the ribozyme splicing site on the TLSL circularization element is preferably located on the distal loop of the stem-loop, and the size of the distal loop should be greater than or equal to 4.

### Example 3. The Hi-Scarless-PIE Circularization System Can Prepare Circular RNA Molecules with Precise Sequences

In this example, multiple methods were used to identify whether the product of the present invention is the target circular RNA molecule. The identification methods include:
Method 1. Identification by RNase R. RNase R is a 3'-5' exoribonuclease that degrades RNA in the 3'-5' direction. It can degrade linear RNA molecules, but circular RNA without 3' and 5' ends cannot be degraded.

Specific steps are as follows: The purified circular RNA or linear circRNA precursor was heated at 65°C for 3 minutes, then immediately cooled on ice for 2 minutes. Then, linear RNA was digested using RNase R (E049, Applied Biological Materials) (37°C, 30 min). After the reaction was terminated, the RNase R-digested RNA was column-purified using Monarch^{®} RNA Cleanup Kit (T2050, New England Biolabs). Then, 1.5% agarose gel was prepared for agarose gel electrophoresis analysis.

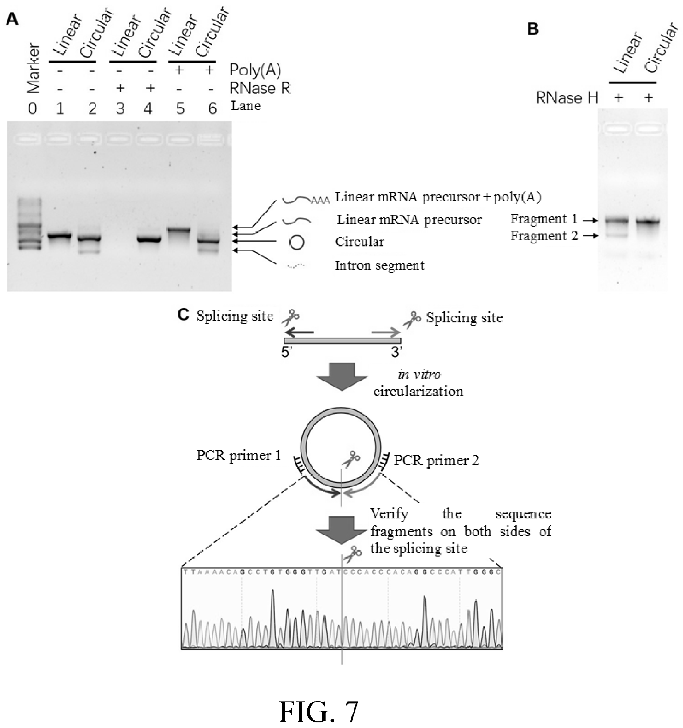

The results are shown in Figure 7A. Lanes 1 and 3 are the linear circRNA precursor (precursor-mRNA, SEQ ID NO: 8) without circularization reaction, lanes 2 and 4 are the products after circularization, and lanes 3 and 4 are the results after RNase R treatment. As shown in Figure 7A, after circularization, the position of the main band in lane 2 was below the band in lane 1, and a distinct band appeared at the very bottom, suggesting that the circularization reaction produced a circular mRNA molecule with reduced molecular weight and a very small intron fragment. The RNase R treatment group showed no band in lane 3, while the main band in lane 4 was not affected by RNase R, indicating that this band was the circular RNA band.

Method 2. Identification by adding Poly(A) fragments. Linear mRNA molecules can be added with hundreds of adenylates (i.e., Poly(A) fragments), increasing their molecular weight. Circular RNA, being closed head-to-tail without a 3' end, cannot be tailed. Therefore, agarose gel electrophoresis can distinguish the size change of RNA before and after tailing to determine whether it is circular RNA.

Specific steps are as follows: The purified circular RNA or linear circRNA precursor was tailed using E. coli Poly(A) Polymerase (M0276, New England Biolabs) (37°C, 30 min). The finished products were then column-purified using Monarch^{®} RNA Cleanup Kit (T2050, New England Biolabs).

The results are shown in Figure 7A. The products after adding Poly(A) to the linear mRNA precursor and the linear mRNA precursor after the circularization reaction were loaded into lanes 5 and 6, respectively. Compared to lane 1, the band in lane 5 (linear mRNA precursor after Poly(A) assay) shifted upward, indicating an increase in molecular weight and successful Poly(A) addition. Compared to lane 4, after the Poly(A) assay on the band presumed to be circular RNA in lane 6, its position was the same as the circular RNA band in lane 4, having the same size, indicating that Poly(A) was not added.

Method 3. Identification using RNase H. RNase H is an endoribonuclease that specifically hydrolyzes RNA in DNA-RNA hybrid strands. Based on the structure difference between linear RNA and circular RNA, after binding the same probe to linear RNA and circular RNA, linear RNA will be cleaved into two bands, while circular RNA will only be cleaved open into linear form, still appearing as one band.

Specific steps are as follows: A 10-fold molar excess of DNA probe was added to the circular RNA obtained after RNase R treatment, then heated at 65°C for 5 min and annealed at room temperature. Digestion was performed with RNase H (M0279, New England Biolabs) at 37°C for 20 minutes, and the digested RNA was column-purified. The DNA probe sequence is SEQ ID NO: 13.

As shown in Figure 7B, after RNase H treatment, the linear mRNA precursor formed two bands, while the circular mRNA molecule formed one band.

Method 4. Verification of ribozyme recognition site sequence accuracy by RT-PCR and Sanger sequencing. The aforementioned methods (1), (2), and (3) can confirm that circular RNA molecules are formed after the circularization reaction, but the accuracy of the ribozyme recognition site sequence needs verification. Therefore, this example also used sequencing across the splicing site to demonstrate that the circularization reaction occurred at the expected position, forming the target circular RNA molecule.

Specific steps are as follows: The circular RNA obtained after RNase R treatment was used with the RevertAid First Strand cDNA Synthesis Kit (K1622, Thermo Fisher Scientific) and Random primers to synthesize the first strand of cDNA. Then, forward and reverse PCR primers were designed upstream and downstream of the splice junction, respectively. Using the cDNA as a template, PCR was performed to obtain an amplified fragment for sequencing verification. The circular mRNA molecule should show the expected RNA sequence spanning the splicing site near the splice junction. Primer sequences are shown in SEQ ID NO: 14 and SEQ ID NO: 15.

The results are shown in Figure 7C. The sequencing results confirmed that the sequences on both sides of the splicing site were consistent with the designed circular RNA sequence, proving that the head and tail of the circular RNA were joined together. The circular mRNA molecule with the correct sequence was successfully obtained.

In summary, this example verified through four complementary methods that the target circular RNA molecule was successfully synthesized in the *in vitro* circularization reaction system of the present invention.

### Example 4. Effect of Different Ribozyme Mutations on Hi-Scarless-PIE Circularization Efficiency

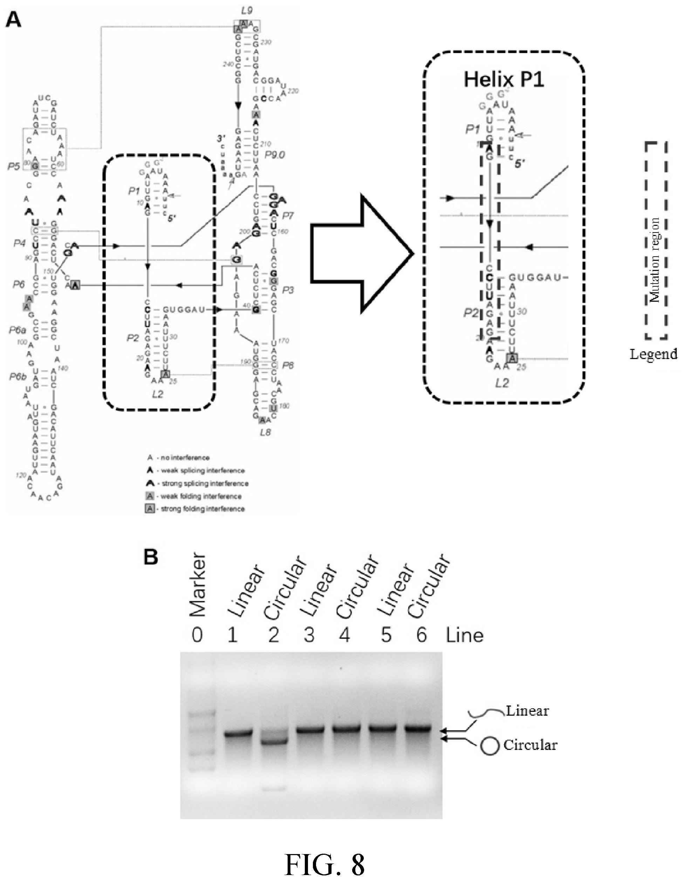

This example determined the impact of different ribozyme modification conditions on Hi-Scarless-PIE circularization efficiency by mutating positions 10-19 at the 5' end of the Anabaena-derived intron accordingly. Figure 8A shows the secondary structure of the native Anabaena intron and the default numbering of nucleotides (the left panel of Figure 8A is cited from the literature: Distinct sites of phosphorothioate substitution interfere with folding and splicing of the Anabaena Group I intron. Lupták A, Doudna JA. Nucleic Acids Res. 2004 Apr 23;32(7):2272-80. doi: 10.1093/nar/gkh548.). An enlarged view of the positions of helix P1 and P2 in the left panel is shown in the right panel of Figure 8A, where the nucleotides within the dashed box are the regions where the native ribozyme is mutated in this example. The nucleotide numbering for this intron in this document follows the labeling method shown in Figure 8A.

The modified ribozyme used in the aforementioned Examples 1, 2, and 3 only mutated positions 10 and 11 at the 5' end of the Anabaena-derived intron accordingly. In this example, positions 10-14, 10-17, and 10-19 at the 5' end of the Anabaena-derived intron in the sequence of circ-CVB3-Gluc-precursor-2 (SEQ ID NO: 10) were mutated accordingly, resulting in the circular RNA precursor molecule sequences as follows: SEQ ID NO: 16, SEQ ID NO: 17, SEQ ID NO: 18. Then, the reaction was carried out according to the method for synthesizing circular RNA described in Example 1, followed by analysis using 1.5% agarose gel electrophoresis.

Experiment Results: The results are shown in Figure 8B. Lanes 1, 3, and 5 are the circular RNA precursor molecules formed after corresponding mutations at positions 10-14, 10-17, and 10-19 at the 5' end of the Anabaena-derived intron, respectively. Lanes 2, 4, and 6 are the circularization products of the precursor molecules after corresponding mutations at positions 10-14, 10-17, and 10-19 at the 5' end of the Anabaena-derived intron, respectively. As shown in Figure 8B, after circularization treatment, the position of the main band below the band in lane 1 was only in the lane 2, and a distinct band appeared at the position of the intron fragment at the very bottom of the lane. In contrast, there were no significant changes in band positions between lane 4 and lane 3, and between lane 6 and lane 5. This indicates that circular RNA molecules can be formed after mutating positions 10-14 at the 5' end of the Anabaena-derived intron in the circular RNA precursor molecule. However, mutating positions 10-17 and 10-19 at the 5' end of the intron may affect some core modules of the ribozyme, thereby impacting its catalytic activity and preventing circular RNA formation.It can be determined from this that, preferably, mutating some or all nucleotides in the region between positions 10-16 at the 5' end of the Anabaena-derived intron in the present invention, so that this region forms partial or complete complementary pairing with the target gene, will ensure that the self-splicing circularization efficiency of the modified ribozyme remains at a high level.

### Example 5. Study on the Universality of the Hi-Scarless-PIE Circularization System Among Different "Ribozyme-TLSL Circularization Modules"

The aforementioned Examples 1, 2, and 3 all used the nucleotide sequence from positions 1 to 94 of CVB3 IRES as the TLSL circularization element (TLSL-CVB3-IRES, SEQ ID NO: 2). To verify that the "ribozyme-TLSL circularization module" of this application is a universal method based on the local secondary structure of the ribozyme recognition site, this example selected multiple TLSL structure elements different from TLSL-CVB3-IRES to verify whether the "ribozyme-TLSL circularization module" composed of them could also achieve efficient scarless circular RNA preparation.

The TLSL structure elements used in this example include: five TLSL structure elements: SEQ ID NO: 19, SEQ ID NO: 20, SEQ ID NO: 21, SEQ ID NO: 22, SEQ ID NO: 23. The nucleotide sequences of these five TLSL elements are different from each other, but they all conform to the description of TLSL structure features in this patent. Among them:
SEQ ID NO: 19 is from HRV-B3 IRES (SEQ ID NO: 24),
SEQ ID NO: 20 is from EV-B107 IRES (SEQ ID NO: 25),
SEQ ID NO: 21 is from PV3 IRES (SEQ ID NO: 26),
SEQ ID NO: 22 is from SimianV4 IRES (SEQ ID NO: 27),
SEQ ID NO: 23 is from BEV1 IRES (SEQ ID NO: 28).

For each TLSL structure element, positions 10 and 11 at the 5' end of the Ana-PIE ribozyme were mutated accordingly. Using the same method as described in Example 1, precursor molecules for circular IRES-GLuc were constructed as follows:
circ-HRV-B3-IRES-GLuc-precursor (SEQ ID NO: 29),
circ-EV-B107-IRES-GLuc-precursor (SEQ ID NO: 30),
circ-PV3-IRES-GLuc-precursor (SEQ ID NO: 31),
circ-SimianV4-IRES-GLuc-precursor (SEQ ID NO: 32),
circ-BEV1-IRES-GLuc-precursor (SEQ ID NO: 33).

Then, using the circular RNA molecule identification method described in Example 3, the effect of preparing circular RNA was verified.

### Results:

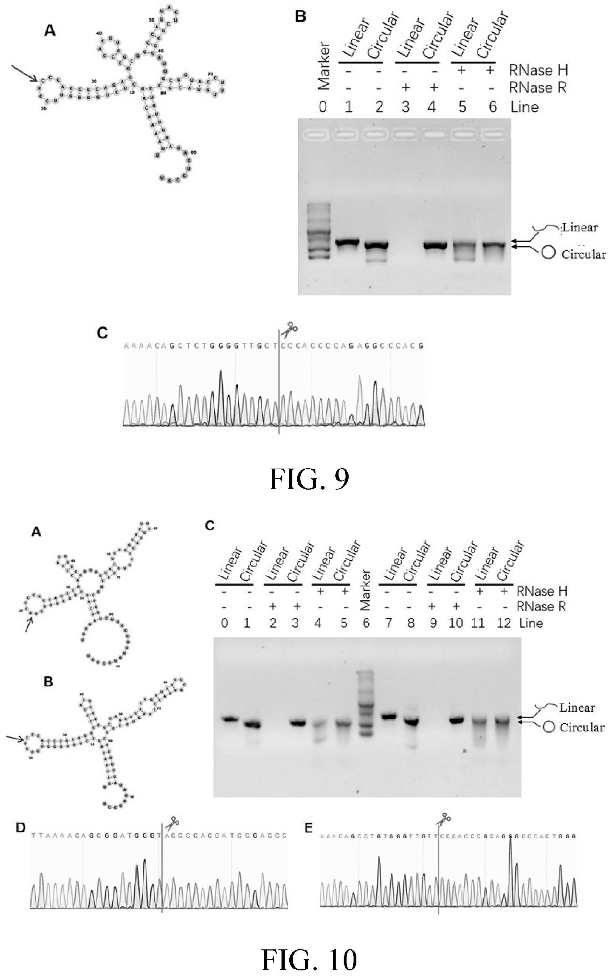

First, Figure 9 shows the experimental results of construct design based on the TLSL structure derived from PV3 IRES and circularization verification. Among them, Figure 9A shows the TLSL structure from PV3 IRES and the selected splicing site (as indicated by the arrow). The verification results are shown in Figure 9B. Lanes 1 and 3 are the linear circRNA precursor (precursor-mRNA) without circularization reaction, lanes 2 and 4 are the products after circularization, and lanes 3 and 4 are the results after RNase R treatment. As shown in Figure 9B, after circularization, the position of the main band in lane 2 was below the band in lane 1, and a distinct band appeared at the very bottom, suggesting that the circularization reaction produced a circular mRNA molecule with reduced molecular weight and a very small intron fragment. In the RNase R treatment group, no band appeared in lane 3, while the band in lane 4, being the circular RNA band, remained unaffected by RNase R. Lane 5 is the linear mRNA precursor after RNase H treatment, and lane 6 is the circular mRNA after RNase H treatment. After RNase H treatment, the linear mRNA precursor formed two bands, while the circular mRNA molecule formed one band. The sequencing results in Figure 9C confirmed that the sequences on both sides of the splicing site were consistent with the designed circular RNA sequence, proving that the head and tail of the circular RNA were joined together. Circular mRNA was successfully obtained.

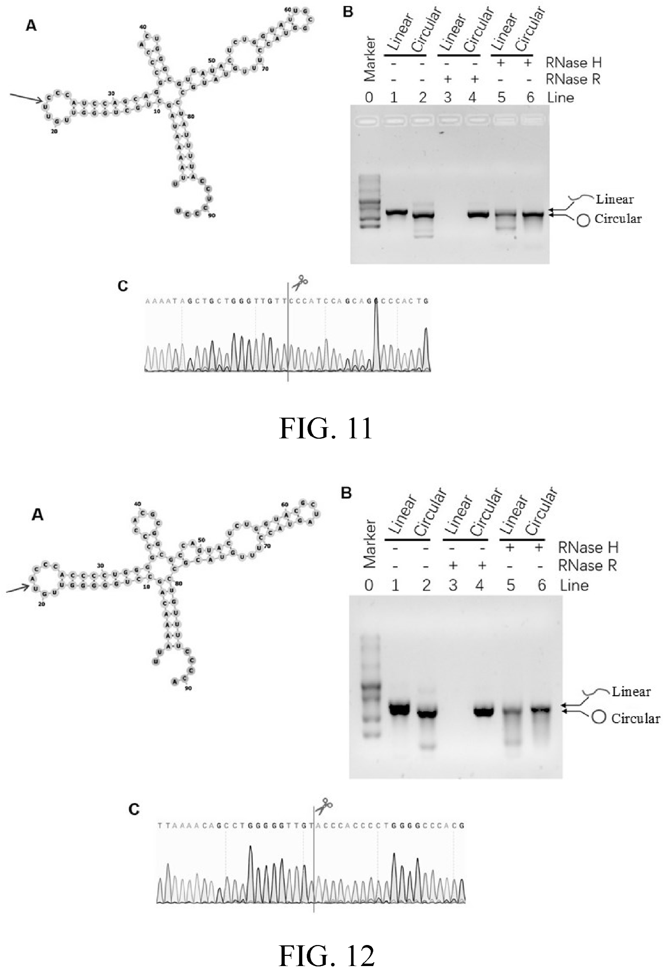

Similarly, Figures 10, 11, and 12 respectively show the search for TLSL structures and selection of splicing sites in different IRES sequences. Among them, Figure 10 shows the experimental results of construct design based on TLSL structures from HRV-B3 IRES and EV-B107 IRES and circularization verification; Figure 11 shows the experimental results of construct design based on the TLSL structure from SimianV4 IRES and circularization verification; Figure 12 shows the experimental results of construct design based on the TLSL structure from BEV1 IRES and circularization verification. The presentation of results is similar to Figure 9. The results in Figures 10, 11, and 12 all indicate that the present invention can successfully and efficiently synthesize circular RNA molecules for different TLSL structures, demonstrating the universality of the Hi-Scarless-PIE system in the present invention.

### Example 6. Practicality Verification of the Hi-Scarless-PIE Circularization System Among Different "Ribozyme-TLSL Circularization Modules"

To further verify the stability of the circularization efficiency of the Hi-Scarless-PIE system among different "ribozyme-TLSL circularization modules," this example quantitatively analyzed the variation in circularization efficiency of different "ribozyme-TLSL circularization modules" by capillary electrophoresis.

Specific experiment method: After determining the concentration of the circular RNA precursor and crude circularized RNA product by Nano-Drop, they were processed using the QIAxcel RNA High Sensitivity Cartridge Kit (929112, QIAGEN) according to the instructions, and then detected and analyzed using the QIAxcel Connect capillary electrophoresis instrument (QIAGEN) according to the operating instructions.

The nucleotide sequences involved in this example are as follows: SEQ ID NO: 29, SEQ ID NO: 30, SEQ ID NO: 31, SEQ ID NO: 32, and SEQ ID NO: 33.

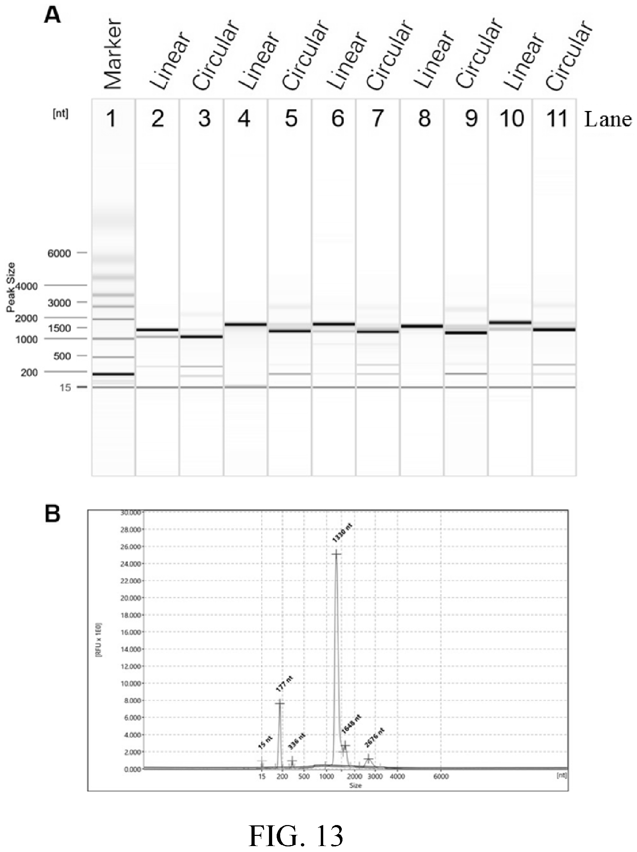

Experiment Effect: Figure 13A shows the detection result of capillary electrophoresis. Lanes 2, 4, 6, 8, and 10 are the circular RNA precursor molecules of SEQ ID NO: 29, SEQ ID NO: 30, SEQ ID NO: 31, SEQ ID NO: 32, and SEQ ID NO: 33, respectively. Lanes 3, 5, 7, 9, and 11 are the products of these circular RNA precursor molecules after the circularization reaction. Empirically, after circularization, the molecular weight of the circular RNA is smaller than that of its linear precursor molecule, and the corresponding band position shifts downward. In addition, the circularization product contains intron peaks and intron dimer peaks, and may contain circular RNA dimer peaks. Figure 13B shows the peak profile of the crude RNA product after the circularization reaction of the circular RNA precursor (SEQ ID NO: 30). Combining Figures 13A and 13B, after circularization of the circular RNA precursor (SEQ ID NO: 30), the molecular weight of the RNA decreased, and the corresponding band position shifted downward (from 1648 nt to 1330 nt), with a 177 nt intron peak and a 336 nt intron dimer peak, as well as a 2676 nt circular RNA dimer peak (due to the capillary electrophoresis system itself, the molecular weight marked by the system may have a slight error compared to the actual molecular weight of the bands). Compared to lanes 2, 4, 6, 8, and 10, most of the bands in lanes 3, 5, 7, 9, and 11 were located at the position of circular RNA, while only a small amount of linear RNA bands were present at the linear RNA position. Circularization efficiency was calculated using the software (QIAxcel ScreenGel 2.0) that comes with the QIAxcel Connect capillary electrophoresis instrument. Here, circularization efficiency = 100% * circular / (linear + circular). The results showed that the circularization efficiencies of SEQ ID NO: 29, SEQ ID NO: 30, SEQ ID NO: 31, SEQ ID NO: 32, and SEQ ID NO: 33 all reached above 90%, specifically: 95.45%, 91.58%, 93.90%, 90.03%, and 92.14%. These results collectively indicate that the Hi-Scarless-PIE system disclosed in the present invention can efficiently circularize (circularization efficiency above 90%) different target sequences.

| **Circular RNA Precursor** | **SEQ ID NO:29** | **SEQ ID NO:30** | **SEQ ID NO:31** | **SEQ ID NO:32** | **SEQ ID NO:33** |
|---|---|---|---|---|---|
| | | | | | |
| **Circular (pg/µl)** | 14178.73 | 12075.06 | 14176.71 | 9779.56 | 14895.51 |
| **Linear(pg/µl)** | 675.56 | 1110.23 | 921.74 | 1082.63 | 1270.84 |
| **Cyclization Efficiency (%)** | 95.45 | 91.58 | 93.90 | 90.03 | 92.14 |

### Example 7. Modular Design of Hi-Scarless-PIE Circular RNA Constructs Using Predefined TLSL Circularization Elements

Considering that not all RNA molecules contain TLSL structure elements, this example verified whether a validated TLSL circularization element could be inserted into a target RNA molecule lacking such an element, enabling efficient circularization using the Hi-Scarless-PIE system. This example used mRNA as a case study for verification. It has been reported that TLSL structure elements in IRES can recruit ribosomes and thus promote protein translation (Structure basis for cloverleaf RNA-initiated viral genome replication. Keerthi Gottipati, Sean C McNeme, Jerricho Tipo, et al. Nucleic Acids Res. 2023 Sep 8;51(16):8850-8863.). Therefore, this example inserted a TLSL circularization element into an IRES to verify, on one hand, whether efficient circularization via Hi-Scarless-PIE is possible after insertion, and on the other hand, whether inserting the TLSL structure element into the IRES could enhance the protein translation level of the circular mRNA.

### Experimental Method:

In this example, the TLSL structure element (TLSL-CVB3-IRES: SEQ ID NO: 2) was inserted at the 5' end of EMCV IRES (SEQ ID NO: 34) and CrPV IRES (SEQ ID NO: 35), respectively, forming new sequences containing the TLSL structure element, named: EMCV+TLSL (SEQ ID NO: 36) and CrPV+TLSL (SEQ ID NO: 37). Then, the target circular RNA sequence (SEQ ID NO: 38) was composed of EMCV+TLSL (SEQ ID NO: 36) and the mRNA coding region of Gaussia Luciferase (GLuc) (SEQ ID NO: 3), and the target circular RNA sequence (SEQ ID NO: 39) was composed of CrPV+TLSL (SEQ ID NO: 37) and the mRNA coding region of Gaussia Luciferase (GLuc) (SEQ ID NO: 3). The 3' end fragment of the Ana-PIE ribozyme (SEQ ID NO: 6), the linear counterparts of the target circular RNA sequences (SEQ ID NO: 38 and SEQ ID NO: 39), and the modified 5' end fragment of the Ana-PIE ribozyme (SEQ ID NO: 7) were connected to each other, forming the corresponding constructs: circ-EMCV+TLSL-IRES-GLuc-precursor (SEQ ID NO: 40) and circ-CrPV+TLSL-IRES-GLuc-precursor (SEQ ID NO: 41). Then, according to the method described in Example 1, the circular RNA precursor molecules were synthesized and circular RNA was prepared *in vitro.* Subsequently, 1.5% agarose gel was prepared for agarose gel electrophoresis analysis. At the same time, circular RNA prepared by the classic PIE system (i.e., circular RNA molecules without the inserted TLSL circularization element) were used as controls (classic PIE-EMCV-IRES-GLuc-precursor: SEQ ID NO: 42 and classic PIE-CrPV-IRES-GLuc-precursor: SEQ ID NO: 43).

Cell Culture: HEK-293T cells were cultured in a 37°C, 5% CO2 incubator (Thermo Fisher Scientific). HEK-293T cells were cultured in high-glucose DMEM medium (L110KJ, Shanghai Yuanpei Biotechnology Co., Ltd.) containing 10% fetal bovine serum (SH30396.03, Hyclone) and 1% penicillin-streptomycin (S110JV, Shanghai Yuanpei Biotechnology Co., Ltd.). Cells were subcultured every 2-3 days.

Cell Transfection: Before transfection, HEK-293T cells were seeded in 96-well plates at 20,000 cells/well. Then 50-100 ng (equimolar amounts of each RNA) of the circular mRNA molecules prepared according to the method in Example 1 were transfected into HEK-293T cells using Lipofectamine 3000 transfection reagent (L3000001, Thermo Fisher Scientific) according to the instructions.

Protein Expression Analysis: 24 hours post-transfection, 10 microliters of culture supernatant were taken, and the luciferase activity in the supernatant was measured using the Pierce Gaussia Luciferase Glow Assay Kit (Thermo Fisher Scientific). Luciferase activity was measured using a SpectraMax i3 microplate reader (Molecular Devices).

### Results:

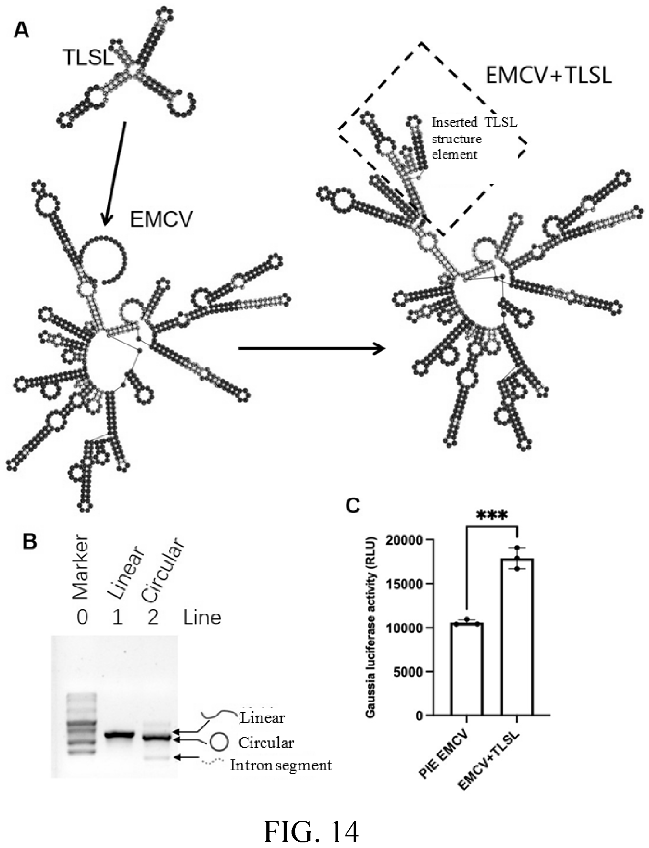

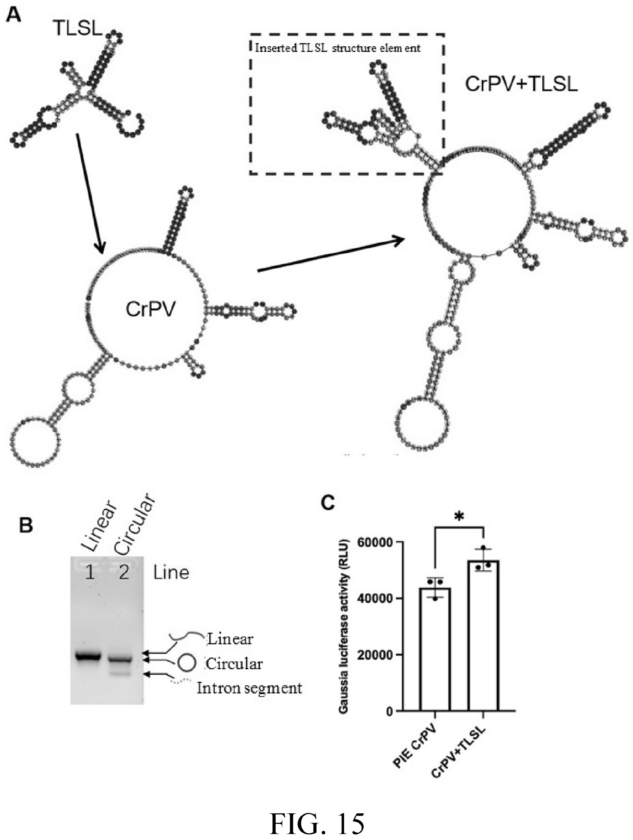

Figure 14A shows the secondary structures of TLSL, EMCV IRES, and EMCV+TLSL. Figure 15A shows the secondary structures of TLSL, CrPV IRES, and CrPV+TLSL. From the secondary structures, it can be seen that the sequence after inserting TLSL formed the TLSL structure element at the expected position, and this structure element did not affect other secondary structure regions on the IRES. The electrophoresis detection results are shown in the agarose gel results in Figures 14B and 15B. Lane 1 in each figure is the linear circRNA precursor (precursor-mRNA) without circularization reaction, and lane 2 is the product after circularization. After circularization treatment, the position of the main band in lane 2 significantly shifted downward, and a distinct band appeared at the very bottom, suggesting that the circularization reaction produced a circular mRNA molecule with reduced molecular weight and a very small intron fragment. This demonstrates that after inserting the TLSL circularization element, efficient circularization using Hi-Scarless-PIE is possible. As shown in the protein expression results in Figures 14C and 15C, the luciferase activity expressed by the circular RNA molecules synthesized using the Hi-Scarless-PIE circularization method of the present invention with the predefined TLSL circularization element inserted was significantly higher than that of circular RNA molecules without the inserted TLSL circularization element (synthesized using the classic PIE method).

Based on the research results of this example, it can be reasonably inferred that this application allows for modular design of the "ribozyme-TLSL circularization element" on any IRES (natural or modified, e.g., by inserting a predefined TLSL structure element to modify the natural IRES), thereby achieving efficient circular mRNA preparation. At the same time, the protein translation level of the IRES is significantly improved after inserting the TLSL circularization element. These results indicate that the modular design concept of the "ribozyme-TLSL circularization element" in this application achieves a "kill two birds with one stone" effect in the application of circular mRNA preparation, simultaneously enabling high-efficiency circularization and enhanced protein translation levels.

### Example 8. TLSL Structure Elements are Abundantly Present in Natural and Non-Natural RNA Molecules

This example conducted a comprehensive analysis of the distribution of TLSL structure elements in various types of RNA molecules. In this example, only the classic cloverleaf TLSL structure was used as a typical case for analysis.

### Experiment Method:

Search for TLSL elements in RNA secondary structures. First, the RNAfold algorithm was used to predict the secondary structure of RNA sequences, and the prediction results were presented using dot-bracket notation. RNAfold predicts the corresponding secondary structure for each RNA sequence. Then, a self-built TLSL search program in this application was used to search the secondary structure files to determine whether each RNA sequence contained a TLSL structure. The matching principle for the TLSL search followed the structure characteristics of the cloverleaf TLSL. In this example, in addition to the basic features meeting the structure description of the classic cloverleaf TLSL, the total length of the TLSL was limited to no more than 200 nt.

Distribution analysis of TLSL structure elements in various major RNA sequence types. IRES, 5'-UTR, 3'-UTR, mRNA coding regions, non-coding RNAs, and non-natural RNA sequences are RNA types closely related to this application. Therefore, for each of the above types of RNA molecules, representative sequences were first searched and downloaded from public databases, then secondary structure prediction and TLSL element searching were performed on the sequences, and finally, a histogram was used to show the proportion of TLSL elements in each type of RNA sequence. The data collection and preprocessing process is as follows:
IRES sequences were obtained from the IRESbase database (IRESbase: A Comprehensive Database of Experimentally Validated Internal Ribosome Entry Sites. Zhao J, Li Y, Wang C, et al. Genomics Proteomics Bioinformatics. 2020 Apr;18(2):129-139.). This database contains a total of 1328 IRES sequences, originating from humans, viruses, and other eukaryotes, etc. This dataset also includes a portion of artificially designed IRES sequences with a length of 174 nt, and this dataset is named "IRES-IRESbase". Whether the IRES sequences from the IRESbase database can efficiently initiate protein translation in mRNA has not been verified by consistent experiments. Therefore, this experiment further collected a set of IRES sequences that have been verified by consistent experiments from the literature. Specifically, from an experimental article published in the journal Nature Biotechnology in 2023 (Engineering circular RNA for enhanced protein production. Chen R, Wang SK, Belk JA, et al. Nat Biotechnol. 2023 Feb;41(2):262-272.), 51 experimentally validated IRES sequences capable of efficiently initiating mRNA translation were extracted. This dataset of 51 sequences is named "IRES-High Activity".

5'-UTR and 3'-UTR sequences were obtained from the UTRdb database (UTRdb 2.0: a comprehensive, expert curated catalog of eukaryotic mRNAs untranslated regions. Lo Giudice C, Zambelli F, Chiara M, et al. Nucleic Acids Res. 2023 Jan 6;51(D1):D337-D344.). The UTRdb database currently contains over 12.90 million 5'-UTR sequences and over 13.19 million 3'-UTR sequences from 673 species. To save computation time, this study used human UTR sequences as a typical case for TLSL analysis. Specifically, UTRdb contains a total of 93,457 5'-UTR sequences and 87,932 3'-UTR sequences. Criteria for inclusion in the analysis: 1) sequence length greater than 150 nt, 2) for sequences from the same gene, only the longest one was retained. Ultimately, a total of 12,661 5'-UTR sequences and 18,414 3'-UTR sequences were included in this analysis.

Human mRNA coding region sequences were obtained from the Consensus Coding Sequence (CCDS) database (Consensus coding sequence (CCDS) database: a standardized set of human and mouse protein-coding regions supported by expert curation. Pujar S, O'Leary NA, Farrell CM, et al. Nucleic Acids Res. 2018 Jan 4:46(D1):D221-D228.). Specifically, the file containing CDS nucleotide sequences (file named "CCDS _nucleotide.current.fna.gz") was downloaded from the official CCDS database website. This version of the CCDS database contains a total of 35,624 human CDS sequences, all of which were included in the analysis.

Non-coding RNA (ncRNA) sequences were obtained from the RNAChallenge benchmark dataset. This dataset is a benchmark dataset used to evaluate the classification performance of ncRNA classification tools and contains 11,040 ncRNA sequences from multiple species (A large-scale benchmark study of tools for the classification of protein-coding and non-coding RNAs. Dalwinder Singh and Joy Roy. Nucleic Acids Research, gkac1092, 2022). The download address for the dataset is https://github.com/cbl-nabi/RNAChallenge. All 11,040 ncRNA sequences were included in this analysis.

### Results:

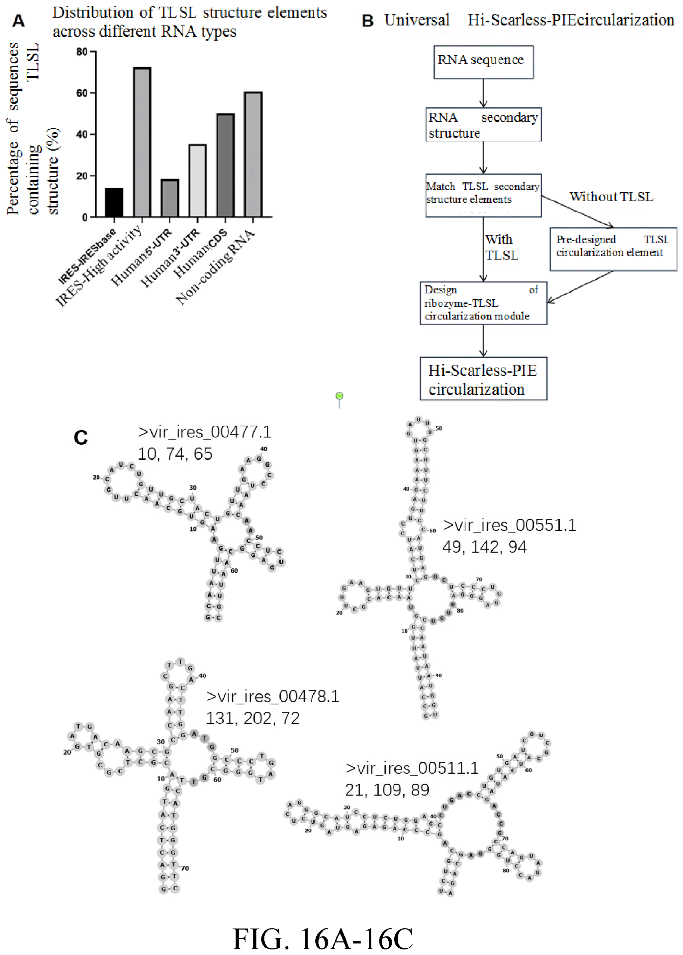

The results are shown in the table below and in Figure 16A. In the "IRES-IRESbase" sequence set, a total of 194 cloverleaf TLSL structure elements were matched in 188 IRES sequences, accounting for 14.16% of all IRES sequences in this analysis. In the "IRES-High Activity" sequence set, a total of 46 cloverleaf TLSL structure elements were matched in 37 IRES sequences, accounting for 72.55% of all high-activity IRES sequences in this analysis. In the human 5'-UTR sequence set, a total of 2,442 cloverleaf TLSL structure elements were matched in 2,327 5'-UTR sequences, accounting for 18.38% of all 5'-UTR sequences in this analysis. In the human 3'-UTR sequence set, a total of 9,766 cloverleaf TLSL structure elements were matched in 6,508 3'-UTR sequences, accounting for 35.34% of all 3'-UTR sequences in this analysis. In the human CDS sequence set, a total of 28,526 cloverleaf TLSL structure elements were matched in 17,896 CDS sequences, accounting for 50.24% of all CDS sequences in this analysis. In the ncRNA sequence set, a total of 14,104 cloverleaf TLSL structure elements were matched in 6,706 ncRNA sequences, accounting for 60.74% of all ncRNA sequences in this analysis.

| | IRES-IRESbase | IRES-High Activity | Human 5'-UTR | Human 3'-UTR | Human CDS | Non-coding RNA |
|---|---|---|---|---|---|---|
| Total Sequences | 1328 | 51 | 12661 | 18414 | 35624 | 11040 |
| Average length | 199 | 638.55 | 318.82 | 1321.84 | 1748.38 | 3064.52 |
| Median Length | 174 | 626 | 242 | 669 | 1323 | 2072 |
| Number of sequences containing TLSL | 188 | 37 | 2327 | 6508 | 17896 | 6706 |
| Number of TLSL elements discovered | 194 | 46 | 2442 | 9766 | 28526 | 14104 |
| Percentage of sequences containing TLSL elements | 14.16% | 72.55% | 18.38% | 35.34% | 50.24% | 60.74% |

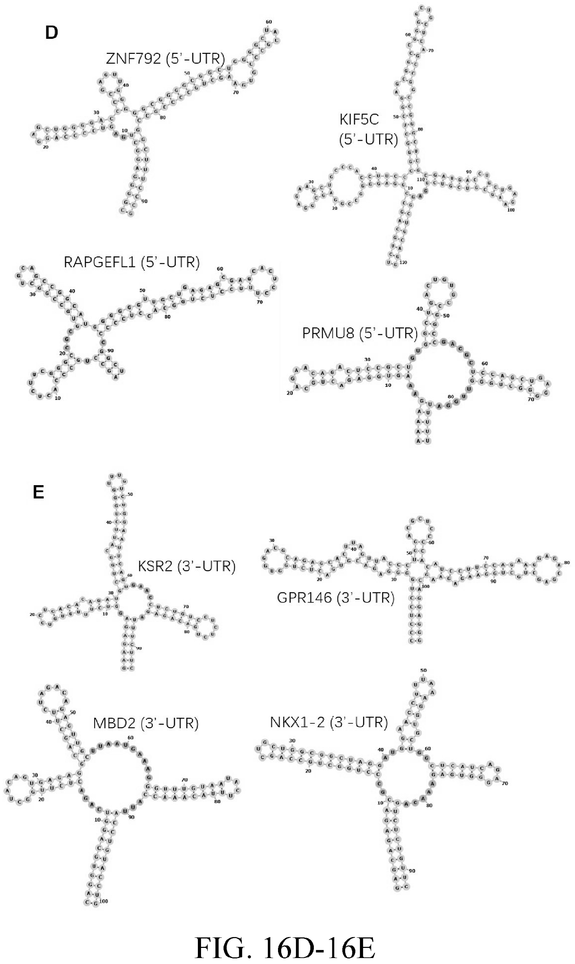

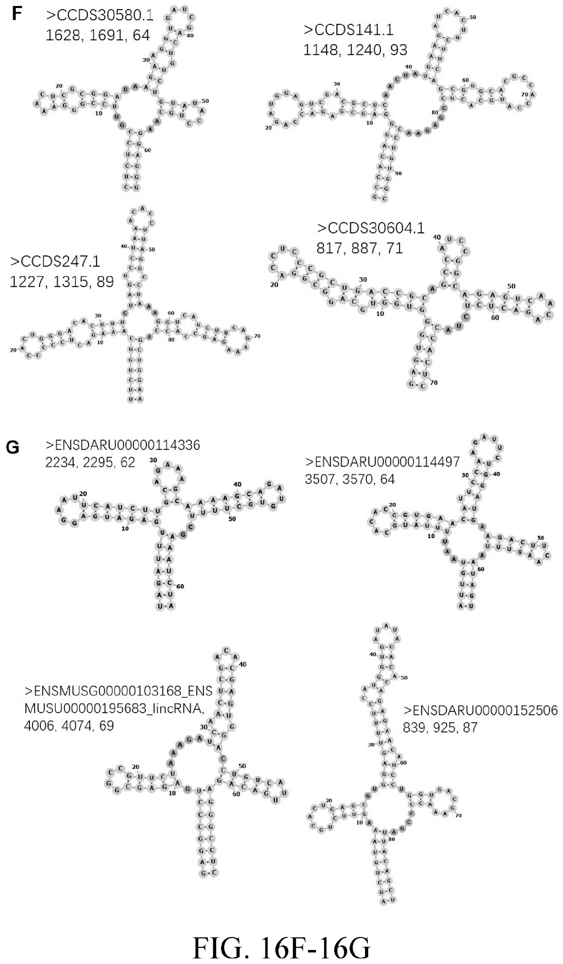

In summary, using the characteristic features of the classic cloverleaf TLSL structure for matching, in the IRES-IRESbase, IRES-High Activity, human 5'-UTR, human 3'-UTR, human CDS, and ncRNA sequence sets analyzed in this example, 14.16%, 72.55%, 18.38%, 35.34%, 50.24%, and 60.74% of the sequences, respectively, contained a typical cloverleaf TLSL structure element (Figure 16A). Figures 16C-G respectively show some representative cloverleaf TLSL structure elements derived from IRES, human 5'-UTR, human 3'-UTR, human CDS, and ncRNA sequence sets. In Figure 16C, the first line of the annotation next to the TLSL structure is the ID number of the IRES from which the TLSL originated, using the IRESbase database ID. The three numbers in the second line of the annotation are the start position, end position, and length of the TLSL within the original IRES sequence. In Figure 16D, the annotation next to the TLSL structure is the name of the gene from which the TLSL originated. In Figure 16E, the annotation next to the TLSL structure is the name of the gene from which the TLSL originated. In Figure 16F, the first line of the annotation next to the TLSL structure is the ID number of the CDS sequence from which the TLSL originated, using the CCDS database ID. The three numbers in the second line of the annotation are the start position, end position, and length of the TLSL within the original CDS sequence. In Figure 16G, the first line of the annotation next to the TLSL structure is the ID number of the ncRNA sequence from which the TLSL originated, using the sequence ID from the RNAChallenge dataset. The three numbers in the second line of the annotation are the start position, end position, and length of the TLSL within the original ncRNA sequence.

In summary, this example demonstrates that TLSL structure elements are widely present in RNA molecules of categories such as IRES, UTR, CDS, and ncRNA. This provides flexibility and broad scope for designing the "ribozyme-TLSL circularization module" in the Hi-Scarless-PIE circularization strategy of the present invention, indicating that the circularization platform of this application has good universality. Theoretically, combined with the reasonable inferences from Examples 5 and 6, the method of this application can achieve high-efficiency circularization preparation for any of the aforementioned RNA sequences containing TLSL structure elements. At the same time, combined with the reasonable inference from Example 7, using a natural IRES or an IRES modified by the methods proposed in this application as the translation initiation sequence, the method of this application can achieve high-efficiency circularization preparation for any mRNA.

### Example 9. Circular RNA Prepared by the Hi-Scarless-PIE Circularization System Has Low Immunogenicity

This example examined the expression levels of immune response factors induced in A549 cells by circular mRNAs prepared using the Anabaena PIE system and the Hi-Scarless-PIE disclosed herein.

Specific steps are as follows:
HPLC Purification: The circularized RNA product after RNase R treatment was heated at 65°C for 5 minutes and then placed on ice for 2 minutes. HPLC purification conditions: Aqueous gel column with silica gel matrix (Dikma Protein SEC-1000 300 x 8.0 mm, 5µm, 99305, Dikma Technologies), flow rate: 0.5 ml/min, nuclease-free buffer: 10 mM Tris, 1 mM EDTA, pH=6. HPLC instrument model: Shimadzu LC-20AD XR High Performance Liquid Chromatograph, using a UV detector at 260 nm to detect the RNA signal and collect the RNA. The collected RNA was column-purified.

Cell Culture: A549 cells (Cell Bank of the Chinese Academy of Sciences) were cultured in a 37°C, 5% CO2 incubator (Thermo Fisher Scientific). A549 cells were cultured in RPMI-1640 medium (L210KJ, Shanghai Yuanpei Biotechnology Co., Ltd.) containing 10% fetal bovine serum (SH30396.03, Hyclone) and 1% penicillin-streptomycin (S110JV, Shanghai Yuanpei Biotechnology Co., Ltd.). Cells were subcultured every 2-3 days.

Immunogenicity Detection: A549 cells were seeded in 12-well plates at 100,000 cells/well. Then, according to the instructions, when the cells reached 70-90% confluence, 1000 ng/well of RNase R-treated circular RNA or further HPLC-purified circular RNA, as well as 3p-hpRNA (tlrl-hprna, InvivoGen), were transfected into the 12-well plates using Lipofectamine 3000 transfection reagent (L3000001, Thermo Fisher Scientific). 16 hours post-transfection, cells were lysed with Trizol, and RNA was extracted. Then, the first strand of cDNA was synthesized using the RevertAid First Strand cDNA Synthesis Kit (K1622, Thermo Fisher Scientific). Subsequently, the expression levels of four immune response proteins, CCL5, IL6, INF-1B, and RIG-1, were detected by quantitative real-time PCR.

The sequences used for quantitative real-time PCR are as follows:
GAPDH-F: GTCTCCTCTGACTTCAACAGCG (SEQ ID NO:44)
GAPDH-R: ACCACCCTGTTGCTGTAGCCAA (SEQ ID NO:45)
IL6-F: AGACAGCCACTCACCTCTTCAG (SEQ ID NO:46)
IL6-R: TTCTGCCAGTGCCTCTTTGCTG (SEQ ID NO:47)
RIG-1-F: CACCTCAGTTGCTGATGAAGGC (SEQ ID NO:48)
RIG-1-R: GTCAGAAGGAAGCACTTGCTACC (SEQ ID NO:49)
CCL5-F: CCTGCTGCTTTGCCTACATTGC (SEQ ID NO:50)
CCL5-R: ACACACTTGGCGGTTCTTTCGG (SEQ ID NO:51)
IFNB1-F: CTTGGATTCCTACAAAGAAGCAGC (SEQ ID NO:52)
IFNB1-R: TCCTCCTTCTGGAACTGCTGCA (SEQ ID NO:53)

### Results:

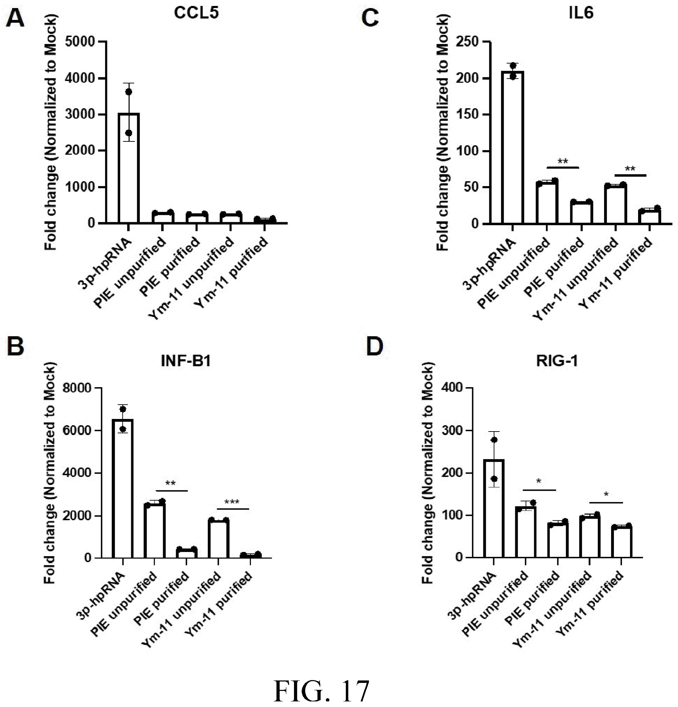

Figure 17 shows the expression levels of immunogenic cytokines induced in A549 cells by circ-GLuc-mRNA prepared using the classic PIE and Hi-Scarless-PIE methods. Among them, the circular RNA precursor molecule prepared by the classic PIE method (SEQ ID NO: 54) was used as a control for immunogenicity comparison, while the precursor molecule for preparing circular RNA by the Hi-Scarless-PIE method used the aforementioned SEQ ID NO: 8. 3p-hpRNA is a triphosphorylated hairpin RNA, a characteristic structure recognized by RIG-I, and is an effective and specific agonist of RIG-I. In this experiment, 3p-hpRNA was used as a positive control expected to exhibit high immunogenicity in A549 cells. In the experimental groups, the "PIE unpurified" group refers to the sample from the classic PIE circularization product without HPLC purification; the "PIE purified" group refers to the sample from the classic PIE circularization product after HPLC purification; the Ym-11unpurified group refers to the sample from the Hi-Scarless-PIE circularization product without HPLC purification; the Ym-11purified group refers to the sample from the Hi-Scarless-PIE circularization product after HPLC purification. The experiment compared the difference in immunogenicity of circular RNAs synthesized by the classic PIE method and the Hi-Scarless PIE method. It also compared the difference in immunogenicity between circular RNAs that underwent HPLC purification and those that did not.

The results are shown in Figure 17. Compared to the positive control, the circ-GLuc-mRNA prepared by both the classic PIE and Hi-Scarless-PIE methods, whether purified by HPLC or not, showed lower expression levels for all four cytokines. Compared to the unpurified groups, the HPLC-purified samples all showed lower expression of IL6, INF-B1, and RIG-1, a trend consistent for samples prepared by both classic PIE and Hi-Scarless-PIE. At the same time, this experiment also observed that samples prepared by classic PIE did not show a reduction in CCL5 expression levels after purification, while samples prepared by Hi-Scarless-PIE showed a significant reduction in CCL5 expression levels after purification. In the groups of samples purified by HPLC, the samples prepared by Hi-Scarless-PIE showed lower expression of CCL5, IL6, and INF-B1 compared to the classic PIE method.

Based on the results of this experiment, the circular RNA prepared by the Hi-Scarless-PIE method exhibits immunogenicity in cells comparable to or lower than that of the classic PIE method.

### Example 10. Circular mRNAs Containing Different IRES Sequences Prepared by the Hi-Scarless-PIE Circularization System Can All Express Proteins In vitro

This example examined the protein expression levels of circular GLuc mRNAs with different IRES sequences prepared by the Hi-Scarless-PIE disclosed herein in HEK-293T and A549 cells.

The nucleotide sequences involved in this example are as follows: SEQ ID NO:8, SEQ ID NO:29, SEQ ID NO:30, SEQ ID NO:31, SEQ ID NO:32, SEQ ID NO:33, SEQ ID NO:55, and SEQ ID NO:56.

### Experiment Method:

Cell Transfection: Before transfection, HEK-293T or A549 cells were seeded in 96-well plates at 20,000 cells/well. Then, according to the instructions, 50-100 ng (equimolar amounts of each RNA) of the circular mRNA molecules prepared according to the method in Example 1 were transfected into HEK-293T and A549 cells, respectively, using Lipofectamine 3000 transfection reagent (L3000001, Thermo Fisher Scientific).

Protein Expression Analysis: 24 hours post-transfection, 10 microliters of culture supernatant were taken, and the luciferase activity in the supernatant was measured using the Pierce Gaussia Luciferase Glow Assay Kit (Thermo Fisher Scientific). Luciferase activity was measured using a SpectraMax i3 microplate reader (Molecular Devices).

### Results:

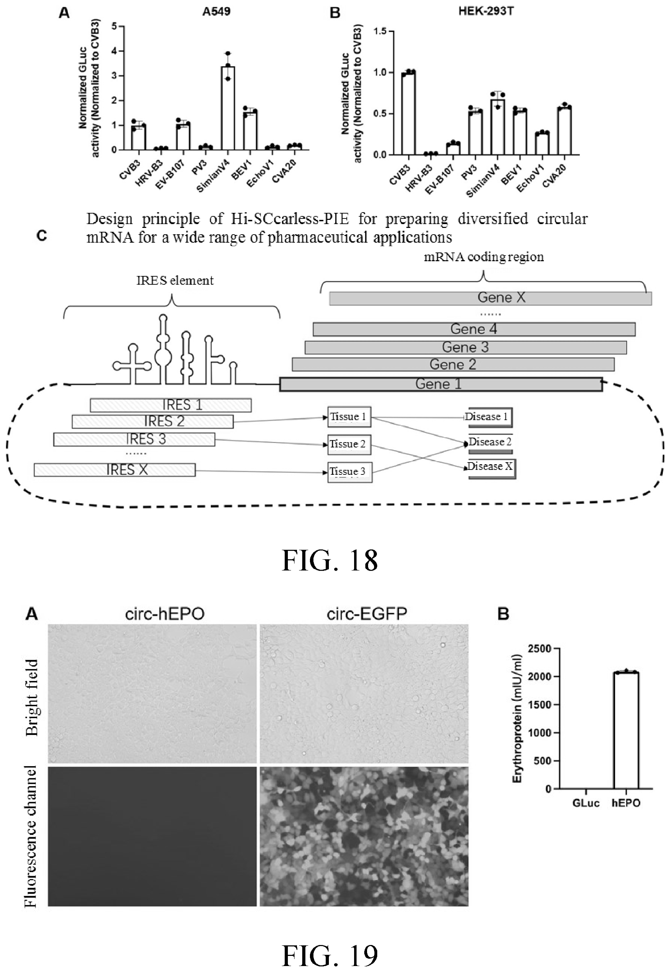

Figures 18A and 18B show the protein expression levels of circular GLuc mRNAs with different IRES sequences in HEK-293T and A549 cells. Using CVB3 IRES as a reference, it can be observed that the protein expression level using the same IRES differs significantly in different cells. For example, the translation level of GLuc driven by PV3 and CVA20 IRES in A549 cells was very low, but it was significantly higher in HEK-293T cells, reaching about half the level of CVB3 IRES. In A549 cells, the protein expression levels of CVB3 IRES and EV-B107 IRES were essentially the same, but in HEK-293T cells, the protein translation level of EV-B107 IRES dropped to about 15% of that of CVB3 IRES. These results indicate that the protein translation level of IRES exhibits cell-type selectivity, and different IRES have different cell selectivity tendencies. This study demonstrates that circular mRNA molecules with different IRES sequences prepared by the Hi-Scarless-PIE system disclosed herein can express proteins at the cellular level. The level of protein expression for the same IRES may differ in different cells, and the level of protein expression for different IRES also differs in the same cell. These results suggest that the protein translation initiation activity of different IRES has cell-type selectivity.

Consistent with our findings, other research teams have also reported the cell and tissue selectivity of IRES protein translation levels. For example, at the 25th Annual Meeting of the American Society of Gene and Cell Therapy (ASGCT) in 2022, Alexander Wesselhoeft reported a high-throughput screening study (Discovery of Translation Initiation Elements Enabled by a Parallel Arrayed Screen of Full-length Viral UTRs in Synthetic Circular RNA, May 16, 2022), which found that IRES activity is cell-type dependent, exhibiting different protein translation levels in different cell types.

Based on this study and the reported fact of cell and tissue selectivity of IRES activity, combined with the universal and practical platform advantages of the Hi-Scarless-PIE circularization system demonstrated collectively in Examples 5, 6, 7, and 8, this application can use the Hi-Scarless-PIE platform to design circular mRNA products for a wide range of pharmaceutical applications. The basic principle is shown in Figure 18C: using a cap-independent translation initiation element (such as IRES) as a non-coding region, this application can design a "ribozyme-TLSL circularization module" in this region to achieve high-efficiency and high-fidelity circular mRNA preparation. Based on the diverse tissue-selective protein translation initiation activities of the IRES library and the flexible protein-coding ability of the mRNA coding region, targeting the corresponding diseased tissues of various diseases, tissue-specific circular IRES-mRNA drugs can be designed for widespread disease treatment. Under this design concept, the IRES region simultaneously undertakes the functions of high-efficiency circularization preparation and tissue-specific protein expression selection, while the sequence of the mRNA coding region does not affect the circularization preparation efficiency. Therefore, this design concept can achieve high-efficiency and high-fidelity circularization preparation and tissue-selective expression for any mRNA sequence, enabling the design of mRNA therapies.

In summary, the Hi-Scarless-PIE invented in this application is a universal circular mRNA preparation platform that can be used for a wide range of biomedical applications.

### Example 11. Detection of Protein Expression Levels of Circular hEPO and EGFP

This example examined the protein expression of circular hEPO and circular EGFP mRNA prepared using the Hi-Scarless-PIE method.

The nucleotide sequences involved in this example are as follows:
EGFP-precursor: SEQ ID NO:57
hEPO-precursor: SEQ ID NO:58

Experiment Method: Before transfection, HEK-293T cells were seeded in 96-well plates at 20,000 cells/well. Then, according to the instructions, 100 ng of the circular EGFP, hEPO, and GLuc mRNA molecules prepared according to the method in Example 1 for the Hi-Scarless-PIE system of the present invention were transfected into HEK-293T cells using Lipofectamine 3000 transfection reagent (L3000001, Thermo Fisher Scientific). After 24 hours, green fluorescence expression was observed using an inverted fluorescence microscope (OLYMPUS CKX53). For HEK-293T cells transfected with circ-hEPO mRNA, 10 microliters of culture supernatant were taken after 24 hours, and the concentration of hEPO protein in the supernatant was detected using the Human Erythropoietin ELISA Kit (hEPO) (ab274397, Abcam) according to the instructions. ELISA detection was performed using a SpectraMax i3 microplate reader (Molecular Devices).

Experimental Results: Figure 19 shows the protein expression of circular hEPO and circular EGFP mRNA prepared using the Hi-Scarless-PIE method. Among them, Figure 19A shows the fluorescence detection result for circ-EGFP protein expression level. Here, circ-hEPO (non-fluorescent) was used as a negative control for EGFP fluorescence detection. The results show that circ-hEPO had no specific green fluorescence, while circ-EGFP showed significant specific green fluorescence. Figure 19B shows the protein expression detection result for circ-hEPO. Here, Gluc was used as a negative control. The results show that the Gluc group had no detectable hEPO signal, while the hEPO group showed a detected hEPO protein concentration of approximately 2000 mIU/ml. This example demonstrates that can the EGFP and hEPO genes can be effectively expressed by the circular RNAs prepared using the Hi-Scarless-PIE method.

### Example 12. Dynamic Trend of In vitro Protein Expression Levels of Circular mRNA Prepared by the Hi-Scarless-PIE Circularization System

This example compared the trends over time of protein expression levels in HEK-293T and A549 cells for capped and tailed linear RNA, circular RNA prepared by the classic PIE system, and circular mRNA prepared by the Hi-Scarless-PIE method. The sequences involved are as follows:
Capped and tailed linear mRNA (SEQ ID NO:59)
Circular mRNA precursor molecule prepared by the classic PIE system, PIE Circular (SEQ ID NO:54)
Circular mRNA precursor molecule prepared by Hi-Scarless-PIE, SEQ-01 Circular (SEQ ID NO:8)

### Experiment Method:

Linear mRNA Synthesis and Purification: The linear mRNA used the 5'-UTR region sequence (SEQ ID NO:60) and the 3'-UTR region sequence (SEQ ID NO:61) from the human beta-globin (HBB) gene. The linear mRNA obtained by *in vitro* transcription according to the method in Example 1 was heated at 65°C for 5 min, then immediately cooled on ice for 5 min. Then, the cap structure was added to the transcription product using Vaccinia Capping Enzyme (M2080, New England Biolabs) and mRNA Cap 2'-O-Methyltransferase (M0366, New England Biolabs) according to New England Biolabs' instructions. Subsequently, a Poly(A) tail was added to the capped transcription product using *E*. *coli* Poly(A) Polymerase (M0276, New England Biolabs). The fully processed products were then purified using the Monarch^{®} RNA Cleanup Kit (T2050, New England Biolabs).

Cell Transfection: Before transfection, HEK-293T or A549 cells were seeded in 96-well plates at 20,000 cells/well. Then, according to the instructions, 50-100 ng (equimolar amounts of each RNA) of circular mRNA prepared by the Anabaena PIE system or the Hi-Scarless-PIE system disclosed herein according to the method in Example 1, or capped and tailed linear mRNA molecules, were transfected into HEK-293T and A549 cells, respectively, using Lipofectamine 3000 transfection reagent (L3000001, Thermo Fisher Scientific).

Protein Expression Analysis: For HEK-293T cells, 10 microliters of culture supernatant were taken at 24 hours (Day 1), 48 hours (Day 2), 72 hours (Day 3), 96 hours (Day 4), 120 hours (Day 5), 144 hours (Day 6), and 168 hours (Day 7) post-transfection. Luciferase activity in the supernatant was measured using the Pierce Gaussia Luciferase Glow Assay Kit (Thermo Fisher Scientific). For A549 cells, 10 microliters of culture supernatant were taken at 24 hours (Day 1), 48 hours (Day 2), and 72 hours (Day 3) post-transfection. Luciferase activity in the supernatant was measured using the Pierce Gaussia Luciferase Glow Assay Kit (Thermo Fisher Scientific). Luciferase activity was measured using a SpectraMax i3 microplate reader (Molecular Devices). After each daily measurement, the culture medium was completely removed and replaced with fresh medium in the culture plates.

### Results:

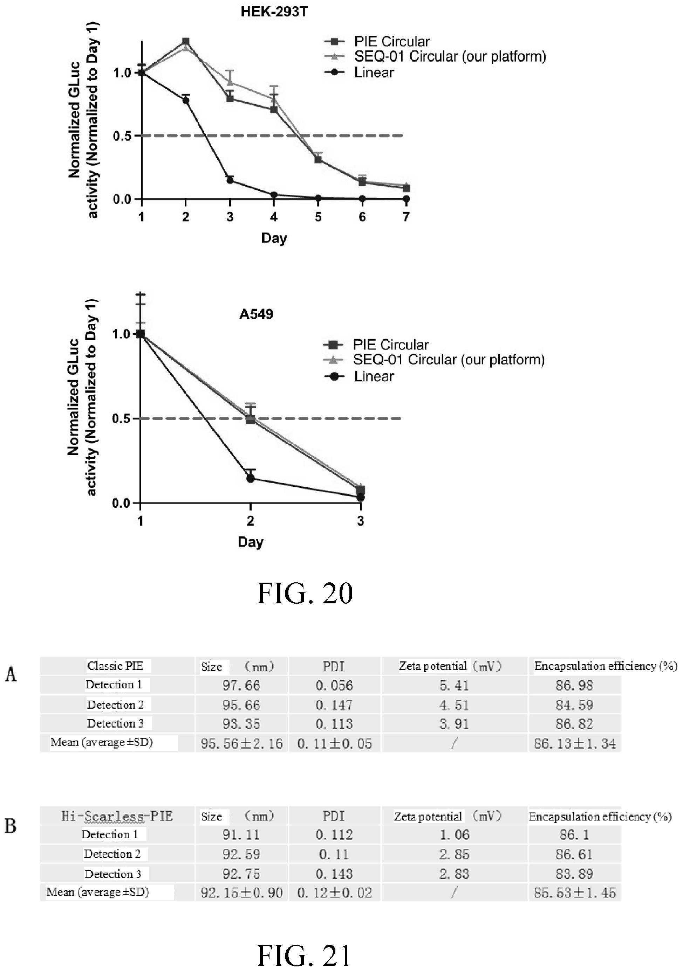

Figure 20 shows the trends over time of protein expression levels in HEK-293T and A549 cells for traditional capped and tailed linear mRNA, circular mRNA prepared by the classic PIE system, and circular mRNA prepared by the Hi-Scarless-PIE disclosed herein. The results are shown in Figure 20. "Linear" refers to the traditional capped and tailed linear mRNA, "PIE Circular" refers to the circular RNA prepared by the classic Anabaena PIE system, and "SEQ-01 Circular" refers to the circular mRNA prepared by the Hi-Scarless-PIE disclosed herein. Figure 20 shows that in HEK-293T cells, both SEQ-01 Circular and PIE Circular continuously and stably expressed high levels of Gaussia Luciferase protein, with a protein expression half-life of 4-5 days, significantly better than the linear mRNA group. In A549 cells, SEQ-01 Circular and PIE Circular showed the same trend in protein expression, both superior to linear mRNA, with a half-life of 2 days.

These results indicate that, similar to the classic PIE system, the circular mRNA prepared by Hi-Scarless-PIE in the present invention has extended stability, a long *in vivo* half-life, and significantly increased protein expression efficiency.

### Example 13. Circular mRNA Prepared by the Hi-Scarless-PIE Circularization System Expresses Protein In vivo in Mice

This example examined the protein expression levels in mice of circular mRNA prepared by the Anabaena PIE system and the Hi-Scarless-PIE disclosed herein.

Experiment Method: Circular mRNA was prepared according to the method described in Example 1: CVB3-FLUC (SEQ ID NO: 62) and its circular RNA precursor molecules were respectively: SEQ ID NO: 63 and SEQ ID NO: 58. Meanwhile, a circular RNA molecule prepared according to the classic PIE method (SEQ ID NO: 64) (its circular RNA precursor molecule was: SEQ ID NO: 65) served as a control group. Additionally, this example also prepared CVB3-hEPO (SEQ ID NO: 66) and its circular RNA molecule precursor (SEQ ID NO: 58), as well as the circular RNA precursor molecule used in the classic PIE preparation scheme (SEQ ID NO: 67). The prepared circular mRNA was purified according to the method described in Example 1.

Circular mRNA-LNP Preparation: The purified product was used to prepare circular mRNA-lipid nanoparticle complexes. The specific steps were as follows: DLin-MC3-DMA: DSPC: Cholesterol: DMG-PEG2000 (AVT (Shanghai) Pharmaceutical Tech Co., Ltd.) were dissolved in ethanol solution at a lipid molar ratio of 50:10:38.5:1.5. Circular RNA was dissolved in 10 mM sodium citrate buffer, pH=4.5. The two phases were rapidly mixed using a microfluidic device (INano L/L+, Mayan (Shanghai) Instrument Technology Co., Ltd.) at a total flow rate of 12 ml/min, with a flow rate ratio of aqueous buffer (circular RNA) to ethanol lipid mixture = 3:1. After preparation, the mixture was ultrafiltered using ultrafiltration tubes to remove ethanol and replace it with PBS solution. Then, the particle size, polydispersity index (PDI), and Zeta potential of the circular RNA-lipid nanoparticle complexes were measured by dynamic light scattering (DLS) using a laser particle size analyzer (Zetasizer Nano-ZSP, Malvern, UK). The content and encapsulation efficiency of circular RNA in the complexes were measured using Quant-iT Ribogreen (Thermo Fisher Scientific).

*In vivo* Administration: The prepared circ-FLuc-mRNA-LNP lipid nanoparticles were injected into the muscles of the right hind leg of 8-week-old Balb/c mice at a dose of 3 µg per mouse. The whole-body fluorescence intensity of the mice was measured on days 1, 3, 7, 14, and 21 post-injections. The prepared circ-FLuc-mRNA-LNP lipid nanoparticles were intravenously injected into 8-week-old Balb/c mice at a dose of 8 µg per mouse. The whole-body fluorescence intensity of the mice was measured on days 1, 3, and 7 post-injections. Before measurement, D-Luciferin potassium salt (ST196-25mg, Beyotime) was injected into the tail vein at a dose of 10 µl/g body weight. 10-20 minutes after injection, when the luciferase signal reached its maximum stable plateau, the fluorescence intensity was measured using a small animal *in vivo* imaging system (PerkinElmer, IVIS Spectrum). The prepared circ-hEPO-mRNA-LNP lipid nanoparticles were intravenously injected into 8-week-old Balb/c mice at a dose of 8 µg per mouse. Blood was collected at 6h, 12h, 24h, 48h, 72h, and 96h post-injection, and the concentration of hEPO protein in the plasma was measured using the Human Erythropoietin ELISA Kit (hEPO) (ab274397, Abcam).

### Results:

Figure 21 shows the preparation and characterization of circular FLuc-mRNA encapsulated in LNP lipid nanoparticles prepared using the classic PIE method and the Hi-Scarless-PIE method, respectively. The characterization results of circ-GLuc mRNA prepared by the classic PIE method (Figure 21A) and the Hi-Scarless-PIE method (Figure 21B) after LNP encapsulation are shown in the figure. The results of this experiment demonstrate that the circular RNA molecules prepared by the Hi-Scarless-PIE method exhibit consistent characteristics when used to prepare LNP encapsulation mixtures.

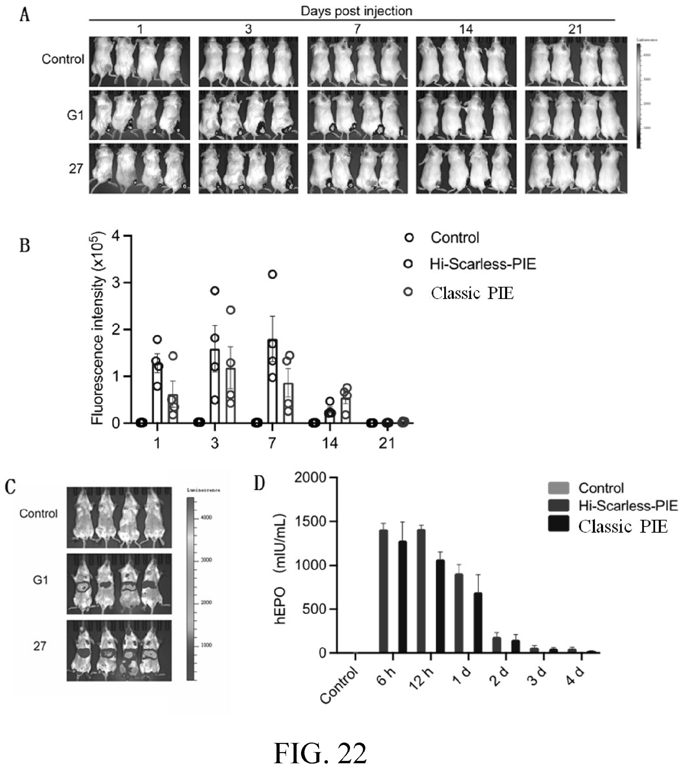

Figure 22 shows the *in vivo* protein expression of circular FLuc-mRNA-LNP lipid nanoparticles prepared using the classic PIE method and the Hi-Scarless-PIE method in mice. Figures 22A and 22B show the expression of FLuc protein after intramuscular injection of circular FLuc-mRNA-LNP and the statistical results of expression levels. Compared to the control group, FLuc protein was significantly expressed after injection of circular FLuc-mRNA-LNP, and the high expression of FLuc protein could last for at least one week. Figure 22C shows the expression of FLuc protein after intravenous injection of circular FLuc-mRNA-LNP. Compared to the control group, significant FLuc protein expression could be detected one day after intravenous injection of circular FLuc-mRNA-LNP.

Figure 22D shows the expression of hEPO in mouse plasma after intravenous injection of circ-hEPO-mRNA-LNP lipid nanoparticles. The concentration of hEPO remained at a high level at 6 hours, 12 hours, and 1 day post-injection, and significantly decreased 2 days post-injection.

The results of this experiment demonstrate that, similar to circular RNA molecules prepared using classic PIE, circular RNA molecules prepared using the Hi-Scarless-PIE method can efficiently express target proteins in mice.

### Example 14. Design and Verification of Uni-cRNA Circular RNA Constructs

In this example, a single stem-loop (SL) circularization element was identified, and the Ana-PIE intron ribozyme was correspondingly modified based on the sequence near the splicing site on the SL. Furthermore, the modified ribozyme and the SL formed a "ribozyme-SL circularization module," ultimately constituting the process and method for a Uni-cRNA circular RNA construct. The effect of the construct in synthesizing circular RNA was experimentally verified.

The design principle of the Uni-cRNA circular RNA construct in the present invention is shown in Figure 23.

The SL circularization element consists of a stem-loop structure and the ribozyme recognition site located on the distal loop.

In this example, multiple approaches were attempted, including searching for stem-loop structures in the IRES region and the CDS (mRNA coding region) region and selecting SL structure elements, and searching for SL structure elements in natural sequences as well as non-natural sequences optimized by algorithms. This example also investigated two cases where the splicing site is located on the distal loop of the SL and on an internal loop. For the above approaches, circular RNA constructs were respectively constructed in this example, and the efficiency of these constructs in preparing circular RNA was tested.

Searching for SL circularization elements in the IRES region: First, the SL circularization element was obtained through the following steps: The secondary structure of CVB3 IRES (SEQ ID NO. 68) is shown in Figure 24A. This schematic diagram of the CVB3 IRES secondary structure is cited from the literature (Nat Biotechnol. 2023;41(2):262-272). Based on the secondary structure of CVB3 IRES, the SL circularization element was selected to be located in the nucleotide sequence from positions 183 to 232 of CVB3 IRES, named Stem-loop-CVB3-IRES (SEQ ID NO. 69). Its secondary structure is shown in Figure 24B. Second, the splicing site was selected: (1) The splicing site is located between U at position 24 and C at position 25 of the Stem-loop-CVB3-IRES sequence. The arrow in Figure 24B indicates the position of the splicing site. In this example, the target circular RNA sequence (SEQ ID NO. 71) was composed of CVB3-IRES (SEQ ID NO. 68) and the mRNA coding region of Gaussia Luciferase (GLuc) (SEQ ID NO. 70). According to the selected splicing site, the Seq 4 sequence was split at this splicing site to form the linear counterpart of the target circular RNA sequence (SEQ ID NO. 72).

Design of a GLuc circular RNA precursor molecule based on the modified Ana-PIE ribozyme: The 3' end fragment of the Ana-PIE ribozyme (SEQ ID NO. 73), the linear counterpart of the target circular RNA sequence (SEQ ID NO. 72), and the modified 5' end fragment of the Ana-PIE ribozyme (SEQ ID NO. 74) were connected to each other to form a GLuc circular RNA precursor molecule based on the modified Ana-PIE ribozyme, named circ-CVB3-IRES-GLuc-precursor (SEQ ID NO. 75).

Synthesis of the above-designed circular RNA precursor molecules and constructs: First, DNA sequences corresponding to SEQ ID NO. 68, SEQ ID NO. 70, SEQ ID NO. 73, and the 5' end fragment of the native Ana-PIE ribozyme (SEQ ID NO. 76) were synthesized (Beijing Tsingke Biotech Co., Ltd.). These DNA sequences were cloned via molecular cloning methods into a linear plasmid vector pUC57 (GenBuilder Plus Cloning Kit, L00744, GenScript Biotech Corporation) containing a T7 promoter generated by PCR, to obtain the corresponding constructs (i.e., the construct using the native Ana-PIE intron). Based on this construct, the sequence of the native Ana-PIE ribozyme 5' end fragment (SEQ ID NO: 76) was modified, wherein A at position 10 of the 5' end of SEQ ID NO: 76 was mutated to G, and G at position 11 was mutated to C, forming the modified Ana-PIE intron construct.

Preparation of linear plasmid template: The plasmid was transformed into Top10 competent cells, plated, and the next day clones were picked and expanded in LB medium containing Amp resistance (37°C/200 rpm/overnight). Then plasmid extraction was performed (Endo-Free Plasmid Mini Kit II, DP118, Tiangen Biotech (Beijing) Co., Ltd.). Subsequently, the linearized plasmid template was prepared by digestion with EcorI (1040, Takara) (37°C, 1 hour). The digestion product was recovered using Monarch^{®} PCR & DNA Cleanup Kit (T1030, New England Biolabs), concentration was determined by Nano-Drop (Thermo), and the digestion product was identified by 1.5% agarose gel electrophoresis. The purified linear plasmid served as the template for *in vitro* transcription.

Preparation of circRNA precursor by *in vitro* transcription: *In vitro* transcription was performed using the HiScribe^{®} T7 High Yield RNA Synthesis Kit (E2040S, New England Biolabs) on the linearized plasmid DNA template (37°C, 2 hours) to synthesize the circRNA precursor. After *in vitro* transcription, the linear DNA template was digested with DNase I (M0303S, New England Biolabs) (37°C, 15 min). Then, the above product was column-purified using Monarch^{®} RNA Cleanup Kit (T2050, New England Biolabs).

circRNA synthesis: The circular RNA precursor molecule was added to a buffer containing GTP (final concentration 2 mM) (50 mM Tris-HCl, 10 mM MgCl₂, 1 mM DTT, pH 7.5). The solution was heated at 55°C for 15 min, followed by column purification (Monarch^{®} RNA Cleanup Kit, T2050, New England Biolabs).

Identification of circular RNA using capillary electrophoresis: After determining the concentration of the circularized RNA precursor and crude circularized RNA product using Nano-Drop (Thermo Fisher Scientific), they were processed using the QIAxcel RNA High Sensitivity Cartridge Kit (929112) according to the instructions, and then detected and analyzed using the QIAxcel Connect capillary electrophoresis instrument according to the operating instructions. In this experiment, the high-sensitivity RNA marker (QIAxcel RNA High-Sensitivity Marker Set, 929112, QIAGEN) was used to indicate RNA molecular bands of different sizes.

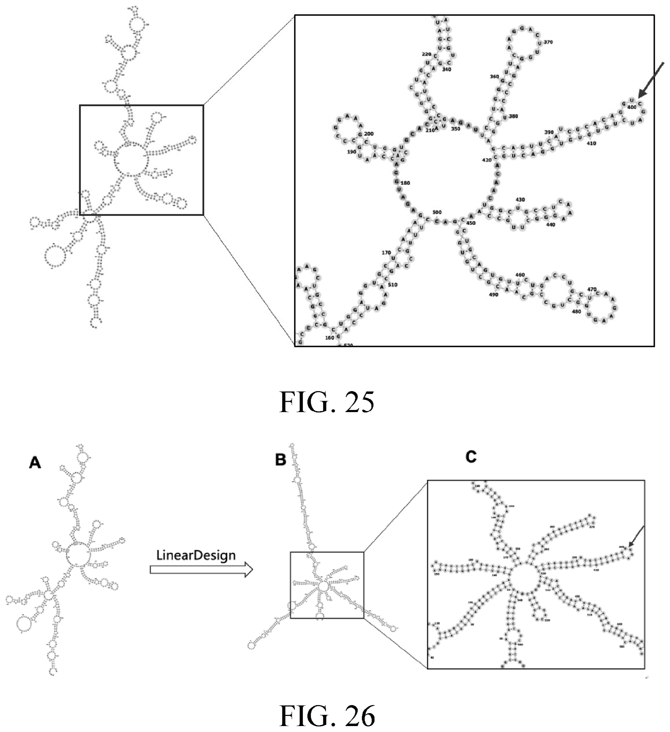

Searching for SL circularization elements in the CDS region:
(1) Natural sequence: In Gaussia Luciferase (SEQ ID NO. 70), based on the secondary structure, the SL circularization element was selected to be located in the nucleotide sequence from positions 384 to 420 of Gaussia Luciferase, named SL-Gaussia Luciferase (SEQ ID NO. 77). Its secondary structure is shown in Figure 25. The splicing site is located between U at position 18 and C at position 19 of the SL-Gaussia Luciferase sequence. The arrow in Figure 25 indicates the position of the splicing site within the full-length GLuc.
(2) Non-natural sequences optimized using computer algorithms: Numerous computer algorithms can optimize RNA sequences, thereby improving the properties or functions of the RNA molecule itself. Among them, Linear Design is a representative algorithm used to optimize the CDS region of mRNA. This algorithm replaces synonymous codons to make the sequence of the mRNA CDS region more stable without affecting the encoded protein product. In this example, within the Gaussia Luciferase sequence optimized by Linear Design (SEQ ID NO. 78), it was found based on the secondary structure that the nucleotide sequence from positions 384 to 420 of the Linear Design-optimized Gaussia Luciferase sequence was also a stem-loop (SEQ ID NO. 79). This stem-loop was selected as the SL circularization element. The secondary structure of the sequence without Linear Design optimization is shown in Figure 26A, and the secondary structure of the sequence after Linear Design optimization is shown in Figure 26B. Figure 26C shows the specific position of the splicing site as indicated by the arrow. The splicing site is located between U at position 18 and G at position 19 of the SL-Gaussia Luciferase sequence. After selecting the splicing site, the corresponding circular RNA precursor molecules (SEQ ID NO. 80 and SEQ ID NO. 81) were synthesized following the method for synthesizing circ-CVB3-IRES-GLuc-precursor described above.

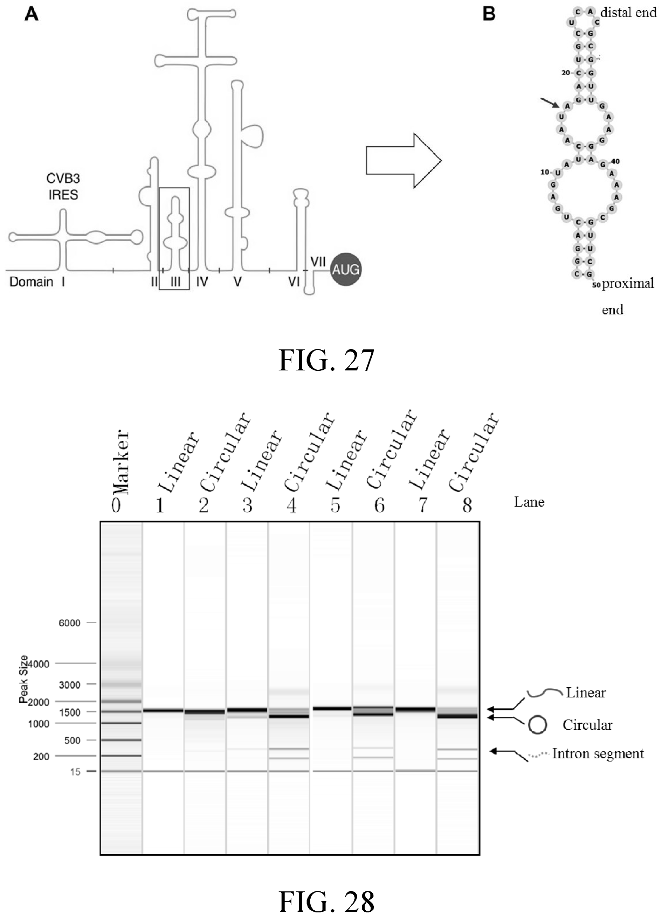

Splicing site on an internal loop: Besides the distal loop in a stem-loop, common loop structures in RNA also include internal loops. Therefore, this example compared the circularization efficiency when the splicing site is located on a distal loop versus on an internal loop. A schematic diagram of the secondary structure of CVB3 IRES is shown in Figure 27A, cited from the literature (Nat Biotechnol. 2023;41(2):262-272). In CVB3 IRES (SEQ ID NO. 68), based on the secondary structure, the SL circularization element was selected to be located in the nucleotide sequence from positions 183 to 232 of CVB3 IRES, named SL-CVB3-IRES (SEQ ID NO. 69). Its secondary structure is shown in Figure 27B. Second, the splicing site was selected: The splicing site is located between U at position 16 and A at position 17 of the SL-CVB3-IRES sequence, as indicated by the arrow in Figure 27B. After selecting the splicing site, the corresponding circular RNA precursor molecule (SEQ ID NO. 82) was synthesized following the method for synthesizing circ-CVB3-IRES-GLuc-precursor described above.

The research results are shown in Figure 28. Capillary lanes 1 and 2 show the circular RNA precursor molecules and the product after circularization for the splicing site on the internal loop, respectively. Comparing lanes 1 and 2, most of the bands in lane 2 were located at the position of linear RNA, with only a small amount of circular bands. The circularization efficiency was calculated using the software (QIAxcel ScreenGel 2.0) that comes with the QIAxcel Connect capillary electrophoresis instrument. Here, circularization efficiency = 100% * circular / (linear + circular). Quantitative results showed that the circularization efficiency for this group was 6.77%. Capillary lanes 5 and 6 show the circular RNA precursor molecule and the product after circularization for the splicing site on the distal loop of the stem-loop in CVB3 IRES, respectively. Comparing lanes 6 and 5, the bands in lane 6 were mainly distributed at the position of circular RNA. Quantitative results showed that the circularization efficiency for this group was 62.41%. The results of these two groups indicate that the circularization efficiency is significantly higher when the splicing site is on the distal loop compared to on the internal loop.

As shown in Figure 28, capillary lanes 3 and 4 show the circular RNA precursor molecule and the product after circularization synthesized with the splicing site on the distal loop of the natural Gaussia Luciferase sequence, respectively. Capillary lanes 7 and 8 show the circular RNA precursor molecule and the product after circularization synthesized with the splicing site on the distal loop of the Linear Design-optimized Gaussia Luciferase sequence, respectively. Compared to the circular RNA precursor molecules, the main band of the circularized products was located at the position of circular RNA. Moreover, the circularization efficiencies for the natural Gaussia Luciferase sequence and the Linear Design-optimized Gaussia Luciferase sequence were comparable, at 78.55% and 79.21%, respectively. These results indicate that the "ribozyme-SL circularization module" can achieve high-efficiency circularization preparation in both natural and non-natural RNA sequences.

The peak data from capillary electrophoresis used for quantitative calculation in this experiment and the circularization efficiencies are shown in the table below.

| | **Lane 2** | **Lane 4** | **Lane 6** | **Lane 8** |
|---|---|---|---|---|
| **Circular (pg/µl)** | 2589.88 | 38577.59 | 23284.58 | 30942.76 |
| **Linear (pg/µl)** | 35670.35 | 10533.99 | 14026.36 | 8122.15 |
| **Circularization Efficiency (%)** | 6.77 | 78.55 | 62.41 | 79.21 |

### Example 15. The Uni-cRNA Circularization System Can Prepare Circular RNA Molecules with Precise Sequences

In this example, multiple methods were used to identify whether the product of the present invention is the target circular RNA molecule. The identification methods include:
1. Identification by RNase R. RNase R is a 3'-5' exoribonuclease that degrades RNA in the 3'-5' direction. It can degrade linear RNA molecules, but circular RNA without 3' and 5' ends cannot be degraded.

Specific steps are as follows: The purified circular RNA or linear circRNA precursor was heated at 65°C for 3 minutes, then immediately cooled on ice for 2 minutes. Then, linear RNA was digested using RNase R (E049, Applied Biological Materials) (37°C, 30 min). After the reaction was terminated, the RNase R-digested RNA was column-purified using Monarch^{®} RNA Cleanup Kit (T2050, New England Biolabs). Then, 1.5% agarose gel was prepared for agarose gel electrophoresis analysis.

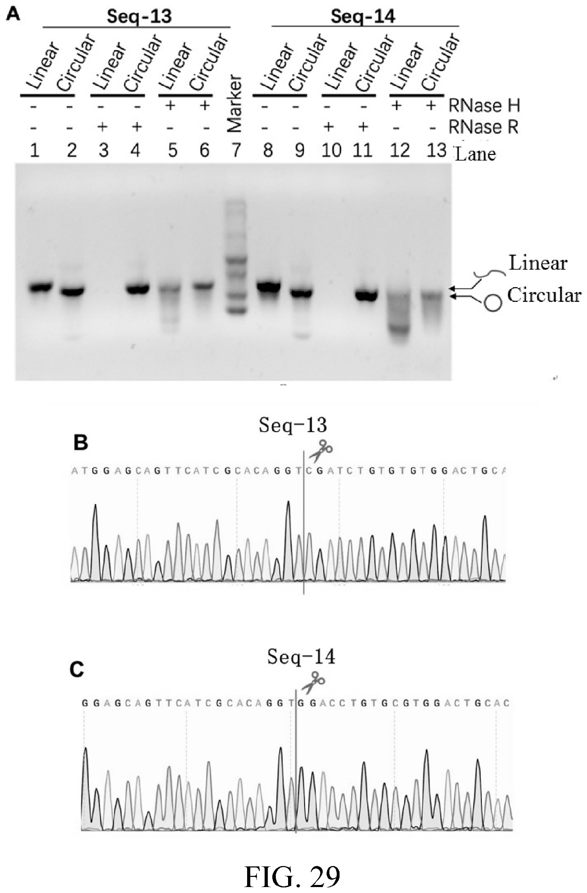

The results are shown in Figure 29A. Lanes 1 and 3 are the linear circRNA precursor (precursor-mRNA, SEQ ID NO: 80) without circularization reaction; lanes 2 and 4 are the products after circularization; lanes 3 and 4 are the results after RNase R treatment. As shown in the figure, after circularization, the position of the main band in lane 2 was below the band in lane 1, and a distinct band appeared at the very bottom, suggesting that the circularization reaction produced a circular mRNA molecule with reduced molecular weight and a very small intron fragment. The RNase R treatment group showed no band in lane 3, while the band presumed to be circular RNA in lane 4 remained unaffected by RNase R. Lanes 8 and 10 are the linear circRNA precursor (precursor-mRNA, SEQ ID NO: 81) without circularization reaction; lanes 9 and 11 are the products after circularization; lanes 10 and 11 are the results after RNase R treatment. As shown in the figure, after circularization, the position of the main band in lane 9 was below the band in lane 8, and a distinct band appeared at the very bottom, suggesting that the circularization reaction produced a circular mRNA molecule with reduced molecular weight and a very small intron fragment. The RNase R treatment group showed no band in lane 10, while the band presumed to be circular RNA in lane 11 remained unaffected by RNase R.

2. Identification using RNase H. RNase H is an endoribonuclease that specifically hydrolyzes RNA in DNA-RNA hybrid strands. Based on the structure difference between linear RNA and circular RNA, after binding the same probe to linear RNA and circular RNA, linear RNA will be cleaved into two bands, while circular RNA will only be cleaved open into linear form, still appearing as one band.

Specific steps are as follows: A 10-fold molar excess of DNA probe was added to the circular RNA obtained after RNase R treatment, then heated at 65°C for 5 min and annealed at room temperature. Digestion was performed with RNase H (M0279, New England Biolabs) at 37°C for 20 minutes, and the digested RNA was column-purified. The DNA probe sequences are shown in SEQ ID NO: 83 and SEQ ID NO: 84.

The experimental results are shown in Figure 29A. In the Seq-13 group, lane 5 is the linear RNA precursor molecule, lane 6 is the product after the circularization reaction. After RNase H treatment of the samples in lanes 5 and 6, lane 5 formed two bands, while lane 6 formed one band, suggesting that lane 6 contains circular RNA. Similarly, in the Seq-14 group, lane 12 is the linear RNA precursor molecule, lane 13 is the product after the circularization reaction. After RNase H treatment, lane 12 formed two bands, while lane 13 formed one band, suggesting that lane 13 contains circular RNA.

3. Verification of ribozyme recognition site sequence accuracy by RT-PCR and Sanger sequencing. The aforementioned methods 1 and 2 can confirm that circular RNA molecules are formed after the circularization reaction, but whether the ligation near the splicing site is accurate requires further sequencing verification. Therefore, this example also used sequencing across the splicing site to demonstrate that the circularization reaction occurred at the expected position, forming the target circular RNA molecule.

Specific steps are as follows: The circular RNA obtained after RNase R treatment was used with the RevertAid First Strand cDNA Synthesis Kit (K1622, Thermo Fisher Scientific) and Random primers to synthesize the first strand of cDNA. Then, forward and reverse PCR primers were designed upstream and downstream of the splice junction, respectively. Using the cDNA as a template, PCR was performed to obtain an amplified fragment for sequencing verification. The circular mRNA molecule should show the expected RNA sequence spanning the splicing site near the splice junction. Primer sequences are shown in SEQ ID NO: 85, SEQ ID NO: 86, SEQ ID NO: 87, and SEQ ID NO: 88.

The results are shown in Figure 29B and Figure 29C. The sequencing results for the Seq-13 group and the Seq-14 group confirmed that the sequences on both sides of the splicing site were consistent with the designed circular RNA sequence, proving that the head and tail of the circular RNA were joined together. The correct circular mRNA molecule was successfully obtained.

In summary, this example verified through three complementary methods that the target circular RNA molecule was successfully synthesized in the *in vitro* circularization reaction system of the present invention.

### Example 16. Circular mRNAs Prepared by the Uni-cRNA Circularization System Can All Express Proteins in Cells

This example examined the protein expression levels of circular GLuc mRNA prepared by Uni-cRNA disclosed herein in HEK-293T and A549 cells.

The nucleotide sequences involved in this example are as follows: SEQ ID NO: 75, SEQ ID NO: 89 (this sequence is the circular RNA construct circ-CVA20-IRES-GLuc-precursor synthesized by searching for a stem-loop in CVA20 IRES, determining the splicing site, and synthesizing according to the method described in Example 14), SEQ ID NO: 90 (this sequence is the circular RNA construct circ-CVB3-IRES-hEPO-precursor synthesized by searching for a stem-loop in hEPO CDS, determining the splicing site, and synthesizing according to the method described in Example 14).

### Experiment Method:

Cell Transfection: Before transfection, HEK-293T or A549 cells were seeded in 96-well plates at 20,000 cells/well. Then, according to the instructions, approximately 100 ng (equimolar amounts of each RNA) of the circular mRNA molecules prepared according to the method in Example 1 and purified by RNase R were transfected into HEK-293T and A549 cells, respectively, using Lipofectamine 3000 transfection reagent (L3000001, Thermo Fisher Scientific).

Protein Expression Analysis: 24 hours post-transfection, for cells transfected with circ-GLuc, 10 microliters of culture supernatant were taken, and the luciferase activity in the supernatant was measured using the Pierce Gaussia Luciferase Glow Assay Kit (Thermo Fisher Scientific). Luciferase activity was measured using a SpectraMax i3 microplate reader (Molecular Devices). For HEK-293T cells transfected with circ-hEPO mRNA, 10 microliters of culture supernatant were taken after 24 hours, and the concentration of hEPO protein in the supernatant was detected using the Human Erythropoietin ELISA Kit (hEPO) (ab274397, Abcam) according to the instructions. ELISA detection was performed using a SpectraMax i3 microplate reader (Molecular Devices).

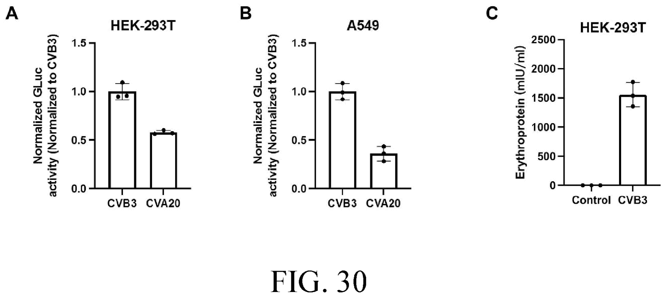

Experimental Results: Figures 30A and 30B respectively show the protein expression levels of circular GLuc mRNAs containing different IRES sequences in HEK-293T and A549 cells. Figure 30C shows the protein expression level of circular hEPO mRNA containing the CVB3 IRES sequence in HEK-293T cells. These results demonstrate that the circular RNAs prepared using the Uni-cRNA method of the present invention can effectively express GLuc and hEPO proteins.

### Example 17. SL Structure Elements are Widely Present in Different Types of RNA Molecules

To confirm the applicability scope of the "ribozyme-SL circularization module" method among diverse RNA molecules, this example conducted a comprehensive analysis of the distribution of SL structure elements in various types of RNA molecules. The stem-loop structure is one of the most fundamental RNA secondary structures and is widely present in various types of RNA molecules. This example only performs an exemplary analysis on a few common RNA categories, selecting one representative dataset for each category for analysis. The present invention mainly involves two major categories of RNA molecules: 1) mRNA (mRNA molecules serve as translation templates, producing corresponding protein products as effector molecules), and 2) non-mRNA (not used to encode polypeptides and proteins, but the RNA molecule itself serves as the effector molecule). Therefore, in this example, human mRNA reference sets were selected as the analysis objects for mRNA, and non-coding RNA (ncRNA) was selected as the analysis object for non-mRNA. In addition, the IRES element is the most commonly used translation initiation regulatory element in circular mRNA, so this example also analyzed the distribution of stem-loops in IRES. For each type of RNA, the sources and acquisition methods of the representative datasets selected in this example are as follows:
5'-UTR and 3'-UTR sequences were obtained from the UTRdb database (UTRdb 2.0: a comprehensive, expert curated catalog of eukaryotic mRNAs untranslated regions. Lo Giudice C, Zambelli F, Chiara M, et al. Nucleic Acids Res. 2023 Jan 6;51(D1):D337-D344.). The UTRdb database currently contains over 12.90 million 5'-UTR sequences and over 13.19 million 3'-UTR sequences from 673 species. To save computation time, this study used human UTR sequences as a typical case for TLSL analysis. Specifically, UTRdb contains a total of 93,457 5'-UTR sequences and 87,932 3'-UTR sequences. Criteria for inclusion in the analysis: 1) sequence length greater than 150 nt, 2) for sequences from the same gene, only the longest one was retained. Ultimately, a total of 12,661 5'-UTR sequences and 18,414 3'-UTR sequences were included in this analysis.

Human mRNA coding region sequences were obtained from the Consensus Coding Sequence (CCDS) database (Consensus coding sequence (CCDS) database: a standardized set of human and mouse protein-coding regions supported by expert curation. Pujar S, O'Leary NA, Farrell CM, et al. Nucleic Acids Res. 2018 Jan 4;46(D1):D221-D228.). Specifically, the file containing CDS nucleotide sequences (file named "CCDS_nucleotide.current.fna.gz") was downloaded from the official CCDS database website. This version of the CCDS database contains a total of 35,624 human CDS sequences, all of which were included in the analysis.

IRES sequences were obtained from the IRESbase database (IRESbase: A Comprehensive Database of Experimentally Validated Internal Ribosome Entry Sites. Zhao J, Li Y, Wang C, et al. Genomics Proteomics Bioinformatics. 2020 Apr;18(2):129-139.). This database contains a total of 1328 IRES sequences, originating from humans, viruses, and other eukaryotes, etc. This dataset also includes a portion of artificially designed IRES sequences with a length of 174 nt.

Non-coding RNA (ncRNA) sequences were obtained from the RNAChallenge benchmark dataset. This dataset is a benchmark dataset used to evaluate the classification performance of algorithms for protein-coding RNA and non-coding RNA, and contains 11,040 ncRNA sequences from multiple species (A large-scale benchmark study of tools for the classification of protein-coding and non-coding RNAs. Dalwinder Singh and Joy Roy. Nucleic Acids Research, gkac1092, 2022). The download address for the dataset is https://github.com/cbl-nabi/RNAChallenge.

### Experimental Method:

Search for stem-loop structures in RNA secondary structures. First, the RNAfold algorithm was used to predict the secondary structure of RNA sequences, and the prediction results were presented using dot-bracket notation. Then, a self-built stem-loop search program in this application was used to search the secondary structure files to determine whether each RNA sequence contained a stem-loop structure, as well as the number of different stem-loops contained. In this example, the length of the stem arms of the stem-loop was limited to ≥2 consecutive complementary paired nucleotides, and the size of the loop was set to contain n (3 ≤ n ≤ 10) unpaired nucleotides.

### Results:

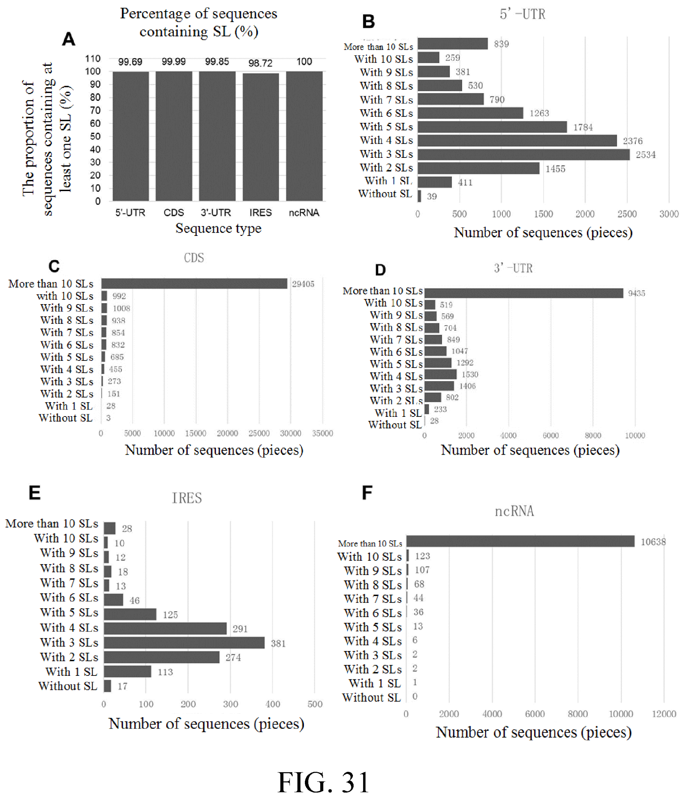

The results are shown in the table below and in Figure 31A. Using the stem-loop structure features for matching, in the human 5'-UTR, human CDS, human 3'-UTR, IRES, and non-coding RNA sequence sets analyzed in this example, the proportions of sequences containing at least one stem-loop were 99.69%, 99.99%, 99.85%, 98.72%, and 100%, respectively (Figure 31A). In addition, Figures 31B-F show the proportions of sequences containing 1, 2, 3, 4, 5, 6, 7, 8, 9, 10, and more than 10 stem-loop structures within the same sequence. The results show that in the human CDS region sequence set, human 3'-UTR sequence set, and ncRNA sequence set, the proportion of sequences containing more than 10 stem-loop structures is very high, accounting for the vast majority of sequences, which provides a massive number of optional splicing sites for the design of the "ribozyme-SL structure module" of the present invention. The number of stem-loops contained in the IRES sequence set is mainly distributed between 1-5, and the number of stem-loops contained in the 5'-UTR sequence set is mainly distributed between 2-6. This may be due to the relatively short average sequence length of IRES and 5'-UTR.

| | Human 5'-UTR | Human CDS | Human 3'-UTR | IRES | Non-coding RNA |
|---|---|---|---|---|---|
| Total Sequences | 12661 | 35624 | 18414 | 1328 | 11040 |
| Average Sequence length | 318.82 | 1748.38 | 1321.84 | 199 | 3064.52 |
| Median Sequence Length | 242 | 1323 | 669 | 174 | 2072 |
| Number of sequences containing SL | 12622 | 35621 | 18386 | 1311 | 11040 |
| Number of Sequences without SL elements | 39 | 3 | 28 | 17 | 0 |
| Percentage of sequences containing SL elements | 99.69% | 99.99% | 99.85% | 98.72% | 100% |

### Discussion

Examples 5, 6, 7, and 8 collectively illustrate that the Hi-Scarless-PIE circularization system based on the modular design concept of the "ribozyme-TLSL circularization module" in the present invention possesses good universality and practicality. Exemplarily (as shown in Figure 16B), for a target mRNA sequence, when the sequence contains a TLSL structure element, the "ribozyme-TLSL circularization module" can be directly designed to achieve high-efficiency circularization preparation. When the target mRNA molecule does not contain a TLSL structure element, a pre-designed TLSL structure element module can be inserted in an appropriate region, thereby designing a "ribozyme-TLSL circularization module" and achieving high-efficiency circularization preparation. This fully demonstrates that the Hi-Scarless-PIE circularization platform proposed in the present invention is a universal circular mRNA preparation platform.

The uses of circular RNA mainly include two categories: mRNA and non-coding RNA. In circular mRNA, when using the IRES + CDS sequence combination, SL structure elements can be matched in almost 100% of the combined sequences, thereby constructing a "ribozyme-SL circularization module" to achieve high-efficiency circular mRNA preparation. SL structure elements exist in almost all non-coding RNAs, so the present invention can correspondingly design "ribozyme-SL circularization modules" to achieve high-efficiency circularization preparation of non-coding RNAs.

In summary, Examples 14, 15, and 17 collectively illustrate that the Uni-cRNA circularization platform based on the "ribozyme-SL circularization module" proposed in the present invention can achieve high-efficiency *in vitro* circularization preparation for almost any RNA molecule. Example 16 demonstrates that circular mRNA prepared by Uni-cRNA can efficiently express proteins. These examples collectively demonstrate that Uni-cRNA of the present invention is a universal, high-efficiency, and high-fidelity RNA circularization technology, suitable for preparing circular mRNA and circular non-coding RNA for a wide range of applications.

All documents mentioned in the present invention are cited as references in this application, as if each document were individually cited as a reference. In addition, it should be understood that after reading the above teaching content of the present invention, those skilled in the art can make various changes or modifications to the present invention, and these equivalent forms also fall within the scope defined by the appended claims of this application.

### ST.26 SEQUENCE LISING - HUMAN READABLE FORMAT

| | |
|---|---|
| Invention Title | 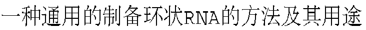 (zh) |
| Applicant File Reference | P2024-0124 |
| Applicant Name | 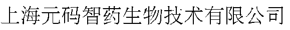 |
| Applicant Name Latin | Byterna therapeutics |
| Inventor Name | - |
| Inventor Name Latin | ZHAO Hui |
| File name | P2024-0124x1b.xm1 |
| DTD version | V1_3 |
| Software name | WIPO Sequence |
| Software version | 2.3.0 |
| Production date | 2024-02-06 |
| Original free text language Code | - |
| Non English free text language Code | - |
| Application number | - |
| Filing date | - |
| Earliest priority number | 2023112106896 |
| Earliest priority date | 2023-09-19 |
| Sequence Total Quantity | 90 |

SEQ ID NO: 1
   length 741
   molecule type RNA
   division PAT
   Key Location/Qualifiers
   source 1..741
      /mol_type=other RNA
      /organism=synthetic construct
   Sequence 
ttaaaacagc ctgtgggttg atcccaccca caggcccatt gggcgctagc actctggtat 60
cacggtacct ttgtgcgcct gttttatacc ccctccccca actgtaactt agaagtaaca 120
cacaccgatc aacagtcagc gtggcacacc agccacgttt tgatcaagca cttctgttac 180
cccggactga gtatcaatag actgctcacg cggttgaagg agaaagcgtt cgttatccgg 240
ccaactactt cgaaaaacct agtaacaccg tggaagttgc agagtgtttc gctcagcact 300
accccagtgt agatcaggtc gatgagtcac cgcattcccc acgggcgacc gtggcggtgg 360
ctgcgttggc ggcctgccca tggggaaacc catgggacgc tctaatacag acatggtgcg 420
aagagtctat tgagctagtt ggtagtcctc cggcccctga atgcggctaa tcctaactgc 480
ggagcacaca ccctcaagcc agagggcagt gtgtcgtaac gggcaactct gcagcggaac 540
cgactacttt gggtgtccgt gtttcatttt attcctatac tggctgctta tggtgacaat 600
tgagagatcg ttaccatata gctattggat tggccatccg gtgactaata gagctattat 660
atatcccttt gttgggttta taccacttag cttgaaagag gttaaaacat tacaattcat 720
tgttaagttg aatacagcaa a 741
SEQ ID NO: 2
   length 94
   molecule type RNA
   division PAT
   Key Location/Qualifiers
   source 1..94
      /mol_type=other RNA
      /organism=synthetic construct
   Sequence 
ttaaaacagc ctgtgggttg atcccaccca caggcccatt gggcgctagc actctggtat 60
cacggtacct ttgtgcgcct gttttatacc ccct 94
SEQ ID NO: 3
   length 558
   molecule type RNA
   division PAT
   Key Location/Qualifiers
   source 1..558
      /mol_type=other RNA
      /organism=synthetic construct
   Sequence 
atgggagtca aagttctgtt tgccctgatc tgcatcgctg tggccgaggc caagcccacc 60
gagaacaacg aagacttcaa catcgtggcc gtggccagca acttcgcgac cacggatctc 120
gatgctgacc gcgggaagtt gcccggcaag aagctgccgc tggaggtgct caaagagatg 180
gaagccaatg cccggaaagc tggctgcacc aggggctgtc tgatctgcct gtcccacatc 240
aagtgcacgc ccaagatgaa gaagttcatc ccaggacgct gccacaccta cgaaggcgac 300
aaagagtccg cacagggcgg cataggcgag gcgatcgtcg acattcctga gattcctggg 360
ttcaaggact tggagcccat ggagcagttc atcgcacagg tcgatctgtg tgtggactgc 420
acaactggct gcctcaaagg gcttgccaac gtgcagtgtt ctgacctgct caagaagtgg 480
ctgccgcaac gctgtgcgac ctttgccagc aagatccagg gccaggtgga caagatcaag 540
ggggccggtg gtgactaa 558
SEQ ID NO: 4
   length 1299
   molecule type RNA
   division PAT
   Key Location/Qualifiers
   source 1..1299
      /mol_type=other RNA
      /organism=synthetic construct
   Sequence 
ttaaaacagc ctgtgggttg atcccaccca caggcccatt gggcgctagc actctggtat 60
cacggtacct ttgtgcgcct gttttatacc ccctccccca actgtaactt agaagtaaca 120
cacaccgatc aacagtcagc gtggcacacc agccacgttt tgatcaagca cttctgttac 180
cccggactga gtatcaatag actgctcacg cggttgaagg agaaagcgtt cgttatccgg 240
ccaactactt cgaaaaacct agtaacaccg tggaagttgc agagtgtttc gctcagcact 300
accccagtgt agatcaggtc gatgagtcac cgcattcccc acgggcgacc gtggcggtgg 360
ctgcgttggc ggcctgccca tggggaaacc catgggacgc tctaatacag acatggtgcg 420
aagagtctat tgagctagtt ggtagtcctc cggcccctga atgcggctaa tcctaactgc 480
ggagcacaca ccctcaagcc agagggcagt gtgtcgtaac gggcaactct gcagcggaac 540
cgactacttt gggtgtccgt gtttcatttt attcctatac tggctgctta tggtgacaat 600
tgagagatcg ttaccatata gctattggat tggccatccg gtgactaata gagctattat 660
atatcccttt gttgggttta taccacttag cttgaaagag gttaaaacat tacaattcat 720
tgttaagttg aatacagcaa aatgggagtc aaagttctgt ttgccctgat ctgcatcgct 780
gtggccgagg ccaagcccac cgagaacaac gaagacttca acatcgtggc cgtggccagc 840
aacttcgcga ccacggatct cgatgctgac cgcgggaagt tgcccggcaa gaagctgccg 900
ctggaggtgc tcaaagagat ggaagccaat gcccggaaag ctggctgcac caggggctgt 960
ctgatctgcc tgtcccacat caagtgcacg cccaagatga agaagttcat cccaggacgc 1020
tgccacacct acgaaggcga caaagagtcc gcacagggcg gcataggcga ggcgatcgtc 1080
gacattcctg agattcctgg gttcaaggac ttggagccca tggagcagtt catcgcacag 1140
 
gtcgatctgt gtgtggactg cacaactggc tgcctcaaag ggcttgccaa cgtgcagtgt 1200
tctgacctgc tcaagaagtg gctgccgcaa cgctgtgcga cctttgccag caagatccag 1260
ggccaggtgg acaagatcaa gggggccggt ggtgactaa 1299
SEQ ID NO: 5
   length 1299
   molecule type RNA
   division PAT
   Key Location/Qualifiers
   source 1..1299
      /mol_type=other RNA
      /organism=synthetic construct
   Sequence 
cccacccaca ggcccattgg gcgctagcac tctggtatca cggtaccttt gtgcgcctgt 60
tttatacccc ctcccccaac tgtaacttag aagtaacaca caccgatcaa cagtcagcgt 120
ggcacaccag ccacgttttg atcaagcact tctgttaccc cggactgagt atcaatagac 180
tgctcacgcg gttgaaggag aaagcgttcg ttatccggcc aactacttcg aaaaacctag 240
taacaccgtg gaagttgcag agtgtttcgc tcagcactac cccagtgtag atcaggtcga 300
tgagtcaccg cattccccac gggcgaccgt ggcggtggct gcgttggcgg cctgcccatg 360
gggaaaccca tgggacgctc taatacagac atggtgcgaa gagtctattg agctagttgg 420
tagtcctccg gcccctgaat gcggctaatc ctaactgcgg agcacacacc ctcaagccag 480
agggcagtgt gtcgtaacgg gcaactctgc agcggaaccg actactttgg gtgtccgtgt 540
ttcattttat tcctatactg gctgcttatg gtgacaattg agagatcgtt accatatagc 600
tattggattg gccatccggt gactaataga gctattatat atccctttgt tgggtttata 660
ccacttagct tgaaagaggt taaaacatta caattcattg ttaagttgaa tacagcaaaa 720
tgggagtcaa agttctgttt gccctgatct gcatcgctgt ggccgaggcc aagcccaccg 780
agaacaacga agacttcaac atcgtggccg tggccagcaa cttcgcgacc acggatctcg 840
atgctgaccg cgggaagttg cccggcaaga agctgccgct ggaggtgctc aaagagatgg 900
aagccaatgc ccggaaagct ggctgcacca ggggctgtct gatctgcctg tcccacatca 960
agtgcacgcc caagatgaag aagttcatcc caggacgctg ccacacctac gaaggcgaca 1020
aagagtccgc acagggcggc ataggcgagg cgatcgtcga cattcctgag attcctgggt 1080
tcaaggactt ggagcccatg gagcagttca tcgcacaggt cgatctgtgt gtggactgca 1140
caactggctg cctcaaaggg cttgccaacg tgcagtgttc tgacctgctc aagaagtggc 1200
tgccgcaacg ctgtgcgacc tttgccagca agatccaggg ccaggtggac aagatcaagg 1260
gggccggtgg tgactaatta aaacagcctg tgggttgat 1299
SEQ ID NO: 6
   length 165
   molecule type RNA
   division PAT
   Key Location/Qualifiers
   source 1..165
      /mol_type=other RNA
      /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatg 165
SEQ ID NO: 7
   length 151
   molecule type RNA
   division PAT
   Key Location/Qualifiers
   source 1..151
      /mol_type=other RNA /organism=synthetic construct
   Sequence 
aaataattgt cccttaaaga agaaattctt taagtggatg ctctcaaact cagggaaacc 60
taaatctagt tatagacaag gcaatcctga gccaagccga agtagtaatt agtaaggcct 120
agtataccaa accgcgatat ctcttttcta g 151
SEQ ID NO: 8
   length 1615
   molecule type RNA
   division PAT
   Key Location/Qualifiers
   source 1..1615
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac ccacaggccc 180
attgggcgct agcactctgg tatcacggta cctttgtgcg cctgttttat accccctccc 240
ccaactgtaa cttagaagta acacacaccg atcaacagtc agcgtggcac accagccacg 300
ttttgatcaa gcacttctgt taccccggac tgagtatcaa tagactgctc acgcggttga 360
aggagaaagc gttcgttatc cggccaacta cttcgaaaaa cctagtaaca ccgtggaagt 420
tgcagagtgt ttcgctcagc actaccccag tgtagatcag gtcgatgagt caccgcattc 480
cccacgggcg accgtggcgg tggctgcgtt ggcggcctgc ccatggggaa acccatggga 540
cgctctaata cagacatggt gcgaagagtc tattgagcta gttggtagtc ctccggcccc 600
tgaatgcggc taatcctaac tgcggagcac acaccctcaa gccagagggc agtgtgtcgt 660
aacgggcaac tctgcagcgg aaccgactac tttgggtgtc cgtgtttcat tttattccta 720
tactggctgc ttatggtgac aattgagaga tcgttaccat atagctattg gattggccat 780
ccggtgacta atagagctat tatatatccc tttgttgggt ttataccact tagcttgaaa 840
gaggttaaaa cattacaatt cattgttaag ttgaatacag caaaatggga gtcaaagttc 900
tgtttgccct gatctgcatc gctgtggccg aggccaagcc caccgagaac aacgaagact 960
tcaacatcgt ggccgtggcc agcaacttcg cgaccacgga tctcgatgct gaccgcggga 1020
agttgcccgg caagaagctg ccgctggagg tgctcaaaga gatggaagcc aatgcccgga 1080
aagctggctg caccaggggc tgtctgatct gcctgtccca catcaagtgc acgcccaaga 1140
tgaagaagtt catcccagga cgctgccaca cctacgaagg cgacaaagag tccgcacagg 1200
gcggcatagg cgaggcgatc gtcgacattc ctgagattcc tgggttcaag gacttggagc 1260
ccatggagca gttcatcgca caggtcgatc tgtgtgtgga ctgcacaact ggctgcctca 1320
aagggcttgc caacgtgcag tgttctgacc tgctcaagaa gtggctgccg caacgctgtg 1380
cgacctttgc cagcaagatc cagggccagg tggacaagat caagggggcc ggtggtgact 1440
aattaaaaca gcctgtgggt tgataaataa ttgtccctta aagaagaaat tctttaagtg 1500
gatgctctca aactcaggga aacctaaatc tagttataga caaggcaatc ctgagccaag 1560
ccgaagtagt aattagtaag gcctagtata ccaaaccgcg atatctcttt tctag 1615
SEQ ID NO: 9
   length 151
   molecule type RNA
   division PAT
   Key Location/Qualifiers
   source 1..151
      /mol_type=other RNA /organism=synthetic construct
   Sequence 
aaataattga gcc[?]taaaga agaaattctt taagtggatg ctctcaaact cagggaaacc 60
taaatctagt tatagacaag gcaatcctga gccaagccga agtagtaatt agtaaggcct 120
agtataccaa accgcgatat ctcttttcta g 151
SEQ ID NO: 10
   length 1615
   molecule type RNA
   division PAT
   Key Location/Qualifiers
   source 1..1615
      /mol_type=other RNA
      /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac ccacaggccc 180
attgggcgct agcactctgg tatcacggta cctttgtgcg cctgttttat accccctccc 240
ccaactgtaa cttagaagta acacacaccg atcaacagtc agcgtggcac accagccacg 300
ttttgatcaa gcacttctgt taccccggac tgagtatcaa tagactgctc acgcggttga 360
aggagaaagc gttcgttatc cggccaacta cttcgaaaaa cctagtaaca ccgtggaagt 420
tgcagagtgt ttcgctcagc actaccccag tgtagatcag gtcgatgagt caccgcattc 480
cccacgggcg accgtggcgg tggctgcgtt ggcggcctgc ccatggggaa acccatggga 540
cgctctaata cagacatggt gcgaagagtc tattgagcta gttggtagtc ctccggcccc 600
tgaatgcggc taatcctaac tgcggagcac acaccctcaa gccagagggc agtgtgtcgt 660
aacgggcaac tctgcagcgg aaccgactac tttgggtgtc cgtgtttcat tttattccta 720
tactggctgc ttatggtgac aattgagaga tcgttaccat atagctattg gattggccat 780
ccggtgacta atagagctat tatatatccc tttgttgggt ttataccact tagcttgaaa 840
gaggttaaaa cattacaatt cattgttaag ttgaatacag caaaatggga gtcaaagttc 900
tgtttgccct gatctgcatc gctgtggccg aggccaagcc caccgagaac aacgaagact 960
tcaacatcgt ggccgtggcc agcaacttcg cgaccacgga tctcgatgct gaccgcggga 1020
agttgcccgg caagaagctg ccgctggagg tgctcaaaga gatggaagcc aatgcccgga 1080
aagctggctg caccaggggc tgtctgatct gcctgtccca catcaagtgc acgcccaaga 1140
tgaagaagtt catcccagga cgctgccaca cctacgaagg cgacaaagag tccgcacagg 1200
gcggcatagg cgaggcgatc gtcgacattc ctgagattcc tgggttcaag gacttggagc 1260
ccatggagca gttcatcgca caggtcgatc tgtgtgtgga ctgcacaact ggctgcctca 1320
aagggcttgc caacgtgcag tgttctgacc tgctcaagaa gtggctgccg caacgctgtg 1380
cgacctttgc cagcaagatc cagggccagg tggacaagat caaggggccc ggtggtgact 1440
aattaaaaca gcctgtgggt tgataaataa ttgagcctta aagaagaaat tctttaagtg 1500
gatgctctca aactcaggga aacctaaatc tagttataga caaggcaatc ctgagccaag 1560
ccgaagtagt aattagtaag gcctagtata ccaaaccgcg atatctcttt tctag 1615
SEQ ID NO: 11
   length 1615
   molecule type RNA
   division PAT
   Key Location/Qualifiers
   source 1..1615
      /mol_type=other RNA
      /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatggatcc cacccacagg 180
cccattgggc gctagcactc tggtatcacg gtacctttgt gcgcctgttt tataccccct 240
cccccaactg taacttagaa gtaacacaca ccgatcaaca gtcagcgtgg cacaccagcc 300
acgttttgat caagcacttc tgttaccccg gactgagtat caatagactg ctcacgcggt 360
tgaaggagaa agcgttcgtt atccggccaa ctacttcgaa aaacctagta acaccgtgga 420
agttgcagag tgtttcgctc agcactaccc cagtgtagat caggtcgatg agtcaccgca 480
ttccccacgg gcgaccgtgg cggtggctgc gttggcggcc tgcccatggg gaaacccatg 540
ggacgctcta atacagacat ggtgcgaaga gtctattgag ctagttggta gtcctccggc 600
ccctgaatgc ggctaatcct aactgcggag cacacaccct caagccagag ggcagtgtgt 660
 
cgtaacgggc aactctgcag cggaaccgac tactttgggt gtccgtgttt cattttattc 720
ctatactggc tgcttatggt gacaattgag agatcgttac catatagcta ttggattggc 780
catccggtga ctaatagagc tattatatat ccctttgttg ggtttatacc acttagcttg 840
aaagaggtta aaacattaca attcattgtt aagttgaata cagcaaaatg ggagtcaaag 900
ttctgtttgc cctgatctgc atcgctgtgg ccgaggccaa gcccaccgag aacaacgaag 960
acttcaacat cgtggccgtg gccagcaact tcgcgaccac ggatctcgat gctgaccgcg 1020
ggaagttgcc cggcaagaag ctgccgctgg aggtgctcaa agagatggaa gccaatgccc 1080
ggaaagctgg ctgcaccagg ggctgtctga tctgcctgtc ccacatcaag tgcacgccca 1140
agatgaagaa gttcatccca ggacgctgcc acacctacga aggcgacaaa gagtccgcac 1200
agggcggcat aggcgaggcg atcgtcgaca ttcctgagat tcctgggttc aaggacttgg 1260
agcccatgga gcagttcatc gcacaggtcg atctgtgtgt ggactgcaca actggctgcc 1320
tcaaagggct tgccaacgtg cagtgttctg acctgctcaa gaagtggctg ccgcaacgct 1380
gtgcgacctt tgccagcaag atccagggcc aggtggacaa gatcaagggg gccggtggtg 1440
actaattaaa acagcctgtg ggttaaataa ttgaccctta aagaagaaat tctttaagtg 1500
gatgctctca aactcaggga aacctaaatc tagttataga caaggcaatc ctgagccaag 1560
ccgaagtagt aattagtaag gcctagtata ccaaaccgcg atatctcttt tctag 1615
SEQ ID NO: 12
   length 1615
   molecule type RNA
   division PAT
   Key Location/Qualifiers
   source 1..1615
      /mol_type=other RNA
      /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatggggcg ctagcactct 180
ggtatcacgg tacctttgtg cgcctgtttt ataccccctc ccccaactgt aacttagaag 240
taacacacac cgatcaacag tcagcgtggc acaccagcca cgttttgatc aagcacttct 300
gttaccccgg actgagtatc aatagactgc tcacgcggtt gaaggagaaa gcgttcgtta 360
tccggccaac tacttcgaaa aacctagtaa caccgtggaa gttgcagagt gtttcgctca 420
gcactacccc agtgtagatc aggtcgatga gtcaccgcat tccccacggg cgaccgtggc 480
ggtggctgcg ttggcggcct gcccatgggg aaacccatgg gacgctctaa tacagacatg 540
gtgcgaagag tctattgagc tagttggtag tcctccggcc cctgaatgcg gctaatccta 600
actgcggagc acacaccctc aagccagagg gcagtgtgtc gtaacgggca actctgcagc 660
ggaaccgact actttgggtg tccgtgtttc attttattcc tatactggct gcttatggtg 720
acaattgaga gatcgttacc atatagctat tggattggcc atccggtgac taatagagct 780
attatatatc cctttgttgg gtttatacca cttagcttga aagaggttaa aacattacaa 840
ttcattgtta agttgaatac agcaaaatgg gagtcaaagt tctgtttgcc ctgatctgca 900
tcgctgtggc cgaggccaag cccaccgaga acaacgaaga cttcaacatc gtggccgtgg 960
ccagcaactt cgcgaccacg gatctcgatg ctgaccgcgg gaagttgccc ggcaagaagc 1020
tgccgctgga ggtgctcaaa gagatggaag ccaatgcccg gaaagctggc tgcaccaggg 1080
gctgtctgat ctgcctgtcc cacatcaagt gcacgcccaa gatgaagaag ttcatcccag 1140
gacgctgcca cacctacgaa ggcgacaaag agtccgcaca gggcggcata ggcgaggcga 1200
tcgtcgacat tcctgagatt cctgggttca aggacttgga gcccatggag cagttcatcg 1260
cacaggtcga tctgtgtgtg gactgcacaa ctggctgcct caaagggctt gccaacgtgc 1320
agtgttctga cctgctcaag aagtggctgc cgcaacgctg tgcgaccttt gccagcaaga 1380
tccagggcca ggtggacaag atcaaggggg ccggtggtga ctaattaaaa cagcctgtgg 1440
gttgatccca cccacaggcc cattaaataa ttgatcctta aagaagaaat tctttaagtg 1500
gatgctctca aactcaggga aacctaaatc tagttataga caaggcaatc ctgagccaag 1560
ccgaagtagt aattagtaag gcctagtata ccaaaccgcg atatctcttt tctag 1615
SEQ ID NO: 13
   length 20
   molecule type DNA
   division PAT
   Key Location/Qualifiers
   source 1..20
      /mol_type=other DNA /organism=synthetic construct
   Sequence
      tgtgcgatga actgctccat 20
SEQ ID NO: 14
   length 22
   molecule type DNA
   division PAT
   Key Location/Qualifiers
   source 1..22
      /mol_type=other DNA /organism=synthetic construct
   Sequence
      aagaagttca tcccaggacg ct 22
SEQ ID NO: 15
   length 20
   molecule type DNA
   division PAT
   Key Location/Qualifiers
   source 1..20
      /mol_type=other DNA /organism=synthetic construct
   Sequence
      ggccacgatg ttgaagtctt 20
SEQ ID NO: 16
   length 1615
   molecule type RNA
   division PAT
   Key Location/Qualifiers
   source 1..1615
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac ccacaggccc 180
attgggcgct agcactctgg tatcacggta cctttgtgcg cctgttttat accccctccc 240
ccaactgtaa cttagaagta acacacaccg atcaacagtc agcgtggcac accagccacg 300
ttttgatcaa gcacttctgt taccccggac tgagtatcaa tagactgctc acgcggttga 360
aggagaaagc gttcgttatc cggccaacta cttcgaaaaa cctagtaaca ccgtggaagt 420
tgcagagtgt ttcgctcagc actaccccag tgtagatcag gtcgatgagt caccgcattc 480
cccacgggcg accgtggcgg tggctgcgtt ggcggcctgc ccatggggaa acccatggga 540
cgctctaata cagacatggt gcgaagagtc tattgagcta gttggtagtc ctccggcccc 600
tgaatgcggc taatcctaac tgcggagcac acaccctcaa gccagagggc agtgtgtcgt 660
aacgggcaac tctgcagcgg aaccgactac tttgggtgtc cgtgtttcat tttattccta 720
tactggctgc ttatggtgac aattgagaga tcgttaccat atagctattg gattggccat 780
ccggtgacta atagagctat tatatatccc tttgttgggt ttataccact tagcttgaaa 840
gaggttaaaa cattacaatt cattgttaag ttgaatacag caaaatggga gtcaaagttc 900
tgtttgccct gatctgcatc gctgtggccg aggccaagcc caccgagaac aacgaagact 960
 
tcaacatcgt ggccgtggcc agcaacttcg cgaccacgga tctcgatgct gaccgcggga 1020
agttgcccgg caagaagctg ccgctggagg tgctcaaaga gatggaagcc aatgcccgga 1080
aagctggctg caccaggggc tgtctgatct gcctgtccca catcaagtgc acgcccaaga 1140
tgaagaagtt catcccagga cgctgccaca cctacgaagg cgacaaagag tccgcacagg 1200
gcggcatagg cgaggcgatc gtcgacattc ctgagattcc tgggttcaag gacttggagc 1260
ccatggagca gttcatcgca caggtcgatc tgtgtgtgga ctgcacaact ggctgcctca 1320
aagggcttgc caacgtgcag tgttctgacc tgctcaagaa gtggctgccg caacgctgtg 1380
cgacctttgc cagcaagatc cagggccagg tggacaagat caaggggccc ggtggtgact 1440
aattaaaaca gcctgtgggt tgataaataa ttgtcaacta aagaagaaat tctttaagtg 1500
gatgctctca aactcaggga aacctaaatc tagttataga caaggcaatc ctgagccaag 1560
ccgaagtagt aattagtaag gcctagtata ccaaaccgcg atatctcttt tctag 1615
SEQ ID NO: 17
   length 1615
   molecule type RNA
   division PAT
   Key Location/Qualifiers
   source 1..1615
      /mol_type=other RNA /organism=synthetic construct
Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac ccacaggccc 180
attgggcgct agcactctgg tatcacggta cctttgtgcg cctgttttat acccctcccc 240
ccaactgtaa cttagaagta acacacaccg atcaacagtc agcgtggcac accagccacg 300
ttttgatcaa gcacttctgt taccccggac tgagtatcaa tagactgctc acgcggttga 360
aggagaaagc gttcgttatc cggccaacta cttcgaaaaa cctagtaaca ccgtggaagt 420
tgcagagtgt ttcgctcagc actaccccag tgtagatcag gtcgatgagt caccgcattc 480
cccacgggcg accgtggcgg tggctgcgtt ggcggcctgc ccatggggaa acccatggga 540
cgctctaata cagacatggt gcgaagagtc tattgagcta gttggtagtc ctccggcccc 600
tgaatgcggc taatcctaac tgcggagcac acaccctcaa gccagagggc agtgtgtcgt 660
aacgggcaac tctgcagcgg aaccgactac tttgggtgtc cgtgtttcat tttattccta 720
tactggctgc ttatggtgac aattgagaga tcgttaccat atagctattg gattggccat 780
ccggtgacta atagagctat tatatatccc tttgttgggt ttataccact tagcttgaaa 840
gaggttaaaa cattacaatt cattgttaag ttgaatacag caaaatggga gtcaaagttc 900
tgtttgccct gatctgcatc gctgtggccg aggccaagcc caccgagaac aacgaagact 960
tcaacatcgt ggccgtggcc agcaacttcg cgaccacgga tctcgatgct gaccgcggga 1020
agttgcccgg caagaagctg ccgctggagg tgctcaaaga gatggaagcc aatgcccgga 1080
aagctggctg caccaggggc tgtctgatct gcctgtccca catcaagtgc acgcccaaga 1140
tgaagaagtt catcccagga cgctgccaca cctacgaagg cgacaaagag tccgcacagg 1200
gcggcatagg cgaggcgatc gtcgacattc ctgagattcc tgggttcaag gacttggagc 1260
ccatggagca gttcatcgca caggtcgatc tgtgtgtgga ctgcacaact ggctgcctca 1320
aagggcttgc caacgtgcag tgttctgacc tgctcaagaa gtggctgccg caacgctgtg 1380
cgacctttgc cagcaagatc cagggccagg tggacaagat caagggggcc ggtggtgact 1440
aattaaaaca gcctgtgggt tgataaataa ttgtcaaccc aagaagaaat tctttaagtg 1500
gatgctctca aactcaggga aacctaaatc tagttataga caaggcaatc ctgagccaag 1560
ccgaagtagt aattagtaag gcctagtata ccaaaccgcg atatctcttt tctag 1615
SEQ ID NO: 18
   length 1615
   molecule type RNA
   division PAT
   Key Location/Qualifiers
   source 1..1615
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac ccacaggccc 180
attgggcgct agcactctgg tatcacggta cctttgtgcg cctgttttat accccctccc 240
ccaactgtaa cttagaagta acacacaccg atcaacagtc agcgtggcac accagccacg 300
ttttgatcaa gcacttctgt taccccggac tgagtatcaa tagactgctc acgcggttga 360
aggagaaagc gttcgttatc cggccaacta cttcgaaaaa cctagtaaca ccgtggaagt 420
tgcagagtgt ttcgctcagc actaccccag tgtagatcag gtcgatgagt caccgcattc 480
cccacgggcg accgtggcgg tggctgcgtt ggcggcctgc ccatggggaa acccatggga 540
cgctctaata cagacatggt gcgaagagtc tattgagcta gttggtagtc ctccggcccc 600
tgaatgcggc taatcctaac tgcggagcac acaccctcaa gccagagggc agtgtgtcgt 660
aacgggcaac tctgcagcgg aaccgactac tttgggtgtc cgtgtttcat tttattccta 720
tactggctgc ttatggtgac aattgagaga tcgttaccat atagctattg gattggccat 780
ccggtgacta atagagctat tatatatccc tttgttgggt ttataccact tagcttgaaa 840
gaggttaaaa cattacaatt cattgttaag ttgaatacag caaaatggga gtcaaagttc 900
tgtttgccct gatctgcatc gctgtggccg aggccaagcc caccgagaac aacgaagact 960
tcaacatcgt ggccgtggcc agcaacttcg cgaccacgga tctcgatgct gaccgcggga 1020
agttgcccgg caagaagctg ccgctggagg tgctcaaaga gatggaagcc aatgcccgga 1080
aagctggctg caccaggggc tgtctgatct gcctgtccca catcaagtgc acgcccaaga 1140
tgaagaagtt catcccagga cgctgccaca cctacgaagg cgacaaagag tccgcacagg 1200
gcggcatagg cgaggcgatc gtcgacattc ctgagattcc tgggttcaag gacttggagc 1260
ccatggagca gttcatcgca caggtcgatc tgtgtgtgga ctgcacaact ggctgcctca 1320
aagggcttgc caacgtgcag tgttctgacc tgctcaagaa gtggctgccg caacgctgtg 1380
cgacctttgc cagcaagatc cagggccagg tggacaagat caaggggccc ggtggtgact 1440
aattaaaaca gcctgtgggt tgataaataa ttgtcaaccc acaaagaaat tctttaagtg 1500
gatgctctca aactcaggga aacctaaatc tagttataga caaggcaatc ctgagccaag 1560
ccgaagtagt aattagtaag gcctagtata ccaaaccgcg atatctcttt tctag 1615
SEQ ID NO: 19
   length 95
   molecule type RNA
   division PAT
   Key Location/Qualifiers
   source 1..95
      /mol_type=other RNA /organism=synthetic construct
   Sequence 
ttaaaacagc ggatgggtac cccaccatcc gacccactgg gtgtagtact ctggtacttc 60
gtacctttgt acgcctgttc ttcccattgt accct 95
SEQ ID NO: 20
   length 95
   molecule type RNA
   division PAT
   Key Location/Qualifiers
   source 1..95
      /mol_type=other RNA /organism=synthetic construct
   Sequence 
ttaaaacagc ctgtgggttg ttcccacccg cagggcccac tgggcgctag cacactggta 60
tcccggtacc cttgtgcgcc tgttttatat accct 95
SEQ ID NO: 21
   length 96
   molecule type RNA
   division PAT
   Key Location/Qualifiers
   source 1..96
      /mol_type=other RNA /organism=synthetic construct
   Sequence 
ttaaaacagc tctggggttg ctcccacccc agaggcccac gtggcggcca gtactctggt 60
attgcggtac ctttgtacgc ctgttttata ctccct 96
SEQ ID NO: 22
   length 93
   molecule type RNA
   division PAT
   Key Location/Qualifiers
   source 1..93
      /mol_type=other RNA /organism=synthetic construct
   Sequence 
ttaaaatagc tgctgggttg ttcccatcca gcaggcccac tgggcgtgat actctggtat 60
tgcggtacct ttgtatgcct attttacctc cct 93
SEQ ID NO: 23
   length 91
   molecule type RNA
   division PAT
   Key Location/Qualifiers
   source 1..91
      /mol_type=other RNA /organism=synthetic construct
   Sequence 
ttaaaacagc ctgggggttg tacccacccc tggggcccac gcggcgccag tactctggta 60
cgctagtacc tttgtacgcc tgttttcccc a 91
SEQ ID NO: 24
   length 626
   molecule type RNA
   division PAT
   Key Location/Qualifiers
   source 1..626
      /mol_type=other RNA /organism=synthetic construct
   Sequence 
ttaaaacagc ggatgggtac cccaccatcc gacccactgg gtgtagtact ctggtacttc 60
gtacctttgt acgcctgttc ttcccattgt acccttcctg aacttccaac ccaagtaacg 120
ttagaagctc aacatttagt acaacaggaa gcaccacatc cagtggtgtt tagtacaagc 180
acttctgttt ccccggagcg aggtataggc tgtacccact gccaaaaacc tttaaccgtt 240
atccgccaac caactacgta aaagctagta gtattatgtt tttaactagg cgttcgatca 300
ggtggatttc ccctccacta gtttggtcga tgaggctagg aattccccac gggtgaccgt 360
gtcctagcct gcgtggcggc caacccagct tatgctggga cgccttttta tagacatggt 420
gtgaagactc gcatgtgctt ggttgtgatt cctccggccc ctgaatgcgg ctaaccttaa 480
ccctggagcc ttgtgtcaca aaccagtgat gataaggtcg taatgagcaa ttccgggacg 540
ggaccgacta ctttgggtgt ccgtgtttct tatttttctt attattgtct tatggtcaca 600
gcatatatat aacatatact gtgatc 626
SEQ ID NO: 25
   length 743
   molecule type RNA
   division PAT
   Key Location/Qualifiers
   source 1..743
      /mol_type=other RNA /organism=synthetic construct
   Sequence 
ttaaaacagc ctgtgggttg ttcccacccg cagggcccac tgggcgctag cacactggta 60
tcccggtacc cttgtgcgcc tgttttatat accctccccc ttatgtaact tagaagtatg 120
attcaaacgg tcgacaggcg gctcagtgca ccaactgagt catgaccaag cacttctgtt 180
accccggact gagtatcaat aagctgttca cacggctgaa ggagaaaacg ttcgttaccc 240
ggccaattac ttcgagaaac ctagtaccac catgaaggtt gcgcagtgtt tcgctccaca 300
caaccccagt gtagatcagg tcgatgagtc accgcattcc ccacgggcga ccgtggcggt 360
ggctgcgttg gcggcctgcc catggggcaa cccatgggac gcttcaatac tgacatggtg 420
tgaagagtct attgagctaa ttggtagtcc tccggcccct gaatgcggct aatcccaact 480
gtggagcaga tactcacaaa ccagtgagcg gtctgtcgta acgggcaact ccgcagcgga 540
accgactact ttgggtgtcc gtgtttcttt ttattcttac attggctgct tatggtgaca 600
attgacaaat tgttaccata tagctattgg attggccatc cggtgacaaa cagagctatt 660
gtttacttgt ttgttggttt cataccatta aattacaagg tcttagaaac tctcaacttt 720
attttgacac tcaatacagc aaa 743
SEQ ID NO: 26
   length 747
   molecule type RNA
   division PAT
   Key Location/Qualifiers
   source 1..747
      /mol_type=other RNA /organism=synthetic construct
   Sequence 
ttaaaacagc tctggggttg ctcccacccc agaggcccac gtggcggcca gtactctggt 60
attgcggtac ctttgtacgc ctgttttata ctccctcccc ccgtgcaact ttagaagaaa 120
tccacaaagt tcaatagagg gggtgcaaac cagcaccacc acgaacaagc acttctgttt 180
ccccggtgat gtcgtataag ctgtacccac ggctgaaggc gacggatccg ttatccgctt 240
gagtacttcg agaagcctag tattaccttg gaatcttcga cgcgttgcgc tcagcactct 300
accccgagtg tagcttaggt cgatgagtct gggcacgccc caccggcgac ggtggcccag 360
gctgcgttgg cggcctaccc atggctatca ccatgggacg ctagttgtga acaaggtgtg 420
aagagcctat tgagctaccc aagagtcctc cggcccctga atgcggctaa tcctaaccac 480
ggagcaagtg tcctcaaccc aggggatggc ttgtcgtaac gcgaaagtct gtggcggaac 540
cgactacttt gggtgtccgt gtttcctttt atttttatgt atggctgctt atggtgacaa 600
tcaaaggttg ttaccataaa gcaatttgga ttggccatcc ggtgagaatc aaacatatta 660
tctacctgtt tgttgggttt tcttctttta cttgaacaat acctctaata ataactgcta 720
tattgtcaat aagacattat catcaca 747
SEQ ID NO: 27
   length 666
   molecule type RNA
   division PAT
   Key Location/Qualifiers
   source 1..666
      /mol_type=other RNA /organism=synthetic construct
   Sequence 
ttaaaatagc tgctgggttg ttcccatcca gcaggcccac tgggcgtgat actctggtat 60
tgcggtacct ttgtatgcct attttacctc ccttccccct gaaacttaga agaaagaaac 120
aaacacgctc actaggtaca ctgcatccag cagtgtaatg agcaagcact tctgtttccc 180
cggaagggat atatggtacg ctgtgcaaac ggcggaaatt aatcctaccg ttaaccgccc 240
atctactccg agaagcctag tacctaattg aacttatcaa tggagttgcg ctcagcaggt 300
gaccctgacc tgccagctcc ggctgatgga cctgggcatc ccccacaggc gactgtggcc 360
caggtcgcgt ggcggccggc ccactctttt gagtgggacg ccttgataat gacaaggtgg 420
gaagagccta ttgggctagc tggtttcctc cggcctcctg aatgcggcta accctaaccc 480
cggagcatat ggtggcaacc cagccactgg tatgtcgtaa cgcgtaagtc tgggatggga 540
ccgactactt tggagagtcc gtgtttccat tattttcttt attgaatctt atggtgacaa 600
attgggagtt actcgggtga cgattgatta ttacttctga aaacgtagag atatagaatc 660
aacaca 666
SEQ ID NO: 28
   length 812
   molecule type RNA
   division PAT
   Key Location/Qualifiers
   source 1..812
      /mol_type=other RNA /organism=synthetic construct
   Sequence 
ttaaaacagc ctgggggttg tacccacccc tggggcccac gcggcgccag tactctggta 60
cgctagtacc tttgtacgcc tgttttcccc acccttaaat aaattaagat taccactgct 120
gtggggagta gtccgactcc gcaccgatac gtcgcaccag tgaactggtt cgcttagaac 180
ctttgcacgg agcagctggt atcccccccc gtaacttaga agcctggaca aaccgaccaa 240
tagaggcgtt gtagccagca gcgcaacggt caagcacttc tgtttccccg gcgccatgcg 300
tcgttacccg cccggcctac tgcgggaagc ctagtaggac cccaatcgga cgcgcggttg 360
cgttcagcca caaccccagt ggtagctctg aggaatggga ctcgcctacc ccccacagca 420
atgtggtggc ttgtccgcgt gtgtcctcgg gttcgctcgt tgagcgatca ccgcaactct 480
gagtaaggtc tcaagagcct actgcgctag gtcggttctc ctccggagcc gtgaatgctg 540
ctaatcccaa cctccgagcg tgtgcgcaca acccagtgtt gctacgtcgt aatgcgtaag 600
ttggaggcgg aacagactac tttcggtact ccgtgtttcc ttaatttttg ttcattatta 660
catggtgaca ttgactgaca tttgtgaatt tgcccgcctg cccttgaaca tagctctcta 720
ttacttgatt gcatttcaca aaacaaccca actcactgtt gaacttgttg acttcgcagt 780
ctacttgaac ttacagtaca atcacattca ca 812
SEQ ID NO: 29
   length 1500
   molecule type RNA
   division PAT
   Key Location/Qualifiers
   source 1..1500
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgacccc accatccgac 180
ccactgggtg tagtactctg gtacttcgta cctttgtacg cctgttcttc ccattgtacc 240
cttcctgaac ttccaaccca agtaacgtta gaagctcaac atttagtaca acaggaagca 300
ccacatccag tggtgtttag tacaagcact tctgtttccc cggagcgagg tataggctgt 360
acccactgcc aaaaaccttt aaccgttatc cgccaaccaa ctacgtaaaa gctagtagta 420
ttatgttttt aactaggcgt tcgatcaggt ggatttcccc tccactagtt tggtcgatga 480
ggctaggaat tccccacggg tgaccgtgtc ctagcctgcg tggcggccaa cccagcttat 540
gctgggacgc ctttttatag acatggtgtg aagactcgca tgtgcttggt tgtgattcct 600
 
ccggcccctg aatgcggcta accttaaccc tggagccttg tgtcacaaac cagtgatgat 660
aaggtcgtaa tgagcaattc cggacgggga ccgactactt tgggtgtccg tgtttcttat 720
ttttcttatt attgtcttat ggtcacagca tatatataac atatactgtg atcatgggag 780
tcaaagttct gtttgccctg atctgcatcg ctgtggccga ggccaagccc accgagaaca 840
acgaagactt caacatcgtg gccgtggcca gcaacttcgc gaccacggat ctcgatgctg 900
accgcgggaa gttgcccggc aagaagctgc cgctggaggt gctcaaagag atggaagcca 960
atgcccggaa agctggctgc accaggggct gtctgatctg cctgtcccac atcaagtgca 1020
cgcccaagat gaagaagttc atcccaggac gctgccacac ctacgaaggc gacaaagagt 1080
ccgcacaggg cggcataggc gaggcgatcg tcgacattcc tgagattcct gggttcaagg 1140
acttggagcc catggagcag ttcatcgcac aggtcgatct gtgtgtggac tgcacaactg 1200
gctgcctcaa agggcttgcc aacgtgcagt gttctgacct gctcaagaag tggctgccgc 1260
aacgctgtgc gacctttgcc agcaagatcc agggccaggt ggacaagatc aagggggccg 1320
gtggtgacta attaaaacag cggatgggta aataattgcc ccttaaagaa gaaattcttt 1380
aagtggatgc tctcaaactc agggaaacct aaatctagtt atagacaagg caatcctgag 1440
ccaagccgaa gtagtaatta gtaaggccta gtataccaaa ccgcgatatc tcttttctag 1500
SEQ ID NO: 30
   length 1617
   molecule type RNA
   division PAT
   Key Location/Qualifiers
   source 1..1617
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac ccgcagggcc 180
cactgggcgc tagcacactg gtatcccggt acccttgtgc gcctgtttta tataccctcc 240
cccttatgta acttagaagt atgattcaaa cggtcgacag gcggctcagt gcaccaactg 300
agtcatgacc aagcacttct gttaccccgg actgagtatc aataagctgt tcacacggct 360
gaaggagaaa acgttcgtta cccggccaat tacttcgaga aacctagtac caccatgaag 420
gttgcgcagt gtttcgctcc acacaacccc agtgtagatc aggtcgatga gtcaccgcat 480
tccccacggg cgaccgtggc ggtggctgcg ttggcggcct gcccatgggg caacccatgg 540
gacgcttcaa tactgacatg gtgtgaagag tctattgagc taattggtag tcctccggcc 600
cctgaatgcg gctaatccca actgtggagc agatactcac aaaccagtga gcggtctgtc 660
gtaacgggca actccgcagc ggaaccgact actttgggtg tccgtgtttc tttttattct 720
tacattggct gcttatggtg acaattgaca aattgttacc atatagctat tggattggcc 780
atccggtgac aaacagagct attgtttact tgtttgttgg tttcatacca ttaaattaca 840
aggtcttaga aactctcaac tttattttga cactcaatac agcaaaatgg gagtcaaagt 900
tctgtttgcc ctgatctgca tcgctgtggc cgaggccaag cccaccgaga acaacgaaga 960
cttcaacatc gtggccgtgg ccagcaactt cgcgaccacg gatctcgatg ctgaccgcgg 1020
gaagttgccc ggcaagaagc tgccgctgga ggtgctcaaa gagatggaag ccaatgcccg 1080
gaaagctggc tgcaccaggg gctgtctgat ctgcctgtcc cacatcaagt gcacgcccaa 1140
gatgaagaag ttcatcccag gacgctgcca cacctacgaa ggcgacaaag agtccgcaca 1200
gggcggcata ggcgaggcga tcgtcgacat tcctgagatt cctgggttca aggacttgga 1260
gcccatggag cagttcatcg cacaggtcga tctgtgtgtg gactgcacaa ctggctgcct 1320
caaagggctt gccaacgtgc agtgttctga cctgctcaag aagtggctgc cgcaacgctg 1380
tgcgaccttt gccagcaaga tccagggcca ggtggacaag atcaaggggg ccggtggtga 1440
ctaattaaaa cagcctgtgg gttgttaaat aattgaccct taaagaagaa attctttaag 1500
tggatgctct caaactcagg gaaacctaaa tctagttata gacaaggcaa tcctgagcca 1560
agccgaagta gtaattagta aggcctagta taccaaaccg cgatatctct tttctag 1617
SEQ ID NO: 31
   length 1621
   molecule type RNA
   division PAT
   Key Location/Qualifiers
   source 1..1621
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac cccagaggcc 180
cacgtggcgg ccagtactct ggtattgcgg tacctttgta cgcctgtttt atactccctc 240
cccccgtgca actttagaag aaatccacaa agttcaatag aggggtgcaa aaccagcacc 300
accacgaaca agcacttctg tttccccggt gatgtcgtat aagctgtacc cacggctgaa 360
ggcgacggat ccgttatccg cttgagtact tcgagaagcc tagtattacc ttggaatctt 420
cgacgcgttg cgctcagcac tctaccccga gtgtagctta ggtcgatgag tctgggcacg 480
ccccaccggc gacggtggcc caggctgcgt tggcggccta cccatggcta tcaccatggg 540
acgctagttg tgaacaaggt gtgaagagcc tattgagcta cccaagagtc ctccggcccc 600
tgaatgcggc taatcctaac cacggagcaa gtgtcctcaa cccaggggat ggcttgtcgt 660
aacgcgaaag tctgtggcgg aaccgactac tttgggtgtc cgtgtttcct tttattttta 720
tgtatggctg cttatggtga caatcaaagg ttgttaccat aaagcaattt ggattggcca 780
tccggtgaga atcaaacata ttatctacct gtttgttggg ttttcttctt ttacttgaac 840
aatacctcta ataataactg ctatattgtc aataagacat tatcatcaca atgggagtca 900
aagttctgtt tgccctgatc tgcatcgctg tggccgaggc caagcccacc gagaacaacg 960
aagacttcaa catcgtggcc gtggccagca acttcgcgac cacggatctc gatgctgacc 1020
gcgggaagtt gcccggcaag aagctgccgc tggaggtgct caaagagatg gaagccaatg 1080
cccggaaagc tggctgcacc aggggctgtc tgatctgcct gtcccacatc aagtgcacgc 1140
ccaagatgaa gaagttcatc ccaggacgct gccacaccta cgaaggcgac aaagagtccg 1200
cacagggcgg cataggcgag gcgatcgtcg acattcctga gattcctggg ttcaaggact 1260
tggagcccat ggagcagttc atcgcacagg tcgatctgtg tgtggactgc acaactggct 1320
gcctcaaagg gcttgccaac gtgcagtgtt ctgacctgct caagaagtgg ctgccgcaac 1380
gctgtgcgac ctttgccagc aagatccagg gccaggtgga caagatcaag ggggccggtg 1440
gtgactaatt aaaacagctc tggggttgct aaataattgg cccttaaaga agaaattctt 1500
taagtggatg ctctcaaact cagggaaacc taaatctagt tatagacaag gcaatcctga 1560
gccaagccga agtagtaatt agtaaggcct agtataccaa accgcgatat ctcttttcta 1620
g 1621
SEQ ID NO: 32
   length 1540
   molecule type RNA
   division PAT
   Key Location/Qualifiers
   source 1..1540
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccat ccagcaggcc 180
cactgggcgt gatactctgg tattgcggta cctttgtatg cctattttac ctcccttccc 240
cctgaaactt agaagaaaga aacaaacacg ctcactaggt acactgcatc cagcagtgta 300
atgagcaagc acttctgttt ccccggaagg gatatatggt acgctgtgca aacggcggaa 360
attaatccta ccgttaaccg cccatctact ccgagaagcc tagtacctaa ttgaacttat 420
caatggagtt gcgctcagca ggtgaccctg acctgccagc tccggctgat ggacctgggc 480
atcccccaca ggcgactgtg gcccaggtcg cgtggcggcc ggcccactct tttgagtggg 540
acgccttgat aatgacaagg tgggaagagc ctattgggct agctggtttc ctccggcctc 600
ctgaatgcgg ctaaccctaa ccccggagca tatggtggca acccagccac tggtatgtcg 660
taacgcgtaa gtctgggatg ggaccgacta ctttggagag tccgtgtttc cattattttc 720
tttattgaat cttatggtga caaattggga gttactcggg tgacgattga ttattacttc 780
tgaaaacgta gagatataga atcaacacaa tgggagtcaa agttctgttt gccctgatct 840
 
gcatcgctgt ggccgaggcc aagcccaccg agaacaacga agacttcaac atcgtggccg 900
tggccagcaa cttcgcgacc acggatctcg atgctgaccg cgggaagttg cccggcaaga 960
agctgccgct ggaggtgctc aaagagatgg aagccaatgc ccggaaagct ggctgcacca 1020
ggggctgtct gatctgcctg tcccacatca agtgcacgcc caagatgaag aagttcatcc 1080
caggacgctg ccacacctac gaaggcgaca aagagtccgc acagggcggc ataggcgagg 1140
cgatcgtcga cattcctgag attcctgggt tcaaggactt ggagcccatg gagcagttca 1200
tcgcacaggt cgatctgtgt gtggactgca caactggctg cctcaaaggg cttgccaacg 1260
tgcagtgttc tgacctgctc aagaagtggc tgccgcaacg ctgtgcgacc tttgccagca 1320
agatccaggg ccaggtggac aagatcaagg gggccggtgg tgactaatta aaatagctgc 1380
tgggttgtta aataattgac ccttaaagaa gaaattcttt aagtggatgc tctcaaactc 1440
agggaaacct aaatctagtt atagacaagg caatcctgag ccaagccgaa gtagtaatta 1500
gtaaggccta gtataccaaa ccgcgatatc tcttttctag 1540
SEQ ID NO: 33
   length 1686
   molecule type RNA
   division PAT
   Key Location/Qualifiers
   source 1..1686
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgaccca cccctggggc 180
ccacgcggcg ccagtactct ggtacgctag tacctttgta cgcctgtttt ccccaccctt 240
aaataaatta agattaccac tgctgtgggg agtagtccga ctccgcaccg atacgtcgca 300
ccagtgaact ggttcgctta gaacctttgc acggagcagc tggtatcccc ccccgtaact 360
tagaagcctg gacaaaccga ccaatagagg cgttgtagcc agcagcgcaa cggtcaagca 420
cttctgtttc cccggcgcca tgcgtcgtta cccgcccggc ctactgcggg aagcctagta 480
ggaccccaat cggacgcgcg gttgcgttca gccacaaccc cagtggtagc tctgaggaat 540
gggactcgcc taccccccac agcaatgtgg tggcttgtcc gcgtgtgtcc tcgggttcgc 600
tcgttgagcg atcaccgcaa ctctgagtaa ggtctcaaga gcctactgcg ctaggtcggt 660
tctcctccgg agccgtgaat gctgctaatc ccaacctccg agcgtgtgcg cacaacccag 720
tgttgctacg tcgtaatgcg taagttggag gcggaacaga ctactttcgg tactccgtgt 780
ttccttaatt tttgttcatt attacatggt gacattgact gacatttgtg aatttgcccg 840
cctgcccttg aacatagctc tctattactt gattgcattt cacaaaacaa cccaactcac 900
tgttgaactt gttgacttcg cagtctactt gaacttacag tacaatcaca ttcacaatgg 960
gagtcaaagt tctgtttgcc ctgatctgca tcgctgtggc cgaggccaag cccaccgaga 1020
acaacgaaga cttcaacatc gtggccgtgg ccagcaactt cgcgaccacg gatctcgatg 1080
ctgaccgcgg gaagttgccc ggcaagaagc tgccgctgga ggtgctcaaa gagatggaag 1140
ccaatgcccg gaaagctggc tgcaccaggg gctgtctgat ctgcctgtcc cacatcaagt 1200
gcacgcccaa gatgaagaag ttcatcccag gacgctgcca cacctacgaa ggcgacaaag 1260
agtccgcaca gggcggcata ggcgaggcga tcgtcgacat tcctgagatt cctgggttca 1320
aggacttgga gcccatggag cagttcatcg cacaggtcga tctgtgtgtg gactgcacaa 1380
ctggctgcct caaagggctt gccaacgtgc agtgttctga cctgctcaag aagtggctgc 1440
cgcaacgctg tgcgaccttt gccagcaaga tccagggcca ggtggacaag atcaaggggg 1500
ccggtggtga ctaattaaaa cagcctgggg gttgtaaata attgcacctt aaagaagaaa 1560
ttctttaagt ggatgctctc aaactcaggg aaacctaaat ctagttatag acaaggcaat 1620
cctgagccaa gccgaagtag taattagtaa ggcctagtat accaaaccgc gatatctctt 1680
ttctag 1686
SEQ ID NO: 34
   length 587
   molecule type RNA
   division PAT
   Key Location/Qualifiers
   source 1..587
      /mol_type=other RNA /organism=synthetic construct
   Sequence 
cccctctccc tccccccccc ctaacgttac tggccgaagc cgcttggaat aaggccggtg 60
tgcgtttgtc tatatgttat tttccaccat attgccgtct tttggcaatg tgagggcccg 120
gaaacctggc cctgtcttct tgacgagcat tcctaggggt ctttcccctc tcgccaaagg 180
aatgcaaggt ctgttgaatg tcgtgaagga agcagttcct ctggaagctt cttgaagaca 240
aacaacgtct gtagcgaccc tttgcaggca gcggaacccc ccacctggcg acaggtgcct 300
ctgcggccaa aagccacgtg tataagatac acctgcaaag gcggcacaac cccagtgcca 360
cgttgtgagt tggatagttg tggaaagagt caaatggctc tcctcaagcg tattcaacaa 420
ggggctgaag gatgcccaga aggtacccca ttgtatggga tctgatctgg ggcctcggta 480
cacatgcttt acatgtgttt agtcgaggtt aaaaaaacgt ctaggccccc cgaaccacgg 540
ggacgtggtt ttcctttgaa aaacacgatg ataatatggc cacaacc 587
SEQ ID NO: 35
   length 223
   molecule type RNA
   division PAT
   Key Location/Qualifiers
   source 1..223
      /mol_type=other RNA /organism=synthetic construct
   Sequence 
agtacccttc accaaagcaa aaatgtgatc ttgcttgtaa atacaatttt gagaggttaa 60
taaattacaa gtagtgctat ttttgtattt aggttagcta tttagcttta cgttccagga 120
tgcctagtgg cagccccaca atatccagga agccctctct gcggtttttc agattaggta 180
gtcgaaaaac ctaagaaatt tacctgctac atttcaagat acc 223
SEQ ID NO: 36
   length 676
   molecule type RNA
   division PAT
   Key Location/Qualifiers
   source 1..676
      /mol_type=other RNA /organism=synthetic construct
   Sequence 
ttaaaacagc ctgtgggttg atcccaccca caggcccatt gggcgctagc actctggtat 60
cacggtacct ttgtgcgcct gttttatacc ccctccccct cccccccccc taacgttact 120
ggccgaagcc gcttggaata aggccggtgt gcgtttgtct atatgttatt ttccaccata 180
ttgccgtctt ttggcaatgt gagggcccgg aaacctggcc ctgtcttctt gacgagcatt 240
cctaggggtc tttcccctct cgccaaagga atgcaaggtc tgttgaatgt cgtgaaggaa 300
gcagttcctc tggaagcttc ttgaagacaa acaacgtctg tagcgaccct ttgcaggcag 360
cggaaccccc cacctggcga caggtgcctc tgcggccaaa agccacgtgt ataagataca 420
cctgcaaagg cggcacaacc ccagtgccac gttgtgagtt ggatagttgt ggaaagagtc 480
aaatggctct cctcaagcgt attcaacaag gggctgaagg atgcccagaa ggtaccccat 540
tgtatgggat ctgatctggg gcctcggtgc acatgcttta catgtgttta gtcgaggtta 600
aaaaaacgtc taggcccccc gaaccacggg gacgtggttt tcctttgaaa aacacgatga 660
taatatggcc acaacc 676
SEQ ID NO: 37
   length 317
   molecule type RNA
   division PAT
   Key Location/Qualifiers
   source 1..317
      /mol_type=other RNA /organism=synthetic construct
   Sequence 
ttaaaacagc ctgtgggttg atcccaccca caggcccatt gggcgctagc actctggtat 60
cacggtacct ttgtgcgcct gttttatacc ccctagtacc cttcaccaaa gcaaaaatgt 120
gatcttgctt gtaaatacaa ttttgagagg ttaataaatt acaagtagtg ctatttttgt 180
atttaggtta gctatttagc tttacgttcc aggatgccta gtggcagccc cacaatatcc 240
aggaagccct ctctgcggtt tttcagatta ggtagtcgaa aaacctaaga aatttacctg 300
ctacatttca agatacc 317
SEQ ID NO: 38
   length 1234
   molecule type RNA
   division PAT
   Key Location/Qualifiers
   source 1..1234
      /mol_type=other RNA /organism=synthetic construct
   Sequence 
ttaaaacagc ctgtgggttg atcccaccca caggcccatt gggcgctagc actctggtat 60
cacggtacct ttgtgcgcct gttttatacc ccctccccct cccccccccc taacgttact 120
ggccgaagcc gcttggaata aggccggtgt gcgtttgtct atatgttatt ttccaccata 180
ttgccgtctt ttggcaatgt gagggcccgg aaacctggcc ctgtcttctt gacgagcatt 240
cctaggggtc tttcccctct cgccaaagga atgcaaggtc tgttgaatgt cgtgaaggaa 300
gcagttcctc tggaagcttc ttgaagacaa acaacgtctg tagcgaccct ttgcaggcag 360
cggaaccccc cacctggcga caggtgcctc tgcggccaaa agccacgtgt ataagataca 420
cctgcaaagg cggcacaacc ccagtgccac gttgtgagtt ggatagttgt ggaaagagtc 480
aaatggctct cctcaagcgt attcaacaag gggctgaagg atgcccagaa ggtaccccat 540
tgtatgggat ctgatctggg gcctcggtgc acatgcttta catgtgttta gtcgaggtta 600
aaaaaacgtc taggcccccc gaaccacggg gacgtggttt tcctttgaaa aacacgatga 660
taatatggcc acaaccatgg gagtcaaagt tctgtttgcc ctgatctgca tcgctgtggc 720
cgaggccaag cccaccgaga acaacgaaga cttcaacatc gtggccgtgg ccagcaactt 780
cgcgaccacg gatctcgatg ctgaccgcgg gaagttgccc ggcaagaagc tgccgctgga 840
ggtgctcaaa gagatggaag ccaatgcccg gaaagctggc tgcaccaggg gctgtctgat 900
ctgcctgtcc cacatcaagt gcacgcccaa gatgaagaag ttcatcccag gacgctgcca 960
cacctacgaa ggcgacaaag agtccgcaca gggcggcata ggcgaggcga tcgtcgacat 1020
tcctgagatt cctgggttca aggacttgga gcccatggag cagttcatcg cacaggtcga 1080
tctgtgtgtg gactgcacaa ctggctgcct caaagggctt gccaacgtgc agtgttctga 1140
cctgctcaag aagtggctgc cgcaacgctg tgcgaccttt gccagcaaga tccagggcca 1200
ggtggacaag atcaaggggg ccggtggtga ctaa 1234
SEQ ID NO: 39
   length 875
   molecule type RNA
   division PAT
   Key Location/Qualifiers
   source 1..875
      /mol_type=other RNA /organism=synthetic construct
   Sequence 
ttaaaacagc ctgtgggttg atcccaccca caggcccatt gggcgctagc actctggtat 60
cacggtacct ttgtgcgcct gttttatacc ccctagtacc cttcaccaaa gcaaaaatgt 120
gatcttgctt gtaaatacaa ttttgagagg ttaataaatt acaagtagtg ctatttttgt 180
atttaggtta gctatttagc tttacgttcc aggatgccta gtggcagccc cacaatatcc 240
 
aggaagccct ctctgcggtt tttcagatta ggtagtcgaa aaacctaaga aatttacctg 300
ctacatttca agataccatg ggagtcaaag ttctgtttgc cctgatctgc atcgctgtgg 360
ccgaggccaa gcccaccgag aacaacgaag acttcaacat cgtggccgtg gccagcaact 420
tcgcgaccac ggatctcgat gctgaccgcg ggaagttgcc cggcaagaag ctgccgctgg 480
aggtgctcaa agagatggaa gccaatgccc ggaaagctgg ctgcaccagg ggctgtctga 540
tctgcctgtc ccacatcaag tgcacgccca agatgaagaa gttcatccca ggacgctgcc 600
acacctacga aggcgacaaa gagtccgcac agggcggcat aggcgaggcg atcgtcgaca 660
ttcctgagat tcctgggttc aaggacttgg agcccatgga gcagttcatc gcacaggtcg 720
atctgtgtgt ggactgcaca actggctgcc tcaaagggct tgccaacgtg cagtgttctg 780
acctgctcaa gaagtggctg ccgcaacgct gtgcgacctt tgccagcaag atccagggcc 840
aggtggacaa gatcaagggg gccggtggtg actaa 875
SEQ ID NO: 40
   length 1550
   molecule type RNA
   division PAT
   Key Location/Qualifiers
   source 1..1550
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac ccacaggccc 180
attgggcgct agcactctgg tatcacggta cctttgtgcg cctgttttat accccctctc 240
cctccccccc ccctaacgtt actggccgaa gccgcttgga ataaggccgg tgtgcgtttg 300
tctatatgtt attttccacc atattgccgt cttttggcaa tgtgagggcc cggaaacctg 360
gccctgtctt cttgacgagc attcctaggg gtctttcccc tctcgccaaa ggaatgcaag 420
gtctgttgaa tgtcgtgaag gaagcagttc ctctggaagc ttcttgaaga caaacaacgt 480
ctgtagcgac cctttgcagg cagcggaacc ccccacctgg cgacaggtgc ctctgcggcc 540
aaaagccacg tgtataagat acacctgcaa aggcggcaca accccagtgc cacgttgtga 600
gttggatagt tgtggaaaga gtcaaatggc tctcctcaag cgtattcaac aaggggctga 660
aggatgccca gaaggtaccc cattgtatgg gatctgatct ggggcctcgg tgcacatgct 720
ttacatgtgt ttagtcgagg ttaaaaaaac gtctaggccc cccgaaccac ggggacgtgg 780
ttttcctttg aaaaacacga tgataatatg gccacaacca tgggagtcaa agttctgttt 840
gccctgatct gcatcgctgt ggccgaggcc aagcccaccg agaacaacga agacttcaac 900
atcgtggccg tggccagcaa cttcgcgacc acggatctcg atgctgaccg cgggaagttg 960
cccggcaaga agctgccgct ggaggtgctc aaagagatgg aagccaatgc ccggaaagct 1020
ggctgcacca ggggctgtct gatctgcctg tcccacatca agtgcacgcc caagatgaag 1080
aagttcatcc caggacgctg ccacacctac gaaggcgaca aagagtccgc acagggcggc 1140
ataggcgagg cgatcgtcga cattcctgag attcctgggt tcaaggactt ggagcccatg 1200
gagcagttca tcgcacaggt cgatctgtgt gtggactgca caactggctg cctcaaaggg 1260
cttgccaacg tgcagtgttc tgacctgctc aagaagtggc tgccgcaacg ctgtgcgacc 1320
tttgccagca agatccaggg ccaggtggac aagatcaagg gggccggtgg tgactaatta 1380
aaacagcctg tgggttgata aataattgtc ccttaaagaa gaaattcttt aagtggatgc 1440
tctcaaactc agggaaacct aaatctagtt atagacaagg caatcctgag ccaagccgaa 1500
gtagtaatta gtaaggccta gtataccaaa ccgcgatatc tcttttctag 1550
SEQ ID NO: 41
   length 1191
   molecule type RNA
   division PAT
   Key Location/Qualifiers
   source 1..1191
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac ccacaggccc 180
attgggcgct agcactctgg tatcacggta cctttgtgcg cctgttttat accccctagt 240
acccttcacc aaagcaaaaa tgtgatcttg cttgtaaata caattttgag aggttaataa 300
attacaagta gtgctatttt tgtatttagg ttagctattt agctttacgt tccaggatgc 360
ctagtggcag ccccacaata tccaggaagc cctctctgcg gtttttcaga ttaggtagtc 420
gaaaaaccta agaaatttac ctgctacatt tcaagatacc atgggagtca aagttctgtt 480
tgccctgatc tgcatcgctg tggccgaggc caagcccacc gagaacaacg aagacttcaa 540
catcgtggcc gtggccagca acttcgcgac cacggatctc gatgctgacc gcgggaagtt 600
gcccggcaag aagctgccgc tggaggtgct caaagagatg gaagccaatg cccggaaagc 660
tggctgcacc aggggctgtc tgatctgcct gtcccacatc aagtgcacgc ccaagatgaa 720
gaagttcatc ccaggacgct gccacaccta cgaaggcgac aaagagtccg cacagggcgg 780
cataggcgag gcgatcgtcg acattcctga gattcctggg ttcaaggact tggagcccat 840
ggagcagttc atcgcacagg tcgatctgtg tgtggactgc acaactggct gcctcaaagg 900
gcttgccaac gtgcagtgtt ctgacctgct caagaagtgg ctgccgcaac gctgtgcgac 960
ctttgccagc aagatccagg gccaggtgga caagatcaag ggggccggtg gtgactaatt 1020
aaaacagcct gtgggttgat aaataattgt cccttaaaga agaaattctt taagtggatg 1080
ctctcaaact cagggaaacc taaatctagt tatagacaag gcaatcctga gccaagccga 1140
agtagtaatt agtaaggcct agtataccaa accgcgatat ctcttttcta g 1191
SEQ ID NO: 42
   length 1636
   molecule type RNA
   division PAT
   Key Location/Qualifiers
   source 1..1636
      /mol_type=other RNA /organism=synthetic construct
   Sequence 
gggagaccct cgaccgtcga ttgtccactg gtcaacaata gatgacttac aactaatcgg 60
aaggtgcaga gactcgacgg gagctaccct aacgtcaaga cgagggtaaa gagagagtcc 120
aattctcaaa gccaataggc agtagcgaaa gctgcaagag aatgaaaatc cgttgacctt 180
aaacggtcgt gtgggttcaa gtccctccac ccccacgccg gaaacgcaat agccgaaaaa 240
caaaaaacaa aaaaaacaaa aaaaaaacca aaaaaacaaa acacacccct ctccctcccc 300
cccccctaac gttactggcc gaagccgctt ggaataaggc cggtgtgcgt ttgtctatat 360
gttattttcc accatattgc cgtcttttgg caatgtgagg gcccggaaac ctggccctgt 420
cttcttgacg agcattccta ggggtctttc ccctctcgcc aaaggaatgc aaggtctgtt 480
gaatgtcgtg aaggaagcag ttcctctgga agcttcttga agacaaacaa cgtctgtagc 540
gaccctttgc aggcagcgga accccccacc tggcgacagg tgcctctgcg gccaaaagcc 600
acgtgtataa gatacacctg caaaggcggc acaaccccag tgccacgttg tgagttggat 660
agttgtggaa agagtcaaat ggctctcctc aagcgtattc aacaaggggc tgaaggatgc 720
ccagaaggta ccccattgta tgggatctga tctggggcct cggtacacat gctttacatg 780
tgtttagtcg aggttaaaaa aacgtctagg ccccccgaac cacggggacg tggttttcct 840
ttgaaaaaca cgatgataat atggccacaa ccatgggagt caaagttctg tttgccctga 900
tctgcatcgc tgtggccgag gccaagccca ccgagaacaa cgaagacttc aacatcgtgg 960
ccgtggccag caacttcgcg accacggatc tcgatgctga ccgcgggaag ttgcccggca 1020
agaagctgcc gctggaggtg ctcaaagaga tggaagccaa tgcccggaaa gctggctgca 1080
ccaggggctg tctgatctgc ctgtcccaca tcaagtgcac gcccaagatg aagaagttca 1140
tcccaggacg ctgccacacc tacgaaggcg acaaagagtc cgcacagggc ggcataggcg 1200
aggcgatcgt cgacattcct gagattcctg ggttcaagga cttggagccc atggagcagt 1260
tcatcgcaca ggtcgatctg tgtgtggact gcacaactgg ctgcctcaaa gggcttgcca 1320
acgtgcagtg ttctgacctg ctcaagaagt ggctgccgca acgctgtgcg acctttgcca 1380
gcaagatcca gggccaggtg gacaagatca agggggccgg tggtgactaa aaaaaacaaa 1440
aaacaaaacg gctattatgc gttaccggcg agacgctacg gacttaaata attgagcctt 1500
aaagaagaaa ttctttaagt ggatgctctc aaactcaggg aaacctaaat ctagttatag 1560
acaaggcaat cctgagccaa gccgaagtag taattagtaa gaccagtgga caatcgacgg 1620
 ataacagcat atctag 1636
SEQ ID NO: 43
   length 1272
   molecule type RNA
   division PAT
   Key Location/Qualifiers
   source 1..1272
      /mol_type=other RNA /organism=synthetic construct
   Sequence 
gggagaccct cgaccgtcga ttgtccactg gtcaacaata gatgacttac aactaatcgg 60
aaggtgcaga gactcgacgg gagctaccct aacgtcaaga cgagggtaaa gagagagtcc 120
aattctcaaa gccaataggc agtagcgaaa gctgcaagag aatgaaaatc cgttgacctt 180
aaacggtcgt gtgggttcaa gtccctccac ccccacgccg gaaacgcaat agccgaaaaa 240
caaaaaacaa aaaaaacaaa aaaaaaacca aaaaaacaaa acacaagtac ccttcaccaa 300
agcaaaaatg tgatcttgct tgtaaataca attttgagag gttaataaat tacaagtagt 360
gctatttttg tatttaggtt agctatttag ctttacgttc caggatgcct agtggcagcc 420
ccacaatatc caggaagccc tctctgcggt ttttcagatt aggtagtcga aaaacctaag 480
aaatttacct gctacatttc aagataccat gggagtcaaa gttctgtttg ccctgatctg 540
catcgctgtg gccgaggcca agcccaccga gaacaacgaa gacttcaaca tcgtggccgt 600
ggccagcaac ttcgcgacca cggatctcga tgctgaccgc gggaagttgc ccggcaagaa 660
gctgccgctg gaggtgctca aagagatgga agccaatgcc cggaaagctg gctgcaccag 720
gggctgtctg atctgcctgt cccacatcaa gtgcacgccc aagatgaaga agttcatccc 780
aggacgctgc cacacctacg aaggcgacaa agagtccgca cagggcggca taggcgaggc 840
gatcgtcgac attcctgaga ttcctgggtt caaggacttg gagcccatgg agcagttcat 900
cgcacaggtc gatctgtgtg tggactgcac aactggctgc ctcaaagggc ttgccaacgt 960
gcagtgttct gacctgctca agaagtggct gccgcaacgc tgtgcgacct ttgccagcaa 1020
gatccagggc caggtggaca agatcaaggg ggccggtggt gactaaaaaa aacaaaaaac 1080
aaaacggcta ttatgcgtta ccggcgagac gctacggact taaataattg agccttaaag 1140
aagaaattct ttaagtggat gctctcaaac tcagggaaac ctaaatctag ttatagacaa 1200
ggcaatcctg agccaagccg aagtagtaat tagtaagacc agtggacaat cgacggataa 1260
cagcatatct ag 1272
SEQ ID NO: 44
   length 22
   molecule type DNA
   division PAT
   Key Location/Qualifiers
   source 1..22
      /mol_type=other DNA /organism=synthetic construct
   Sequence
      gtctcctctg acttcaacag cg 22
SEQ ID NO: 45
   length 22
   molecule type DNA
   division PAT
   Key Location/Qualifiers
   source 1..22
      /mol_type=other DNA /organism=synthetic construct
   Sequence
      accaccctgt tgctgtagcc aa 22
SEQ ID NO: 46
   length 22
   molecule type DNA
   division PAT
   Key Location/Qualifiers
   source 1..22
      /mol_type=other DNA /organism=synthetic construct
Sequence
   agacagccac tcacctcttc ag 22
SEQ ID NO: 47
   length 22
   molecule type DNA
   division PAT
   Key Location/Qualifiers
   source 1..22
      /mol_type=other DNA /organism=synthetic construct
Sequence
   ttctgccagt gcctctttgc tg 22
SEQ ID NO: 48
   length 22
   molecule type DNA
   division PAT
   Key Location/Qualifiers
   source 1..22
      /mol_type=other DNA /organism=synthetic construct
Sequence
   cacctcagtt gctgatgaag gc 22
SEQ ID NO: 49
   length 23
   molecule type DNA
   division PAT
   Key Location/Qualifiers
   source 1..23
      /mol_type=other DNA /organism=synthetic construct
Sequence
   gtcagaagga agcacttgct acc 23
SEQ ID NO: 50
   length 22
   molecule type DNA
   division PAT
   Key Location/Qualifiers
   source 1..22
      /mol_type=other DNA /organism=synthetic construct
   Sequence
      cctgctgctt tgcctacatt gc 22
SEQ ID NO: 51
   length 22
   molecule type DNA
   division PAT
   Key Location/Qualifiers
   source 1..22
      /mol_type=other DNA /organism=synthetic construct
   Sequence
      acacacttgg cggttctttc gg 22
SEQ ID NO: 52
   length 24
   molecule type DNA
   division PAT
   Key Location/Qualifiers
   source 1..24
      /mol_type=other DNA /organism=synthetic construct
   Sequence
      cttggattcc tacaaagaag cagc 24
SEQ ID NO: 53
   length 22
   molecule type DNA
   division PAT
   Key Location/Qualifiers
   source 1..22
      /mol_type=other DNA /organism=synthetic construct
   Sequence
      tcctccttct ggaactgctg ca 22
SEQ ID NO: 54
   length 1790
   molecule type RNA
   division PAT
   Key Location/Qualifiers
   source 1..1790 /mol_type=other RNA /organism=synthetic construct
   Sequence 
gggagaccct cgaccgtcga ttgtccactg gtcaacaata gatgacttac aactaatcgg 60
aaggtgcaga gactcgacgg gagctaccct aacgtcaaga cgagggtaaa gagagagtcc 120
aattctcaaa gccaataggc agtagcgaaa gctgcaagag aatgaaaatc cgttgacctt 180
aaacggtcgt gtgggttcaa gtccctccac ccccacgccg gaaacgcaat agccgaaaaa 240
caaaaaacaa aaaaaacaaa aaaaaaacca aaaaaacaaa acacattaaa acagcctgtg 300
ggttgatccc acccacaggc ccattgggcg ctagcactct ggtatcacgg tacctttgtg 360
cgcctgtttt ataccccctc ccccaactgt aacttagaag taacacacac cgatcaacag 420
tcagcgtggc acaccagcca cgttttgatc aagcacttct gttaccccgg actgagtatc 480
 
aatagactgc tcacgcggtt gaaggagaaa gcgttcgtta tccggccaac tacttcgaaa 540
aacctagtaa caccgtggaa gttgcagagt gtttcgctca gcactacccc agtgtagatc 600
aggtcgatga gtcaccgcat tccccacggg cgaccgtggc ggtggctgcg ttggcggcct 660
gcccatgggg aaacccatgg gacgctctaa tacagacatg gtgcgaagag tctattgagc 720
tagttggtag tcctccggcc cctgaatgcg gctaatccta actgcggagc acacaccctc 780
aagccagagg gcagtgtgtc gtaacgggca actctgcagc ggaaccgact actttgggtg 840
tccgtgtttc attttattcc tatactggct gcttatggtg acaattgaga gatcgttacc 900
atatagctat tggattggcc atccggtgac taatagagct attatatatc cctttgttgg 960
gtttatacca cttagcttga aagaggttaa aacattacaa ttcattgtta agttgaatac 1020
agcaaaatgg gagtcaaagt tctgtttgcc ctgatctgca tcgctgtggc cgaggccaag 1080
cccaccgaga acaacgaaga cttcaacatc gtggccgtgg ccagcaactt cgcgaccacg 1140
gatctcgatg ctgaccgcgg gaagttgccc ggcaagaagc tgccgctgga ggtgctcaaa 1200
gagatggaag ccaatgcccg gaaagctggc tgcaccaggg gctgtctgat ctgcctgtcc 1260
cacatcaagt gcacgcccaa gatgaagaag ttcatcccag gacgctgcca cacctacgaa 1320
ggcgacaaag agtccgcaca gggcggcata ggcgaggcga tcgtcgacat tcctgagatt 1380
cctgggttca aggacttgga gcccatggag cagttcatcg cacaggtcga tctgtgtgtg 1440
gactgcacaa ctggctgcct caaaggcctt gccaacgtgc agtgttctga cctgctcaag 1500
aagtggctgc cgcaacgctg tgcgaccttt gccagcaaga tccaggccca ggtggacaag 1560
atcaaggggg ccggtggtga ctaaaaaaaa caaaaaacaa aacggctatt atgcgttacc 1620
ggcgagacgc tacggactta aataattgag ccttaaagaa gaaattcttt aagtggatgc 1680
tctcaaactc agggaaacct aaatctagtt atagacaagg caatcctgag ccaagccgaa 1740
gtagtaatta gtaagaccag tggacaatcg acggataaca gcatatctag 1790
SEQ ID NO: 55
   length 1617
   molecule type RNA
   division PAT
   Key Location/Qualifiers
   source 1..1617
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac ccacagggcc 180
cactgggcgt tagcacactg gtatcacggt acctttgtgc gcctgtttta tactcccccc 240
cctaaggaaa ctttagaagc aaagcaattg tgatcaatag tgggtatggc acaccagtca 300
tatcttgatc aagcacttct gttcccccgg acttagtacc aatagactgc tcaagcggtt 360
gaaggggaaa acgttcgtta tccggccaac tacttcgaga aacctagtag caccatgaaa 420
gttgcggagt gtttcgctca gcacttcccc cgtgtagatc aggctgatga gtcaccgtat 480
tccccacggg cgaccgtgac ggtggctgcg ttggcggcct gcccatgggg taacccatgg 540
gacgctctaa aacagacacg gtgcgaagag tctattgagc tagttggtag tcctccggcc 600
cctgaatgcg gccaatccta actgcggagc acatactccc aatccaggga gcagtgtgtc 660
gtaatgggta actctgcagc ggaaccgact actttgggtg tccgtgtttc cttttattct 720
cacattgact gcttatggtg acaattgaaa gattgttacc atatagctat tggattggtc 780
atccggtgag caatagagct attgtttatc aatttgttgg atttgtacca ctcaactttt 840
ctgttttgag aacactcaac tacatcttac tgctaaacac atcaaaatgg gagtcaaagt 900
tctgtttgcc ctgatctgca tcgctgtggc cgaggccaag cccaccgaga acaacgaaga 960
cttcaacatc gtggccgtgg ccagcaactt cgcgaccacg gatctcgatg ctgaccgcgg 1020
gaagttgccc ggcaagaagc tgccgctgga ggtgctcaaa gagatggaag ccaatgcccg 1080
gaaagctggc tgcaccaggg gctgtctgat ctgcctgtcc cacatcaagt gcacgcccaa 1140
gatgaagaag ttcatcccag gacgctgcca cacctacgaa ggcgacaaag agtccgcaca 1200
gggcggcata ggcgaggcga tcgtcgacat tcctgagatt cctgggttca aggacttgga 1260
gcccatggag cagttcatcg cacaggtcga tctgtgtgtg gactgcacaa ctggctgcct 1320
caaagggctt gccaacgtgc agtgttctga cctgctcaag aagtggctgc cgcaacgctg 1380
tgcgaccttt gccagcaaga tccaggccca ggtggacaag atcaaggggg ccggtggtga 1440
ctaattaaaa cagcctgtgg gttgttaaat aattgaccct taaagaagaa attctttaag 1500
tggatgctct caaactcagg gaaacctaaa tctagttata gacaaggcaa tcctgagcca 1560
 agccgaagta gtaattagta aggcctagta taccaaaccg cgatatctct tttctag 1617
SEQ ID NO: 56
   length 1618
   molecule type RNA
   division PAT
   Key Location/Qualifiers
   source 1..1618
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgaccca ccccagaggc 180
ccacgtggcg gctagtactc cggtattacg gtacccttgt acgcctgttt tatactccct 240
ccctcgtaac ttagaagcac aaaaccaagt tcaatagaag ggggtgcaaa ccagtaccac 300
cacgaacaag catttctgtt tccccggtga tgttgtatag actgcccgcg cggttgaaag 360
caacggatcc gttacccgct caagtacttc gagaagccta gtattacctt ggaatcttcg 420
atgcgttgcg ttcagcactc gaccctggag tgtagcttag gctgatgagt ctggacgtcc 480
ctcaccggtg acggtggtcc aggctgcgtt ggcggcctac ctatggctaa cgccatagga 540
cgctagttgt gaacaaggtg tgaagagcct attgagctac ttgagagtcc tccggcccct 600
gaatgcggct aatcctaacc atggagcagg cggtcacaga ccagtgacta gcttgtcgta 660
atgcgcaagt ctatggcgga accgactact ttgggtgtcc gtgtttcctt ttatttttat 720
tatggctgct tatggtgaca atcattgatt gttatcataa agcgaattgg attggccatc 780
cggtgaaagc gagacttact atttacttac ttgttggact taccacactt aatacattta 840
ttctaggtgt cacttgtata gcaattagaa tcaaacagtt gcatcataat gggagtcaaa 900
gttctgtttg ccctgatctg catcgctgtg gccgaggcca agcccaccga gaacaacgaa 960
gacttcaaca tcgtggccgt ggccagcaac ttcgcgacca cggatctcga tgctgaccgc 1020
gggaagttgc ccggcaagaa gctgccgctg gaggtgctca aagagatgga agccaatgcc 1080
cggaaagctg gctgcaccag gggctgtctg atctgcctgt cccacatcaa gtgcacgccc 1140
aagatgaaga agttcatccc aggacgctgc cacacctacg aaggcgacaa agagtccgca 1200
cagggcggca taggcgaggc gatcgtcgac attcctgaga ttcctgggtt caaggacttg 1260
gagcccatgg agcagttcat cgcacaggtc gatctgtgtg tggactgcac aactggctgc 1320
ctcaaagggc ttgccaacgt gcagtgttct gacctgctca agaagtggct gccgcaacgc 1380
tgtgcgacct ttgccagcaa gatccagggc caggtggaca agatcaaggg ggccggtggt 1440
gactaattaa aacagctctg gggttgtaaa taattgcacc ttaaagaaga aattctttaa 1500
gtggatgctc tcaaactcag ggaaacctaa atctagttat agacaaggca atcctgagcc 1560
aagccgaagt agtaattagt aaggcctagt ataccaaacc gcgatatctc ttttctag 1618
SEQ ID NO: 57
   length 1777
   molecule type RNA
   division PAT
   Key Location/Qualifiers
   source 1..1777 /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac ccacaggccc 180
attgggcgct agcactctgg tatcacggta cctttgtgcg cctgttttat accccctccc 240
ccaactgtaa cttagaagta acacacaccg atcaacagtc agcgtggcac accagccacg 300
ttttgatcaa gcacttctgt taccccggac tgagtatcaa tagactgctc acgcggttga 360
aggagaaagc gttcgttatc cggccaacta cttcgaaaaa cctagtaaca ccgtggaagt 420
tgcagagtgt ttcgctcagc actaccccag tgtagatcag gtcgatgagt caccgcattc 480
 
cccacgggcg accgtggcgg tggctgcgtt ggcggcctgc ccatggggaa acccatggga 540
cgctctaata cagacatggt gcgaagagtc tattgagcta gttggtagtc ctccggcccc 600
tgaatgcggc taatcctaac tgcggagcac acaccctcaa gccagagggc agtgtgtcgt 660
aacgggcaac tctgcagcgg aaccgactac tttgggtgtc cgtgtttcat tttattccta 720
tactggctgc ttatggtgac aattgagaga tcgttaccat atagctattg gattggccat 780
ccggtgacta atagagctat tatatatccc tttgttgggt ttataccact tagcttgaaa 840
gaggttaaaa cattacaatt cattgttaag ttgaatacag caaaatggtg agcaagggcg 900
aggagctgtt caccggggtg gtgcccatcc tggtcgagct ggacggcgac gtaaacggcc 960
acaagttcag cgtgtccggc gagggcgagg gcgatgccac ctacggcaag ctgaccctga 1020
agttcatctg caccaccggc aagctgcccg tgccctggcc caccctcgtg accaccctga 1080
cctacggcgt gcagtgcttc agccgctacc ccgaccacat gaagcagcac gacttcttca 1140
agtccgccat gcccgaaggc tacgtccagg agcgcaccat cttcttcaag gacgacggca 1200
actacaagac ccgcgccgag gtgaagttcg agggcgacac cctggtgaac cgcatcgagc 1260
tgaagggcat cgacttcaag gaggacggca acatcctggg gcacaagctg gagtacaact 1320
acaacagcca caacgtctat atcatggccg acaagcagaa gaacggcatc aaggtgaact 1380
tcaagatccg ccacaacatc gaggacggca gcgtgcagct cgccgaccac taccagcaga 1440
acacccccat cggcgacggc cccgtgctgc tgcccgacaa ccactacctg agcacccagt 1500
ccgccctgag caaagacccc aacgagaagc gcgatcacat ggtcctgctg gagttcgtga 1560
ccgccgccgg gatcactctc ggcatggacg agctgtacaa gtagttaaaa cagcctgtgg 1620
gttgataaat aattgtccct taaagaagaa attctttaag tggatgctct caaactcagg 1680
gaaacctaaa tctagttata gacaaggcaa tcctgagcca agccgaagta gtaattagta 1740
aggcctagta taccaaaccg cgatatctct tttctag 1777
SEQ ID NO: 58
   length 1639
   molecule type RNA
   division PAT
   Key Location/Qualifiers
   source 1..1639
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcccac ccacaggccc 180
attgggcgct agcactctgg tatcacggta cctttgtgcg cctgttttat accccctccc 240
ccaactgtaa cttagaagta acacacaccg atcaacagtc agcgtggcac accagccacg 300
ttttgatcaa gcacttctgt taccccggac tgagtatcaa tagactgctc acgcggttga 360
aggagaaagc gttcgttatc cggccaacta cttcgaaaaa cctagtaaca ccgtggaagt 420
tgcagagtgt ttcgctcagc actaccccag tgtagatcag gtcgatgagt caccgcattc 480
cccacgggcg accgtggcgg tggctgcgtt ggcggcctgc ccatggggaa acccatggga 540
cgctctaata cagacatggt gcgaagagtc tattgagcta gttggtagtc ctccggcccc 600
tgaatgcggc taatcctaac tgcggagcac acaccctcaa gccagagggc agtgtgtcgt 660
aacgggcaac tctgcagcgg aaccgactac tttgggtgtc cgtgtttcat tttattccta 720
tactggctgc ttatggtgac aattgagaga tcgttaccat atagctattg gattggccat 780
ccggtgacta atagagctat tatatatccc tttgttgggt ttataccact tagcttgaaa 840
gaggttaaaa cattacaatt cattgttaag ttgaatacag caaaatgggg gtgcacgaat 900
gtcctgcctg gctgtggctt ctcctgtccc tgctgtcgct ccctctgggc ctcccagtcc 960
tgggcgcccc accacgcctc atctgtgaca gccgagtcct ggagaggtac ctcttggagg 1020
ccaaggaggc cgagaatatc acgacgggct gtgctgaaca ctgcagcttg aatgagaata 1080
tcactgtccc agacaccaaa gttaatttct atgcctggaa gaggatggag gtcgggcagc 1140
aggccgtaga agtctggcag ggcctggccc tgctgtcgga agctgtcctg cggggccagg 1200
ccctgttggt caactcttcc cagccgtggg agcccctgca gctgcatgtg gataaagccg 1260
tcagtggcct tcgcagcctc accactctgc ttcgggctct gggagcccag aaggaagcca 1320
tctcccctcc agatgcggcc tcagctgctc cactccgaac aatcactgct gacactttcc 1380
gcaaactctt ccgagtctac tccaatttcc tccggggaaa gctgaagctg tacacagggg 1440
aggcctgcag gacaggggac agatgattaa aacagcctgt gggttgataa ataattgtcc 1500
cttaaagaag aaattcttta agtggatgct ctcaaactca gggaaaccta aatctagtta 1560
 
tagacaaggc aatcctgagc caagccgaag tagtaattag taaggcctag tataccaaac 1620
cgcgatatct cttttctag 1639
SEQ ID NO: 59
   length 745
   molecule type RNA
   division PAT
   Key Location/Qualifiers
   source 1..745
      /mol_type=other RNA /organism=synthetic construct
   Sequence 
gggacatttg cttctgacac aactgtgttc actagcaacc tcaaacagac accatgggag 60
tcaaagttct gtttgccctg atctgcatcg ctgtggccga ggccaagccc accgagaaca 120
acgaagactt caacatcgtg gccgtggcca gcaacttcgc gaccacggat ctcgatgctg 180
accgcgggaa gttgcccggc aagaagctgc cgctggaggt gctcaaagag atggaagcca 240
atgcccggaa agctggctgc accaggggct gtctgatctg cctgtcccac atcaagtgca 300
cgcccaagat gaagaagttc atcccaggac gctgccacac ctacgaaggc gacaaagagt 360
ccgcacaggg cggcataggc gaggcgatcg tcgacattcc tgagattcct gggttcaagg 420
acttggagcc catggagcag ttcatcgcac aggtcgatct gtgtgtggac tgcacaactg 480
gctgcctcaa agggcttgcc aacgtgcagt gttctgacct gctcaagaag tggctgccgc 540
aacgctgtgc gacctttgcc agcaagatcc agggccaggt ggacaagatc aagggggccg 600
gtggtgacta agctcgcttt cttgctgtcc aatttctatt aaaggttcct ttgttcccta 660
agtccaacta ctaaactggg ggatattatg aagggccttg agcatctgga ttctgcctaa 720
taaaaaacat ttattttcat tgcaa 745
SEQ ID NO: 60
   length 50
   molecule type RNA
   division PAT
   Key Location/Qualifiers
   source 1..50
      /mol_type=other RNA /organism=synthetic construct
   Sequence
      acatttgctt ctgacacaac tgtgttcact agcaacctca aacagacacc 50
SEQ ID NO: 61
   length 134
   molecule type RNA
   division PAT
   Key Location/Qualifiers
   source 1..134
      /mol_type=other RNA /organism=synthetic construct
   Sequence 
gctcgctttc ttgctgtcca atttctatta aaggttcctt tgttccctaa gtccaactac 60
taaactgggg gatattatga agggccttga gcatctggat tctgcctaat aaaaaacatt 120
tattttcatt gcaa 134
SEQ ID NO: 62
   length 2394
   molecule type RNA
   division PAT
   Key Location/Qualifiers
   source 1..2394
      /mol_type=other RNA /organism=synthetic construct
   Sequence 
ttaaaacagc ctgtgggttg atcccaccca caggcccatt gggcgctagc actctggtat 60
cacggtacct ttgtgcgcct gttttatacc ccctccccca actgtaactt agaagtaaca 120
cacaccgatc aacagtcagc gtggcacacc agccacgttt tgatcaagca cttctgttac 180
cccggactga gtatcaatag actgctcacg cggttgaagg agaaagcgtt cgttatccgg 240
ccaactactt cgaaaaacct agtaacaccg tggaagttgc agagtgtttc gctcagcact 300
accccagtgt agatcaggtc gatgagtcac cgcattcccc acgggcgacc gtggcggtgg 360
ctgcgttggc ggcctgccca tggggaaacc catgggacgc tctaatacag acatggtgcg 420
aagagtctat tgagctagtt ggtagtcctc cggcccctga atgcggctaa tcctaactgc 480
ggagcacaca ccctcaagcc agagggcagt gtgtcgtaac gggcaactct gcagcggaac 540
cgactacttt gggtgtccgt gtttcatttt attcctatac tggctgctta tggtgacaat 600
tgagagatcg ttaccatata gctattggat tggccatccg gtgactaata gagctattat 660
atatcccttt gttgggttta taccacttag cttgaaagag gttaaaacat tacaattcat 720
tgttaagttg aatacagcaa aatggaggac gccaagaaca tcaagaaggg ccccgccccc 780
ttctaccccc tggaggacgg caccgccggc gagcagctgc acaaggccat gaagcggtac 840
gccctggtgc ccggcaccat cgccttcacc gacgcccaca tcgaggtgga catcacctac 900
gccgagtact tcgagatgag cgtgcggctg gccgaggcca tgaagcggta cggcctgaac 960
accaaccacc ggatcgtggt gtgcagcgag aacagcctgc agttcttcat gcccgtgctg 1020
ggcgccctgt tcatcggcgt ggccgtggcc cccgccaacg acatctacaa cgagcgggag 1080
ctgctgaaca gcatgggcat cagccagccc accgtggtgt tcgtgagcaa gaagggcctg 1140
cagaagatcc tgaacgtgca gaagaagctg cccatcatcc agaagatcat catcatggac 1200
agcaagaccg actaccaggg cttccagagc atgtacacct tcgtgaccag ccacctgccc 1260
cccggcttca acgagtacga cttcgtgccc gagagcttcg accgggacaa gaccatcgcc 1320
ctgatcatga acagcagcgg cagcaccggc ctgcccaagg gcgtggccct gccccaccgg 1380
accgcctgcg tgcggttcag ccacgcccgg gaccccatct tcggcaacca gatcatcccc 1440
gacaccgcca tcctgagcgt ggtgcccttc caccacggct tcggcatgtt caccaccctg 1500
ggctacctga tctgcggctt ccgggtggtg ctgatgtacc ggttcgagga ggagctgttc 1560
ctgcggagcc tgcaggacta caagatccag agcgccctgc tggtgcccac cctgttcagc 1620
ttcttcgcca agagcaccct gatcgacaag tacgacctga gcaacctgca cgagatcgcc 1680
agcggcggcg cccccctgag caaggaggtg ggcgaggccg tggccaagcg gttccacctg 1740
cccggcatcc ggcagggcta cggcctgacc gagaccacca gcgccatcct gatcaccccc 1800
gagggcgacg acaagcccgg cgccgtgggc aaggtggtgc ccttcttcga ggccaaggtg 1860
gtggacctgg acaccggcaa gaccctgggc gtgaaccagc ggggcgagct gtgcgtgcgg 1920
ggccccatga tcatgagcgg ctacgtgaac aaccccgagg ccaccaacgc cctgatcgac 1980
aaggacggct ggctgcacag cggcgacatc gcctactggg acgaggacga gcacttcttc 2040
atcgtggacc ggctgaagag cctgatcaag tacaagggct accaggtggc ccccgccgag 2100
ctggagagca tcctgctgca gcaccccaac atcttcgacg ccggcgtggc cggcctgccc 2160
gacgacgacg ccggcgagct gcccgccgcc gtggtggtgc tggagcacgg caagaccatg 2220
accgagaagg agatcgtgga ctacgtggcc agccaggtga ccaccgccaa gaagctgcgg 2280
ggcggcgtgg tgttcgtgga cgaggtgccc aagggcctga ccggcaagct ggacgcccgg 2340
aagatccggg agatcctgat caaggccaag aagggcggca agatcgccgt gtga 2394
SEQ ID NO: 63
   length 2710
   molecule type RNA
   division PAT
   Key Location/Qualifiers
   source 1..2710
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
 
caattctcaa agccaatagg cagtagcgaa agctgcaaga gaatgcccac ccacaggccc 180
attgggcgct agcactctgg tatcacggta cctttgtgcg cctgttttat acccctcccc 240
ccaactgtaa cttagaagta acacacaccg atcaacagtc agcgtggcac accagccacg 300
ttttgatcaa gcacttctgt taccccggac tgagtatcaa tagactgctc acgcggttga 360
aggagaaagc gttcgttatc cggccaacta cttcgaaaaa cctagtaaca ccgtggaagt 420
tgcagagtgt ttcgctcagc actaccccag tgtagatcag gtcgatgagt caccgcattc 480
cccacgggcg accgtggcgg tggctgcgtt ggcggcctgc ccatggggaa acccatggga 540
cgctctaata cagacatggt gcgaagagtc tattgagcta gttggtagtc ctccggcccc 600
tgaatgcggc taatcctaac tgcggagcac acaccctcaa gccagagggc agtgtgtcgt 660
aacgggcaac tctgcagcgg aaccgactac tttgggtgtc cgtgtttcat tttattccta 720
tactggctgc ttatggtgac aattgagaga tcgttaccat atagctattg gattggccat 780
ccggtgacta atagagctat tatatatccc tttgttgggt ttataccact tagcttgaaa 840
gaggttaaaa cattacaatt cattgttaag ttgaatacag caaaatggag gacgccaaga 900
acatcaagaa gggccccgcc cccttctacc ccctggagga cggcaccgcc ggcgagcagc 960
tgcacaaggc catgaagcgg tacgccctgg tgcccggcac catcgccttc accgacgccc 1020
acatcgaggt ggacatcacc tacgccgagt acttcgagat gagcgtgcgg ctggccgagg 1080
ccatgaagcg gtacggcctg aacaccaacc accggatcgt ggtgtgcagc gagaacagcc 1140
tgcagttctt catgcccgtg ctgggcgccc tgttcatcgg cgtggccgtg gcccccgcca 1200
acgacatcta caacgagcgg gagctgctga acagcatggg catcagccag cccaccgtgg 1260
tgttcgtgag caagaagggc ctgcagaaga tcctgaacgt gcagaagaag ctgcccatca 1320
tccagaagat catcatcatg gacagcaaga ccgactacca gggcttccag agcatgtaca 1380
ccttcgtgac cagccacctg ccccccggct tcaacgagta cgacttcgtg cccgagagct 1440
tcgaccggga caagaccatc gccctgatca tgaacagcag cggcagcacc ggcctgccca 1500
agggcgtggc cctgccccac cggaccgcct gcgtgcggtt cagccacgcc cgggacccca 1560
tcttcggcaa ccagatcatc cccgacaccg ccatcctgag cgtggtgccc ttccaccacg 1620
gcttcggcat gttcaccacc ctgggctacc tgatctgcgg cttccgggtg gtgctgatgt 1680
accggttcga ggaggagctg ttcctgcgga gcctgcagga ctacaagatc cagagcgccc 1740
tgctggtgcc caccctgttc agcttcttcg ccaagagcac cctgatcgac aagtacgacc 1800
tgagcaacct gcacgagatc gccagcggcg gcgcccccct gagcaaggag gtgggcgagg 1860
ccgtggccaa gcggttccac ctgcccggca tccggcaggg ctacggcctg accgagacca 1920
ccagcgccat cctgatcacc cccgagggcg acgacaagcc cggcgccgtg ggcaaggtgg 1980
tgcccttctt cgaggccaag gtggtggacc tggacaccgg caagaccctg ggcgtgaacc 2040
agcggggcga gctgtgcgtg cggggcccca tgatcatgag cggctacgtg aacaaccccg 2100
aggccaccaa cgccctgatc gacaaggacg gctggctgca cagcggcgac atcgcctact 2160
gggacgagga cgagcacttc ttcatcgtgg accggctgaa gagcctgatc aagtacaagg 2220
gctaccaggt ggcccccgcc gagctggaga gcatcctgct gcagcacccc aacatcttcg 2280
acgccggcgt ggccggcctg cccgacgacg acgccggcga gctgcccgcc gccgtggtgg 2340
tgctggagca cggcaagacc atgaccgaga aggagatcgt ggactacgtg gccagccagg 2400
tgaccaccgc caagaagctg cggggcggcg tggtgttcgt ggacgaggtg cccaagggcc 2460
tgaccggcaa gctggacgcc cggaagatcc gggagatcct gatcaaggcc aagaagggcg 2520
gcaagatcgc cgtgtgatta aaacagcctg tgggttgata aataattgtc ccttaaagaa 2580
gaaattcttt aagtggatgc tctcaaactc agggaaacct aaatctagtt atagacaagg 2640
caatcctgag ccaagccgaa gtagtaatta gtaaggccta gtataccaaa ccgcgatatc 2700
tcttttctag 2710
SEQ ID NO: 64
   length 2570
   molecule type RNA
   division PAT
   Key Location/Qualifiers
   source 1..2570
      /mol_type=other RNA /organism=synthetic construct
   Sequence 
aaaatccgtt gaccttaaac ggtcgtgtgg gttcaagtcc ctccaccccc acgccggaaa 60
cgcaatagcc gaaaaacaaa aaacaaaaaa aacaaaaaaa aaaccaaaaa aacaaaacac 120
attaaaacag cctgtgggtt gatcccaccc acaggcccat tgggcgctag cactctggta 180
tcacggtacc tttgtgcgcc tgttttatac cccctccccc aactgtaact tagaagtaac 240
 
acacaccgat caacagtcag cgtggcacac cagccacgtt ttgatcaagc acttctgtta 300
ccccggactg agtatcaata gactgctcac gcggttgaag gagaaagcgt tcgttatccg 360
gccaactact tcgaaaaacc tagtaacacc gtggaagttg cagagtgttt cgctcagcac 420
taccccagtg tagatcaggt cgatgagtca ccgcattccc cacggcgacc cgtggcggtg 480
gctgcgttgg cggcctgccc atggggaaac ccatgggacg ctctaataca gacatggtgc 540
gaagagtcta ttgagctagt tggtagtcct ccggcccctg aatgcggcta atcctaactg 600
cggagcacac accctcaagc cagagggcag tgtgtcgtaa cgggcaactc tgcagcggaa 660
ccgactactt tgggtgtccg tgtttcattt tattcctata ctggctgctt atggtgacaa 720
ttgagagatc gttaccatat agctattgga ttggccatcc ggtgactaat agagctatta 780
tatatccctt tgttgggttt ataccactta gcttgaaaga ggttaaaaca ttacaattca 840
ttgttaagtt gaatacagca aaatggagga cgccaagaac atcaagaagg gccccgcccc 900
cttctacccc ctggaggacg gcaccgccgg cgagcagctg cacaaggcca tgaagcggta 960
cgccctggtg cccggcacca tcgccttcac cgacgcccac atcgaggtgg acatcaccta 1020
cgccgagtac ttcgagatga gcgtgcggct ggccgaggcc atgaagcggt acggcctgaa 1080
caccaaccac cggatcgtgg tgtgcagcga gaacagcctg cagttcttca tgcccgtgct 1140
gggcgccctg ttcatcggcg tggccgtggc ccccgccaac gacatctaca acgagcggga 1200
gctgctgaac agcatgggca tcagccagcc caccgtggtg ttcgtgagca agaagggcct 1260
gcagaagatc ctgaacgtgc agaagaagct gcccatcatc cagaagatca tcatcatgga 1320
cagcaagacc gactaccagg gcttccagag catgtacacc ttcgtgacca gccacctgcc 1380
ccccggcttc aacgagtacg acttcgtgcc cgagagcttc gaccgggaca agaccatcgc 1440
cctgatcatg aacagcagcg gcagcaccgg cctgcccaag ggcgtggccc tgccccaccg 1500
gaccgcctgc gtgcggttca gccacgcccg ggaccccatc ttcggcaacc agatcatccc 1560
cgacaccgcc atcctgagcg tggtgccctt ccaccacggc ttcggcatgt tcaccaccct 1620
gggctacctg atctgcggct tccgggtggt gctgatgtac cggttcgagg aggagctgtt 1680
cctgcggagc ctgcaggact acaagatcca gagcgccctg ctggtgccca ccctgttcag 1740
cttcttcgcc aagagcaccc tgatcgacaa gtacgacctg agcaacctgc acgagatcgc 1800
cagcggcggc gcccccctga gcaaggaggt gggcgaggcc gtggccaagc ggttccacct 1860
gcccggcatc cggcagggct acggcctgac cgagaccacc agcgccatcc tgatcacccc 1920
cgagggcgac gacaagcccg gcgccgtggg caaggtggtg cccttcttcg aggccaaggt 1980
ggtggacctg gacaccggca agaccctggg cgtgaaccag cggggcgagc tgtgcgtgcg 2040
gggccccatg atcatgagcg gctacgtgaa caaccccgag gccaccaacg ccctgatcga 2100
caaggacggc tggctgcaca gcggcgacat cgcctactgg gacgaggacg agcacttctt 2160
catcgtggac cggctgaaga gcctgatcaa gtacaagggc taccaggtgg cccccgccga 2220
gctggagagc atcctgctgc agcaccccaa catcttcgac gccggcgtgg ccggcctgcc 2280
cgacgacgac gccggcgagc tgcccgccgc cgtggtggtg ctggagcacg gcaagaccat 2340
gaccgagaag gagatcgtgg actacgtggc cagccaggtg accaccgcca agaagctgcg 2400
gggcggcgtg gtgttcgtgg acgaggtgcc caagggcctg accggcaagc tggacgcccg 2460
gaagatccgg gagatcctga tcaaggccaa gaagggcggc aagatcgccg tgtgaaaaaa 2520
acaaaaaaca aaacggctat tatgcgttac cggcgagacg ctacggactt 2570
SEQ ID NO: 65
   length 2885
   molecule type RNA
   division PAT
   Key Location/Qualifiers
   source 1..2885
      /mol_type=other RNA /organism=synthetic construct
   Sequence 
gggagaccct cgaccgtcga ttgtccactg gtcaacaata gatgacttac aactaatcgg 60
aaggtgcaga gactcgacgg gagctaccct aacgtcaaga cgagggtaaa gagagagtcc 120
aattctcaaa gccaataggc agtagcgaaa gctgcaagag aatgaaaatc cgttgacctt 180
aaacggtcgt gtgggttcaa gtccctccac ccccacgccg gaaacgcaat agccgaaaaa 240
caaaaaacaa aaaaaacaaa aaaaaaacca aaaaaacaaa acacattaaa acagcctgtg 300
ggttgatccc acccacaggc ccattgggcg ctagcactct ggtatcacgg tacctttgtg 360
cgcctgtttt ataccccctc ccccaactgt aacttagaag taacacacac cgatcaacag 420
tcagcgtggc acaccagcca cgttttgatc aagcacttct gttaccccgg actgagtatc 480
aatagactgc tcacgcggtt gaaggagaaa gcgttcgtta tccggccaac tacttcgaaa 540
 
aacctagtaa caccgtggaa gttgcagagt gtttcgctca gcactacccc agtgtagatc 600
aggtcgatga gtcaccgcat tccccacggg cgaccgtggc ggtggctgcg ttggcggcct 660
gcccatgggg aaacccatgg gacgctctaa tacagacatg gtgcgaagag tctattgagc 720
tagttggtag tcctccggcc cctgaatgcg gctaatccta actgcggagc acacaccctc 780
aagccagagg gcagtgtgtc gtaacgggca actctgcagc ggaaccgact actttgggtg 840
tccgtgtttc attttattcc tatactggct gcttatggtg acaattgaga gatcgttacc 900
atatagctat tggattggcc atccggtgac taatagagct attatatatc cctttgttgg 960
gtttatacca cttagcttga aagaggttaa aacattacaa ttcattgtta agttgaatac 1020
agcaaaatgg aggacgccaa gaacatcaag aagggccccg cccccttcta ccccctggag 1080
gacggcaccg ccggcgagca gctgcacaag gccatgaagc ggtacgccct ggtgcccggc 1140
accatcgcct tcaccgacgc ccacatcgag gtggacatca cctacgccga gtacttcgag 1200
atgagcgtgc ggctggccga ggccatgaag cggtacggcc tgaacaccaa ccaccggatc 1260
gtggtgtgca gcgagaacag cctgcagttc ttcatgcccg tgctgggcgc cctgttcatc 1320
ggcgtggccg tggcccccgc caacgacatc tacaacgagc gggagctgct gaacagcatg 1380
ggcatcagcc agcccaccgt ggtgttcgtg agcaagaagg gcctgcagaa gatcctgaac 1440
gtgcagaaga agctgcccat catccagaag atcatcatca tggacagcaa gaccgactac 1500
cagggcttcc agagcatgta caccttcgtg accagccacc tgccccccgg cttcaacgag 1560
tacgacttcg tgcccgagag cttcgaccgg gacaagacca tcgccctgat catgaacagc 1620
agcggcagca ccggcctgcc caagggcgtg gccctgcccc accggaccgc ctgcgtgcgg 1680
ttcagccacg cccgggaccc catcttcggc aaccagatca tccccgacac cgccatcctg 1740
agcgtggtgc ccttccacca cggcttcggc atgttcacca ccctgggcta cctgatctgc 1800
ggcttccggg tggtgctgat gtaccggttc gaggaggagc tgttcctgcg gagcctgcag 1860
gactacaaga tccagagcgc cctgctggtg cccaccctgt tcagcttctt cgccaagagc 1920
accctgatcg acaagtacga cctgagcaac ctgcacgaga tcgccagcgg cggcgccccc 1980
ctgagcaagg aggtgggcga ggccgtggcc aagcggttcc acctgcccgg catccggcag 2040
ggctacggcc tgaccgagac caccagcgcc atcctgatca cccccgaggg cgacgacaag 2100
cccggcgccg tgggcaaggt ggtgcccttc ttcgaggcca aggtggtgga cctggacacc 2160
ggcaagaccc tgggcgtgaa ccagcggggc gagctgtgcg tgcggggccc catgatcatg 2220
agcggctacg tgaacaaccc cgaggccacc aacgccctga tcgacaagga cggctggctg 2280
cacagcggcg acatcgccta ctgggacgag gacgagcact tcttcatcgt ggaccggctg 2340
aagagcctga tcaagtacaa gggctaccag gtggcccccg ccgagctgga gagcatcctg 2400
ctgcagcacc ccaacatctt cgacgccggc gtggccggcc tgcccgacga cgacgccggc 2460
gagctgcccg ccgccgtggt ggtgctggag cacggcaaga ccatgaccga gaaggagatc 2520
gtggactacg tggccagcca ggtgaccacc gccaagaagc tgcggggcgg cgtggtgttc 2580
gtggacgagg tgcccaaggg cctgaccggc aagctggacg cccggaagat ccgggagatc 2640
ctgatcaagg ccaagaaggg cggcaagatc gccgtgtgaa aaaaacaaaa aacaaaacgg 2700
ctattatgcg ttaccggcga gacgctacgg acttaaataa ttgagcctta aagaagaaat 2760
tctttaagtg gatgctctca aactcaggga aacctaaatc tagttataga caaggcaatc 2820
ctgagccaag ccgaagtagt aattagtaag accagtggac aatcgacgga taacagcata 2880
tctag 2885
SEQ ID NO: 66
   length 1323
   molecule type RNA
   division PAT
   Key Location/Qualifiers
   source 1..1323
      /mol_type=other RNA /organism=synthetic construct
   Sequence 
ttaaaacagc ctgtgggttg atcccaccca caggcccatt gggcgctagc actctggtat 60
cacggtacct ttgtgcgcct gttttatacc ccctccccca actgtaactt agaagtaaca 120
cacaccgatc aacagtcagc gtggcacacc agccacgttt tgatcaagca cttctgttac 180
cccggactga gtatcaatag actgctcacg cggttgaagg agaaagcgtt cgttatccgg 240
ccaactactt cgaaaaacct agtaacaccg tggaagttgc agagtgtttc gctcagcact 300
accccagtgt agatcaggtc gatgagtcac cgcattcccc acgggcgacc gtggcggtgg 360
ctgcgttggc ggcctgccca tggggaaacc catgggacgc tctaatacag acatggtgcg 420
aagagtctat tgagctagtt ggtagtcctc cggcccctga atgcggctaa tcctaactgc 480
 
ggagcacaca ccctcaagcc agagggcagt gtgtcgtaac gggcaactct gcagcggaac 540
cgactacttt gggtgtccgt gtttcatttt attcctatac tggctgctta tggtgacaat 600
tgagagatcg ttaccatata gctattggat tggccatccg gtgactaata gagctattat 660
atatcccttt gttgggttta taccacttag cttgaaagag gttaaaacat tacaattcat 720
tgttaagttg aatacagcaa aatgggggtg cacgaatgtc ctgcctggct gtggcttctc 780
ctgtccctgc tgtcgctccc tctgggcctc ccagtcctgg gcgccccacc acgcctcatc 840
tgtgacagcc gagtcctgga gaggtacctc ttggaggcca aggaggccga gaatatcacg 900
acgggctgtg ctgaacactg cagcttgaat gagaatatca ctgtcccaga caccaaagtt 960
aatttctatg cctggaagag gatggaggtc gggcagcagg ccgtagaagt ctggcagggc 1020
ctggccctgc tgtcggaagc tgtcctgcgg ggccaggccc tgttggtcaa ctcttcccag 1080
ccgtgggagc ccctgcagct gcatgtggat aaagccgtca gtggccttcg cagcctcacc 1140
actctgcttc gggctctggg agcccagaag gaagccatct cccctccaga tgcggcctca 1200
gctgctccac tccgaacaat cactgctgac actttccgca aactcttccg agtctactcc 1260
aatttcctcc ggggaaagct gaagctgtac acaggggagg cctgcaggac aggggacaga 1320
tga 1323
SEQ ID NO: 67
   length 1814
   molecule type RNA
   division PAT
   Key Location/Qualifiers
   source 1..1814
      /mol_type=other RNA /organism=synthetic construct
   Sequence 
gggagaccct cgaccgtcga ttgtccactg gtcaacaata gatgacttac aactaatcgg 60
aaggtgcaga gactcgacgg gagctaccct aacgtcaaga cgagggtaaa gagagagtcc 120
aattctcaaa gccaataggc agtagcgaaa gctgcaagag aatgaaaatc cgttgacctt 180
aaacggtcgt gtgggttcaa gtccctccac ccccacgccg gaaacgcaat agccgaaaaa 240
caaaaaacaa aaaaaacaaa aaaaaaacca aaaaaacaaa acacattaaa acagcctgtg 300
ggttgatccc acccacaggc ccattgggcg ctagcactct ggtatcacgg tacctttgtg 360
cgcctgtttt ataccccctc ccccaactgt aacttagaag taacacacac cgatcaacag 420
tcagcgtggc acaccagcca cgttttgatc aagcacttct gttaccccgg actgagtatc 480
aatagactgc tcacgcggtt gaaggagaaa gcgttcgtta tccggccaac tacttcgaaa 540
aacctagtaa caccgtggaa gttgcagagt gtttcgctca gcactacccc agtgtagatc 600
aggtcgatga gtcaccgcat tccccacggg cgaccgtggc ggtggctgcg ttggcggcct 660
gcccatgggg aaacccatgg gacgctctaa tacagacatg gtgcgaagag tctattgagc 720
tagttggtag tcctccggcc cctgaatgcg gctaatccta actgcggagc acacaccctc 780
aagccagagg gcagtgtgtc gtaacgggca actctgcagc ggaaccgact actttgggtg 840
tccgtgtttc attttattcc tatactggct gcttatggtg acaattgaga gatcgttacc 900
atatagctat tggattggcc atccggtgac taatagagct attatatatc cctttgttgg 960
gtttatacca cttagcttga aagaggttaa aacattacaa ttcattgtta agttgaatac 1020
agcaaaatgg gggtgcacga atgtcctgcc tggctgtggc ttctcctgtc cctgctgtcg 1080
ctccctctgg gcctcccagt cctgggcgcc ccaccacgcc tcatctgtga cagccgagtc 1140
ctggagaggt acctcttgga ggccaaggag gccgagaata tcacgacggg ctgtgctgaa 1200
cactgcagct tgaatgagaa tatcactgtc ccagacacca aagttaattt ctatgcctgg 1260
aagaggatgg aggtcgggca gcaggccgta gaagtctggc agggcctggc cctgctgtcg 1320
gaagctgtcc tgcggggcca ggccctgttg gtcaactctt cccagccgtg ggagcccctg 1380
cagctgcatg tggataaagc cgtcagtggc cttcgcagcc tcaccactct gcttcgggct 1440
ctgggagccc agaaggaagc catctcccct ccagatgcgg cctcagctgc tccactccga 1500
acaatcactg ctgacacttt ccgcaaactc ttccgagtct actccaattt cctccgggga 1560
aagctgaagc tgtacacagg ggaggcctgc aggacagggg acagatgaaa aaaacaaaaa 1620
acaaaacggc tattatgcgt taccggcgag acgctacgga cttaaataat tgagccttaa 1680
agaagaaatt ctttaagtgg atgctctcaa actcaggaaa acctaaatct agttatagac 1740
aaggcaatcc tgagccaagc cgaagtagta attagtaaga ccagtggaca atcgacggat 1800
aacagcatat ctag 1814
SEQ ID NO: 68
   length 741
   molecule type RNA
   division PAT
   Key Location/Qualifiers
   source 1..741
      /mol_type=other RNA /organism=synthetic construct
   Sequence 
ttaaaacagc ctgtgggttg atcccaccca caggcccatt gggcgctagc actctggtat 60
cacggtacct ttgtgcgcct gttttatacc ccctccccca actgtaactt agaagtaaca 120
cacaccgatc aacagtcagc gtggcacacc agccacgttt tgatcaagca cttctgttac 180
cccggactga gtatcaatag actgctcacg cggttgaagg agaaagcgtt cgttatccgg 240
ccaactactt cgaaaaacct agtaacaccg tggaagttgc agagtgtttc gctcagcact 300
accccagtgt agatcaggtc gatgagtcac cgcattcccc acgggcgacc gtggcggtgg 360
ctgcgttggc ggcctgccca tggggaaacc catgggacgc tctaatacag acatggtgcg 420
aagagtctat tgagctagtt ggtagtcctc cggcccctga atgcggctaa tcctaactgc 480
ggagcacaca ccctcaagcc agagggcagt gtgtcgtaac gggcaactct gcagcggaac 540
cgactacttt gggtgtccgt gtttcatttt attcctatac tggctgctta tggtgacaat 600
tgagagatcg ttaccatata gctattggat tggccatccg gtgactaata gagctattat 660
atatcccttt gttgggttta taccacttag cttgaaagag gttaaaacat tacaattcat 720
tgttaagttg aatacagcaa a 741
SEQ ID NO: 69
   length 50
   molecule type RNA
   division PAT
   Key Location/Qualifiers
   source 1..50 /mol_type=other RNA /organism=synthetic construct
Sequence
   cggactgagt atcaatagac tgctcacgcg gttgaaggag aaagcgttcg 50
SEQ ID NO: 70
   length 558
   molecule type RNA
   division PAT
   Key Location/Qualifiers
   source 1..558 /mol_type=other RNA /organism=synthetic construct
   Sequence 
atgggagtca aagttctgtt tgccctgatc tgcatcgctg tggccgaggc caagcccacc 60
gagaacaacg aagacttcaa catcgtggcc gtggccagca acttcgcgac cacggatctc 120
gatgctgacc gcgggaagtt gcccggcaag aagctgccgc tggaggtgct caaagagatg 180
gaagccaatg cccggaaagc tggctgcacc aggggctgtc tgatctgcct gtcccacatc 240
aagtgcacgc ccaagatgaa gaagttcatc ccaggacgct gccacaccta cgaaggcgac 300
aaagagtccg cacagggcgg cataggcgag gcgatcgtcg acattcctga gattcctggg 360
ttcaaggact tggagcccat ggagcagttc atcgcacagg tcgatctgtg tgtggactgc 420
acaactggct gcctcaaagg gcttgccaac gtgcagtgtt ctgacctgct caagaagtgg 480
ctgccgcaac gctgtgcgac ctttgccagc aagatccagg gccaggtgga caagatcaag 540
ggggccggtg gtgactaa 558
SEQ ID NO: 71
   length 1299
   molecule type RNA
   division PAT
   Key Location/Qualifiers
   source 1..1299 /mol_type=other RNA /organism=synthetic construct
   Sequence 
ttaaaacagc ctgtgggttg atcccaccca caggcccatt gggcgctagc actctggtat 60
cacggtacct ttgtgcgcct gttttatacc ccctccccca actgtaactt agaagtaaca 120
cacaccgatc aacagtcagc gtggcacacc agccacgttt tgatcaagca cttctgttac 180
cccggactga gtatcaatag actgctcacg cggttgaagg agaaagcgtt cgttatccgg 240
ccaactactt cgaaaaacct agtaacaccg tggaagttgc agagtgtttc gctcagcact 300
accccagtgt agatcaggtc gatgagtcac cgcattcccc acgggcgacc gtggcggtgg 360
ctgcgttggc ggcctgccca tggggaaacc catgggacgc tctaatacag acatggtgcg 420
aagagtctat tgagctagtt ggtagtcctc cggcccctga atgcggctaa tcctaactgc 480
ggagcacaca ccctcaagcc agagggcagt gtgtcgtaac gggcaactct gcagcggaac 540
cgactacttt gggtgtccgt gtttcatttt attcctatac tggctgctta tggtgacaat 600
tgagagatcg ttaccatata gctattggat tggccatccg gtgactaata gagctattat 660
atatcccttt gttgggttta taccacttag cttgaaagag gttaaaacat tacaattcat 720
tgttaagttg aatacagcaa aatgggagtc aaagttctgt ttgccctgat ctgcatcgct 780
gtggccgagg ccaagcccac cgagaacaac gaagacttca acatcgtggc cgtggccagc 840
aacttcgcga ccacggatct cgatgctgac cgcgggaagt tgcccggcaa gaagctgccg 900
ctggaggtgc tcaaagagat ggaagccaat gcccggaaag ctggctgcac caggggctgt 960
ctgatctgcc tgtcccacat caagtgcacg cccaagatga agaagttcat cccaggacgc 1020
tgccacacct acgaaggcga caaagagtcc gcacagggcg gcataggcga ggcgatcgtc 1080
gacattcctg agattcctgg gttcaaggac ttggagccca tggagcagtt catcgcacag 1140
gtcgatctgt gtgtggactg cacaactggc tgcctcaaag ggcttgccaa cgtgcagtgt 1200
tctgacctgc tcaagaagtg gctgccgcaa cgctgtgcga cctttgccag caagatccag 1260
ggccaggtgg acaagatcaa gggggccggt ggtgactaa 1299
SEQ ID NO: 72
   length 1299
   molecule type RNA
   division PAT
   Key Location/Qualifiers
   source 1..1299 /mol_type=other RNA /organism=synthetic construct
   Sequence 
cacgcggttg aaggagaaag cgttcgttat ccggccaact acttcgaaaa acctagtaac 60
accgtggaag ttgcagagtg tttcgctcag cactacccca gtgtagatca ggtcgatgag 120
tcaccgcatt ccccacgggc gaccgtggcg gtggctgcgt tggcggcctg cccatgggga 180
aacccatggg acgctctaat acagacatgg tgcgaagagt ctattgagct agttggtagt 240
cctccggccc ctgaatgcgg ctaatcctaa ctgcggagca cacaccctca agccagaggg 300
cagtgtgtcg taacgggcaa ctctgcagcg gaaccgacta ctttgggtgt ccgtgtttca 360
ttttattcct atactggctg cttatggtga caattgagag atcgttacca tatagctatt 420
ggattggcca tccggtgact aatagagcta ttatatatcc ctttgttggg tttataccac 480
ttagcttgaa agaggttaaa acattacaat tcattgttaa gttgaataca gcaaaatggg 540
agtcaaagtt ctgtttgccc tgatctgcat cgctgtggcc gaggccaagc ccaccgagaa 600
caacgaagac ttcaacatcg tggccgtggc cagcaacttc gcgaccacgg atctcgatgc 660
tgaccgcggg aagttgcccg gcaagaagct gccgctggag gtgctcaaag agatggaagc 720
caatgcccgg aaagctggct gcaccagggg ctgtctgatc tgcctgtccc acatcaagtg 780
cacgcccaag atgaagaagt tcatcccagg acgctgccac acctacgaag gcgacaaaga 840
gtccgcacag ggcggcatag gcgaggcgat cgtcgacatt cctgagattc ctgggttcaa 900
ggacttggag cccatggagc agttcatcgc acaggtcgat ctgtgtgtgg actgcacaac 960
tggctgcctc aaagggcttg ccaacgtgca gtgttctgac ctgctcaaga agtggctgcc 1020
gcaacgctgt gcgacctttg ccagcaagat ccagggccag gtggacaaga tcaagggggc 1080
 
cggtggtgac taattaaaac agcctgtggg ttgatcccac ccacaggccc attgggcgct 1140
agcactctgg tatcacggta cctttgtgcg cctgttttat acccctcccc ccaactgtaa 1200
cttagaagta acacacaccg atcaacagtc agcgtggcac accagccacg ttttgatcaa 1260
gcacttctgt taccccggac tgagtatcaa tagactgct 1299
SEQ ID NO: 73
   length 165
   molecule type RNA
   division PAT
   Key Location/Qualifiers
   source 1..165
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatg 165
SEQ ID NO: 74
   length 151
   molecule type RNA
   division PAT
   Key Location/Qualifiers
   source 1..151
      /mol_type=other RNA /organism=synthetic construct
   Sequence 
aaataattgg cccttaaaga agaaattctt taagtggatg ctctcaaact cagggaaacc 60
taaatctagt tatagacaag gcaatcctga gccaagccga agtagtaatt agtaaggcct 120
agtataccaa accgcgatat ctcttttcta g 151
SEQ ID NO: 75
   length 1615
   molecule type RNA
   division PAT
   Key Location/Qualifiers
   source 1..1615
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcacgc ggttgaagga 180
gaaagcgttc gttatccggc caactacttc gaaaaaccta gtaacaccgt ggaagttgca 240
gagtgtttcg ctcagcacta ccccagtgta gatcaggtcg atgagtcacc gcattcccca 300
cgggcgaccg tggcggtggc tgcgttggcg gcctgcccat ggggaaaccc atgggacgct 360
ctaatacaga catggtgcga agagtctatt gagctagttg gtagtcctcc ggcccctgaa 420
tgcggctaat cctaactgcg gagcacacac cctcaagcca gagggcagtg tgtcgtaacg 480
ggcaactctg cagcggaacc gactactttg ggtgtccgtg tttcatttta ttcctatact 540
ggctgcttat ggtgacaatt gagagatcgt taccatatag ctattggatt ggccatccgg 600
tgactaatag agctattata tatccctttg ttgggtttat accacttagc ttgaaagagg 660
ttaaaacatt acaattcatt gttaagttga atacagcaaa atgggagtca aagttctgtt 720
tgccctgatc tgcatcgctg tggccgaggc caagcccacc gagaacaacg aagacttcaa 780
catcgtggcc gtggccagca acttcgcgac cacggatctc gatgctgacc gcgggaagtt 840
gcccggcaag aagctgccgc tggaggtgct caaagagatg gaagccaatg cccggaaagc 900
 
tggctgcacc aggggctgtc tgatctgcct gtcccacatc aagtgcacgc ccaagatgaa 960
gaagttcatc ccaggacgct gccacaccta cgaaggcgac aaagagtccg cacagggcgg 1020
cataggcgag gcgatcgtcg acattcctga gattcctggg ttcaaggact tggagcccat 1080
ggagcagttc atcgcacagg tcgatctgtg tgtggactgc acaactggct gcctcaaagg 1140
gcttgccaac gtgcagtgtt ctgacctgct caagaagtgg ctgccgcaac gctgtgcgac 1200
ctttgccagc aagatccagg gccaggtgga caagatcaag ggggccggtg gtgactaatt 1260
aaaacagcct gtgggttgat cccacccaca ggcccattgg gcgctagcac tctggtatca 1320
cggtaccttt gtgcgcctgt tttatacccc ctcccccaac tgtaacttag aagtaacaca 1380
caccgatcaa cagtcagcgt ggcacaccag ccacgttttg atcaagcact tctgttaccc 1440
cggactgagt atcaatagac tgctaaataa ttggccctta aagaagaaat tctttaagtg 1500
gatgctctca aactcaggga aacctaaatc tagttataga caaggcaatc ctgagccaag 1560
ccgaagtagt aattagtaag gcctagtata ccaaaccgcg atatctcttt tctag 1615
SEQ ID NO: 76
   length 151
   molecule type RNA
   division PAT
   Key Location/Qualifiers
   source 1..151
      /mol_type=other RNA /organism=synthetic construct
   Sequence 
aaataattga gccttaaaga agaaattctt taagtggatg ctctcaaact cagggaaacc 60
taaatctagt tatagacaag gcaatcctga gccaagccga agtagtaatt agtaaggcct 120
agtataccaa accgcgatat ctcttttcta g 151
SEQ ID NO: 77
   length 37
   molecule type RNA
   division PAT
   Key Location/Qualifiers
   source 1..37
      /mol_type=other RNA /organism=synthetic construct
   Sequence
      gcagttcatc gcacaggtcg atctgtgtgt ggactgc 37
SEQ ID NO: 78
   length 558
   molecule type RNA
   division PAT
   Key Location/Qualifiers
   source 1..558
      /mol_type=other RNA /organism=synthetic construct
   Sequence 
atgggcgtca aggtgctctt tgccctgatc tgcatcgcgg tggcggaggc gaagcccacg 60
gagaacaatg aggacttcaa cattgttgcc gtggcttcga acttcgccac cactgactta 120
gatgcagatc ggggcaagct tccggggaag aagcttcccc tggaagttct caaggagatg 180
gaggcgaacg ctcgcaaggc cggttgcacc cgtgggtgct tgatttgtct ctctcatatc 240
aagtgcaccc ccaagatgaa gaaattcatc cctgggcggt gtcacactta tgagggagac 300
aaagagagcg cccagggtgg gatcggcgag gcgatcgtcg acatccctga gatccctggg 360
ttcaaggacc ttgagcccat ggagcagttc atcgcacagg tggacctgtg cgtggactgc 420
accactggat gtttgaaggg actggccaac gttcagtgca gcgacttgct gaagaagtgg 480
ctgcctcagc gttgtgccac gttcgcttca aagatccagg gtcaggtcga caagatcaag 540
 ggcgccggcg gcgattaa 558
SEQ ID NO: 79
   length 37
   molecule type RNA
   division PAT
   Key Location/Qualifiers
   source 1..37
      /mol_type=other RNA /organism=synthetic construct
   Sequence
      gcagttcatc gcacaggtgg acctgtgcgt ggactgc 37
SEQ ID NO: 80
   length 1615
   molecule type RNA
   division PAT
   Key Location/Qualifiers
   source 1..1615
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgcgatc tgtgtgtgga 180
ctgcacaact ggctgcctca aagggcttgc caacgtgcag tgttctgacc tgctcaagaa 240
gtggctgccg caacgctgtg cgacctttgc cagcaagatc cagggccagg tggacaagat 300
caagggggcc ggtggtgact aattaaaaca gcctgtgggt tgatcccacc cacaggccca 360
ttgggcgcta gcactctggt atcacggtac ctttgtgcgc ctgttttata ccccctcccc 420
caactgtaac ttagaagtaa cacacaccga tcaacagtca gcgtggcaca ccagccacgt 480
tttgatcaag cacttctgtt accccggact gagtatcaat agactgctca cgcggttgaa 540
ggagaaagcg ttcgttatcc ggccaactac ttcgaaaaac ctagtaacac cgtggaagtt 600
gcagagtgtt tcgctcagca ctaccccagt gtagatcagg tcgatgagtc accgcattcc 660
ccacgggcga ccgtggcggt ggctgcgttg gcggcctgcc catggggaaa cccatgggac 720
gctctaatac agacatggtg cgaagagtct attgagctag ttggtagtcc tccggcccct 780
gaatgcggct aatcctaact gcggagcaca caccctcaag ccagagggca gtgtgtcgta 840
acgggcaact ctgcagcgga accgactact ttgggtgtcc gtgtttcatt ttattcctat 900
actggctgct tatggtgaca attgagagat cgttaccata tagctattgg attggccatc 960
cggtgactaa tagagctatt atatatccct ttgttgggtt tataccactt agcttgaaag 1020
aggttaaaac attacaattc attgttaagt tgaatacagc aaaatgggag tcaaagttct 1080
gtttgccctg atctgcatcg ctgtggccga ggccaagccc accgagaaca acgaagactt 1140
caacatcgtg gccgtggcca gcaacttcgc gaccacggat ctcgatgctg accgcgggaa 1200
gttgcccggc aagaagctgc cgctggaggt gctcaaagag atggaagcca atgcccggaa 1260
agctggctgc accaggggct gtctgatctg cctgtcccac atcaagtgca cgcccaagat 1320
gaagaagttc atcccaggac gctgccacac ctacgaaggc gacaaagagt ccgcacaggg 1380
cggcataggc gaggcgatcg tcgacattcc tgagattcct gggttcaagg acttggagcc 1440
catggagcag ttcatcgcac aggtaaataa ttgcccctta aagaagaaat tctttaagtg 1500
gatgctctca aactcaggga aacctaaatc tagttataga caaggcaatc ctgagccaag 1560
ccgaagtagt aattagtaag gcctagtata ccaaaccgcg atatctcttt tctag 1615
SEQ ID NO: 81
   length 1615
   molecule type RNA
   division PAT
   Key Location/Qualifiers
   source 1..1615
      /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgggacc tgtgcgtgga 180
ctgcaccact ggatgtttga agggactggc caacgttcag tgcagcgact tgctgaagaa 240
gtggctgcct cagcgttgtg ccacgttcgc ttcaaagatc cagggtcagg tcgacaagat 300
caagggcgcc ggcggcgatt aattaaaaca gcctgtgggt tgatcccacc cacaggccca 360
ttgggcgcta gcactctggt atcacggtac ctttgtgcgc ctgttttata ccccctcccc 420
caactgtaac ttagaagtaa cacacaccga tcaacagtca gcgtggcaca ccagccacgt 480
tttgatcaag cacttctgtt accccggact gagtatcaat agactgctca cgcggttgaa 540
ggagaaagcg ttcgttatcc ggccaactac ttcgaaaaac ctagtaacac cgtggaagtt 600
gcagagtgtt tcgctcagca ctaccccagt gtagatcagg tcgatgagtc accgcattcc 660
ccacgggcga ccgtggcggt ggctgcgttg gcggcctgcc catggggaaa cccatgggac 720
gctctaatac agacatggtg cgaagagtct attgagctag ttggtagtcc tccggcccct 780
gaatgcggct aatcctaact gcggagcaca caccctcaag ccagagggca gtgtgtcgta 840
acgggcaact ctgcagcgga accgactact ttgggtgtcc gtgtttcatt ttattcctat 900
actggctgct tatggtgaca attgagagat cgttaccata tagctattgg attggccatc 960
cggtgactaa tagagctatt atatatccct ttgttgggtt tataccactt agcttgaaag 1020
aggttaaaac attacaattc attgttaagt tgaatacagc aaaatgggcg tcaaggtgct 1080
ctttgccctg atctgcatcg cggtggcgga ggcgaagccc acggagaaca atgaggactt 1140
caacattgtt gccgtggctt cgaacttcgc caccactgac ttagatgcag atcggggcaa 1200
gcttccgggg aagaagcttc ccctggaagt tctcaaggag atggaggcga acgctcgcaa 1260
ggccggttgc acccgtgggt gcttgatttg tctctctcat atcaagtgca cccccaagat 1320
gaagaaattc atccctgggc ggtgtcacac ttatgaggga gacaaagaga gcgcccaggg 1380
tgggatcggc gaggcgatcg tcgacatccc tgagatccct gggttcaagg accttgagcc 1440
catggagcag ttcatcgcac aggtaaataa ttgcccctta aagaagaaat tctttaagtg 1500
gatgctctca aactcaggga aacctaaatc tagttataga caaggcaatc ctgagccaag 1560
ccgaagtagt aattagtaag gcctagtata ccaaaccgcg atatctcttt tctag 1615
SEQ ID NO: 82
   length 1615
   molecule type RNA
   division PAT
   Key Location/Qualifiers
   source 1..1615 /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatgagact gctcacgcgg 180
ttgaaggaga aagcgttcgt tatccggcca actacttcga aaaacctagt aacaccgtgg 240
aagttgcaga gtgtttcgct cagcactacc ccagtgtaga tcaggtcgat gagtcaccgc 300
attccccacg ggcgaccgtg gcggtggctg cgttggcggc ctgcccatgg ggaaacccat 360
gggacgctct aatacagaca tggtgcgaag agtctattga gctagttggt agtcctccgg 420
cccctgaatg cggctaatcc taactgcgga gcacacaccc tcaagccaga gggcagtgtg 480
tcgtaacggg caactctgca gcggaaccga ctactttggg tgtccgtgtt tcattttatt 540
cctatactgg ctgcttatgg tgacaattga gagatcgtta ccatatagct attggattgg 600
ccatccggtg actaatagag ctattatata tccctttgtt gggtttatac cacttagctt 660
gaaagaggtt aaaacattac aattcattgt taagttgaat acagcaaaat gggagtcaaa 720
gttctgtttg ccctgatctg catcgctgtg gccgaggcca agcccaccga gaacaacgaa 780
gacttcaaca tcgtggccgt ggccagcaac ttcgcgacca cggatctcga tgctgaccgc 840
gggaagttgc ccggcaagaa gctgccgctg gaggtgctca aagagatgga agccaatgcc 900
cggaaagctg gctgcaccag gggctgtctg atctgcctgt cccacatcaa gtgcacgccc 960
 
aagatgaaga agttcatccc aggacgctgc cacacctacg aaggcgacaa agagtccgca 1020
cagggcggca taggcgaggc gatcgtcgac attcctgaga ttcctgggtt caaggacttg 1080
gagcccatgg agcagttcat cgcacaggtc gatctgtgtg tggactgcac aactggctgc 1140
ctcaaagggc ttgccaacgt gcagtgttct gacctgctca agaagtggct gccgcaacgc 1200
tgtgcgacct ttgccagcaa gatccagggc caggtggaca agatcaaggg ggccggtggt 1260
gactaattaa aacagcctgt gggttgatcc cacccacagg cccattgggc gctagcactc 1320
tggtatcacg gtacctttgt gcgcctgttt tataccccct cccccaactg taacttagaa 1380
gtaacacaca ccgatcaaca gtcagcgtgg cacaccagcc acgttttgat caagcacttc 1440
tgttaccccg gactgagtat caataaataa ttgttcctta aagaagaaat tctttaagtg 1500
gatgctctca aactcaggga aacctaaatc tagttataga caaggcaatc ctgagccaag 1560
ccgaagtagt aattagtaag gcctagtata ccaaaccgcg atatctcttt tctag 1615
SEQ ID NO: 83
   length 20
   molecule type DNA
   division PAT
   Key Location/Qualifiers
   source 1..20 /mol_type=other DNA /organism=synthetic construct
   Sequence
      ggcattggct tccatctctt 20
SEQ ID NO: 84
   length 21
   molecule type DNA
   division PAT
   Key Location/Qualifiers
   source 1..21 /mol_type=other DNA /organism=synthetic construct
   Sequence
      cctccatctc cttgagaact t 21
SEQ ID NO: 85
   length 22
   molecule type DNA
   division PAT
   Key Location/Qualifiers
   source 1..22 /mol_type=other DNA /organism=synthetic construct
   Sequence
      aagaagttca tcccaggacg ct 22
SEQ ID NO: 86
   length 20
   molecule type DNA
   division PAT
   Key Location/Qualifiers
   source 1..20 /mol_type=other DNA /organism=synthetic construct
   Sequence
      ggccacgatg ttgaagtctt 20
SEQ ID NO: 87
   length 24
   molecule type DNA
   division PAT
   Key Location/Qualifiers
   source 1..24 /mol_type=other DNA /organism=synthetic construct
Sequence
   atatagctat tggattggcc atcc 24
SEQ ID NO: 88
   length 19
   molecule type DNA
   division PAT
   Key Location/Qualifiers
   source 1..19 /mol_type=other DNA /organism=synthetic construct
Sequence
   cttcaaccgc gtgagcagt 19
SEQ ID NO: 89
   length 1618
   molecule type RNA
   division PAT
   Key Location/Qualifiers
   source 1..1618 /mol_type=other RNA /organism=synthetic construct
   Sequence 
gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatggattg ttatcataaa 180
gcgaattgga ttggccatcc ggtgaaagcg agacttacta tttacttact tgttggactt 240
accacactta atacatttat tctaggtgtc acttgtatag caattagaat caaacagttg 300
catcataatg ggagtcaaag ttctgtttgc cctgatctgc atcgctgtgg ccgaggccaa 360
gcccaccgag aacaacgaag acttcaacat cgtggccgtg gccagcaact tcgcgaccac 420
ggatctcgat gctgaccgcg ggaagttgcc cggcaagaag ctgccgctgg aggtgctcaa 480
agagatggaa gccaatgccc ggaaagctgg ctgcaccagg ggctgtctga tctgcctgtc 540
ccacatcaag tgcacgccca agatgaagaa gttcatccca ggacgctgcc acacctacga 600
aggcgacaaa gagtccgcac agggcggcat aggcgaggcg atcgtcgaca ttcctgagat 660
tcctgggttc aaggacttgg agcccatgga gcagttcatc gcacaggtcg atctgtgtgt 720
ggactgcaca actggctgcc tcaaagggct tgccaacgtg cagtgttctg acctgctcaa 780
gaagtggctg ccgcaacgct gtgcgacctt tgccagcaag atccagggcc aggtggacaa 840
gatcaagggg gccggtggtg actaattaaa acagctctgg ggttgtaccc accccagagg 900
cccacgtggc ggctagtact ccggtattac ggtacccttg tacgcctgtt ttatactccc 960
tccctcgtaa cttagaagca caaaaccaag ttcaatagaa gggggtgcaa accagtacca 1020
ccacgaacaa gcatttctgt ttccccggtg atgttgtata gactgcccgc gcggttgaaa 1080
gcaacggatc cgttacccgc tcaagtactt cgagaagcct agtattacct tggaatcttc 1140
gatgcgttgc gttcagcact cgaccctgga gtgtagctta ggctgatgag tctggacgtc 1200
cctcaccggt gacggtggtc caggctgcgt tggcggccta cctatggcta acgccatagg 1260
 
acgctagttg tgaacaaggt gtgaagagcc tattgagcta cttgagagtc ctccggcccc 1320
tgaatgcggc taatcctaac catggagcag gcggtcacag accagtgact agcttgtcgt 1380
aatgcgcaag tctatggcgg aaccgactac tttgggtgtc cgtgtttcct tttattttta 1440
ttatggctgc ttatggtgac aatcattaaa taattgatcc ttaaagaaga aattctttaa 1500
gtggatgctc tcaaactcag ggaaacctaa atctagttat agacaaggca atcctgagcc 1560
aagccgaagt agtaattagt aaggcctagt ataccaaacc gcgatatctc ttttctag 1618
SEQ ID NO: 90
   length 1639
   molecule type RNA
   division PAT
   Key Location/Qualifiers
   source 1..1639 /mol_type=other RNA /organism=synthetic construct
   Sequence

gggtcacccg tcgcgcggtt tggtatacta ggccaacaat agatgactta caactaatcg 60
gaaggtgcag agactcgacg ggagctaccc taacgtcaag acgagggtaa agagagagtc 120
caattctcaa agccaatagg cagtagcgaa agctgcaaga gaatggtcct gcggggccag 180
gccctgttgg tcaactcttc ccagccgtgg gagcccctgc agctgcatgt ggataaagcc 240
gtcagtggcc ttcgcagcct caccactctg cttcgggctc tgggagccca gaaggaagcc 300
atctcccctc cagatgcggc ctcagctgct ccactccgaa caatcactgc tgacactttc 360
cgcaaactct tccgagtcta ctccaatttc ctccggggaa agctgaagct gtacacaggg 420
gaggcctgca ggacagggga cagatgatta aaacagcctg tgggttgatc ccacccacag 480
gcccattggg cgctagcact ctggtatcac ggtacctttg tgcgcctgtt ttataccccc 540
tcccccaact gtaacttaga agtaacacac accgatcaac agtcagcgtg gcacaccagc 600
cacgttttga tcaagcactt ctgttacccc ggactgagta tcaatagact gctcacgcgg 660
ttgaaggaga aagcgttcgt tatccggcca actacttcga aaaacctagt aacaccgtgg 720
aagttgcaga gtgtttcgct cagcactacc ccagtgtaga tcaggtcgat gagtcaccgc 780
attccccacg ggcgaccgtg gcggtggctg cgttggcggc ctgcccatgg ggaaacccat 840
gggacgctct aatacagaca tggtgcgaag agtctattga gctagttggt agtcctccgg 900
cccctgaatg cggctaatcc taactgcgga gcacacaccc tcaagccaga gggcagtgtg 960
tcgtaacggg caactctgca gcggaaccga ctactttggg tgtccgtgtt tcattttatt 1020
cctatactgg ctgcttatgg tgacaattga gagatcgtta ccatatagct attggattgg 1080
ccatccggtg actaatagag ctattatata tccctttgtt gggtttatac cacttagctt 1140
gaaagaggtt aaaacattac aattcattgt taagttgaat acagcaaaat gggggtgcac 1200
gaatgtcctg cctggctgtg gcttctcctg tccctgctgt cgctccctct gggcctccca 1260
gtcctgggcg ccccaccacg cctcatctgt gacagccgag tcctggagag gtacctcttg 1320
gaggccaagg aggccgagaa tatcacgacg ggctgtgctg aacactgcag cttgaatgag 1380
aatatcactg tcccagacac caaagttaat ttctatgcct ggaagaggat ggaggtcggg 1440
cagcaggccg tagaagtctg gcagggcctg gccctgctgt cggaagctaa ataattggcc 1500
cttaaagaag aaattcttta agtggatgct ctcaaactca gggaaaccta aatctagtta 1560
tagacaaggc aatcctgagc caagccgaag tagtaattag taaggcctag tataccaaac 1620
cgcgatatct cttttctag 1639

## Claims

1. A polynucleotide for preparing a circular RNA, comprising from the 5' end to the 3' end operatively linked module Z1, module L to be circularized, and module Z2, and the polynucleotide is in a linear structure;
the modules Z1 and Z2 reconstitute into a ribozyme with self-splicing function, and can perform self-splicing (cleavage and ligation) under suitable conditions, thereby causing the module L to be circularized to form the circular RNA, wherein the splicing site is located in the distal loop region of the stem-loop structure formed by the module L to be circularized after forming circular RNA.

2. The polynucleotide of claim 1, wherein the stem-loop structure is a single stem-loop structure or a poly stem-loop structure.

3. The polynucleotide of claim 2, wherein the single stem-loop structure has a stem region and a distal loop region located at the distal end of the stem region.

4. The polynucleotide of claim 2, wherein the poly stem-loop structure has 2-5 distal loop regions.

5. The polynucleotide of claim 1, wherein the polynucleotide has the structure shown in Formula I:
Z1-L-Z2 (Formula I)
Z1 comprises the 3' end fragment h1 of the ribozyme;
L is the linear counterpart of the target sequence;
Z2 comprises the 5' end fragment h2 of the ribozyme.

6. The polynucleotide of claim 1, wherein the Z1 further comprises the 3' end fragment h1 of the ribozyme and a random sequence r1, and the Z2 further comprises the 5' end fragment h2 of the ribozyme and a random sequence r2, and the structure formula of the polynucleotide is as shown in Formula II:
r1-h1-L-h2-r2 (Formula II)
wherein r1 and r2 are reverse complementary sequences.

7. The polynucleotide of claim 1, wherein the ribozyme is an autocatalytic splicing ribozyme, and the ribozyme can recognize a specific site in module L.

8. The polynucleotide of claim 1, wherein the ribozyme can recognize a specific secondary structure in L, i.e., the secondary structure to be spliced constituted by the specific splicing site and its surrounding sequences thereof.

9. The polynucleotide of claim 1, wherein the ribozyme is a modified ribozyme obtained by partially or completely mutating positions 10 to 16 at the 5' end of the Anabaena pre-tRNA-Leu gene intron.

10. The polynucleotide of claim 1, wherein the module L is determined by the following method:
(1) directly connecting the 5' and 3' ends of the linear target sequence to be circularized to form a target circular sequence;
(2) determining a splicing site (SS) based on the local secondary structure of the target sequence, wherein the polynucleotide sequences on both sides of the splicing site are ribozyme recognition sites;
(3) splitting the target circular sequence at the selected splicing site (SS) to form the module L.

11. The polynucleotide of claim 10, wherein the splicing site is located in a TLSL structure in the multiple stem-loop structure, the TLSL is composed of a multi-arm junction and multiple distal loops connected, wherein one arm of the multi-arm junction is a terminal arm, the terminal arm is not connected to a distal loop, and each of the other arms is respectively connected to a distal loop to form a closed connection, forming a continuous multi-distal-loop structure with exposed 5' and 3' ends only on the terminal arm.

12. The polynucleotide of claim 10, wherein the splicing site is located in an SL structure in the single stem-loop structure, the SL is composed of an arm connection and a distal loop connected, the splicing site is located between nucleotide U (or T) and an adjacent nucleotide on the distal loop, and the nucleotide U (or T) is located at the 3' end of the module L formed after splitting at the splicing site.

13. A vector comprising the polynucleotide of claim 1.

14. A method for preparing a circular RNA, comprising the steps of:
(1) determining a splicing site based on the local secondary structure of the target sequence, obtaining the module L in the polynucleotide of claim 1, i.e., the linear counterpart of the target sequence;
(2) connecting the 3' end fragment of the ribozyme, the linear counterpart of the target sequence, and the 5' end fragment of the ribozyme by an appropriate method to obtain a circular RNA precursor molecule;
(3) the circular RNA precursor molecule obtained in step (2) undergoes a self-splicing reaction under a suitable condition to produce the target circular RNA.

15. A circular RNA, which is formed by self-splicing of the polynucleotide of claim 1.

16. A pharmaceutical composition, comprising (a) the polynucleotide for preparing a circular RNA of claim 1, the circular RNA of claim 15, or a combination thereof; and (b) a pharmaceutically acceptable carrier thereof.

17. An engineered host cell, comprising the circular RNA of claim 15, or comprising the vector of claim 13, or having integrated into its genome a coding sequence for producing the polynucleotide of claim 1.

18. A method for producing a polypeptide or protein in a cell, comprising: contacting the cell with (1) the circular RNA of claim 15, or (ii) the vector of claim 13, under a condition where the polypeptide or protein encoding nucleic acid sequence of the circular RNA can be translated and the polypeptide or protein can be produced in the cell;
alternatively, the method comprising: culturing the engineered host cell of claim 17 under a condition suitable for expression, thereby producing the polypeptide or protein;
alternatively, the method comprising:, transfecting the circular RNA of claim 15 into the cell using lipid transfection, nanocarrier, polymer, or electroporation, thereby producing the polypeptide or protein.

19. Use of the circular RNA of claim 15, the vector of claim 13, the pharmaceutical composition of claim 16, the engineered host cell of claim 17, or the method for producing a polypeptide or protein in a cell of claim 18, for the preparation of a pharmaceutical composition or a cosmetic composition.
